(19) Europäisches Patentamt
European Patent Office
Office européen des brevets

(11) Publication number : 0 675 112 A1

# (12) EUROPEAN PATENT APPLICATION

(21) Application number : 95302188.8

(22) Date of filing : 31.03.95

(51) Int. Cl.[6] : C07D 233/54, A61K 31/415, A61K 38/05, A61K 38/06, A61K 38/07, C07D 521/00, C07D 401/12, C07K 5/023, C07K 5/027, C07K 5/062, C07K 5/065, C07K 5/083, C07K 5/117

(30) Priority : 31.03.94 US 221153
19.08.94 US 292916

(43) Date of publication of application :
04.10.95 Bulletin 95/40

(84) Designated Contracting States :
AT BE CH DE DK ES FR GB GR IE IT LI LU MC NL PT SE

(71) Applicant : BRISTOL-MYERS SQUIBB COMPANY
P.O. Box 4000
Princeton, NJ 08543-4000 (US)

(72) Inventor : Hunt, John T.
7 Skyfield Drive
Princeton, New Jersey 08540 (JP)

(74) Representative : Thomas, Roger Tamlyn et al
D. Young & Co.
21 New Fetter Lane
London EC4A 1DA (GB)

## (54) Imidazole-containing inhibitors of farnesyl protein transferase.

(57) Inhibition of farnesyl transferase, which is an enzyme involved in ras oncogene expression, is effected by compounds of the formula

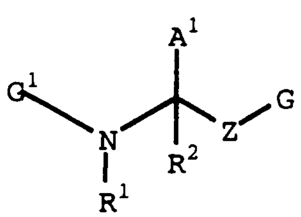

their enantiomers, diastereomers, and pharmaceutically acceptable salts, prodrugs, and solvates, wherein :

G is

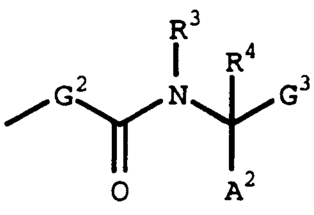 ,

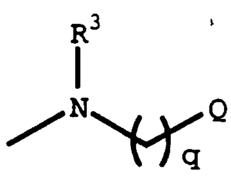 ,



Jouve, 18, rue Saint-Denis, 75001 PARIS

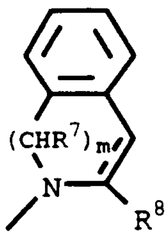

or

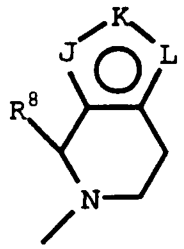

;

$G^1$ is

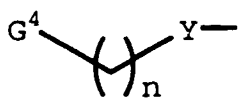

or

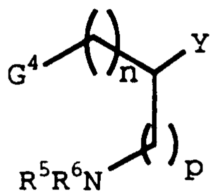

;

$G^2$ is

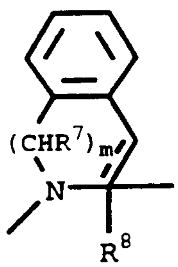

,

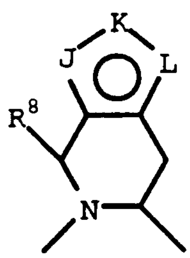

or $-NR^{10}-CH(Q^1)-$ ;

J, K and L are each, independently, N, $NR^9$, O, S or $CR^{10}$ with the provisos that only one of the groups J, K and L can be O or S, and at least one of the groups J or L must be N, $NR^9$, O or S to form a fused five-membered heteroring ; the bond between J and K or K and L may also form one side of a phenyl ring fused to the fused five-membered heteroring ;

Q is aryl ;

$Q^1$, $A^1$ and $A^2$ are each, independently, H, alkyl, substituted alkyl, phenyl or substituted phenyl ;

$G^3$ is $R^{11}$, $-C(O)OR^{11}$, $-C(O)NR^{11}R^{12}$, 5-tetrazolyl, $-C(O)N(R^{13})OR^{11}$, $-C(O)NHSO_2R^{14}$ or $-CH_2OR^{11}$ ;

$G^4$ is

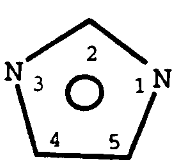

attached at the 1, 2, 4 or 5 position and optionally substituted, at any of the available position or positions on the ring, with halo, alkyl or substituted alkyl having 1 to 20 carbon atoms, alkoxy, aryl, aralkyl, hydroxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, N-hydroxycarbamyl, N-alkylcarbamyl, N-dialkylcarbamyl, alkoxycarbonyl, phenyl, substituted phenyl, or a combinaton of these groups ;

Y and Z are each, independently, $-CH_2-$ or $-C(O)-$ ;

$R^1$ - $R^{14}$ are each, independently, H or alkyl having 1 to 20 carbon atoms ;

$R^7$, $R^8$ and $R^{14}$ may also be aryl or aralkyl, and $R^3$, $R^9$, $R^{11}$, $R^{12}$ and $R^{13}$ may also be aralkyl ;

m, n and p are each, independently, 0 or an integer from 1 to 2 ;

q is 0 or an integer from 1 to 4 ; and

the dotted line represents an optional double bond.

This application is a continuation-in-part of application serial number 08/292,916 filed on August 19, 1994, which in turn is a continuation-in-part of application serial number 08/221,153 filed on March 31, 1994. The entire contents of these applications are hereby incorporated by reference.

This invention relates to compounds that inhibit farnesyl-protein transferase and Ras protein farnesylation, thereby making them useful as anti-cancer agents. The compounds are also useful in the treatment of diseases, other than cancer, associated with signal transduction pathways operating through Ras and those associated with CAAX-containing proteins other than Ras that are also post-translationally modified by the enzyme farnesyl protein transferase. The compounds may also act as inhibitors of other prenyl transferases, and thus be effective in the treatment of diseases associated with other prenyl modifications of proteins.

The mammalian ras gene family comprises three genes, H-ras, K-ras and N-ras. The Ras proteins are a family of GTP-binding and hydrolyzing proteins that regulate cell growth and differentiation. Overproduction of normal Ras proteins or mutations that inhibit their GTPase activity can lead to uncontrolled cell division.

The transforming activity of Ras is dependent on localization of the protein to plasma membranes. This membrane binding occurs via a series of post-translational modifications of the cytosolic Ras proteins. The first and mandatory step in this sequence of events is the farnesylation of these proteins. The reaction is catalyzed by the enzyme farnesyl protein transferase (FPT), and farnesyl pyrophosphate (FPP) serves as the farnesyl group donor in this reaction. The Ras C-terminus contains a sequence motif termed a "Cys-$Aaa_1$-$Aaa_2$-Xaa" box (CAAX box), wherein Cys is cysteine, Aaa is an aliphatic amino acid, and Xaa is a serine or methionine. Farnesylation occurs on the cysteinyl residue of the CAAX box (Cys-186), thereby attaching the prenyl group on the protein via a thio-ether linkage.

In accordance with the present invention, a compound of the formula

I

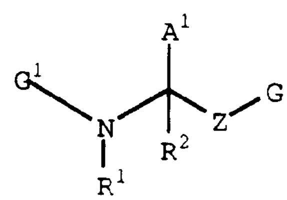

its enantiomers and diastereomers, and pharmaceutically acceptable salts, prodrugs and solvates thereof inhibit S-farnesyl protein transferase, which is an enzyme involved in Ras oncogene function. In formula I and throughout this specification, unless otherwise specified, the above symbols are defined as follows:

G is

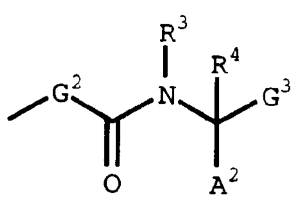 , 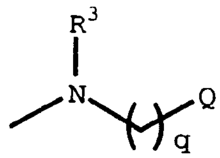 ,

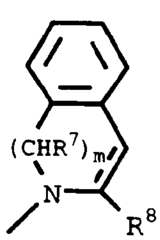 or 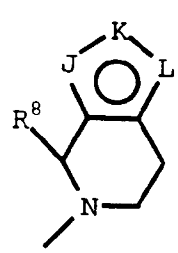 ;

$G^1$ is

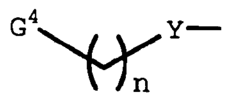

or

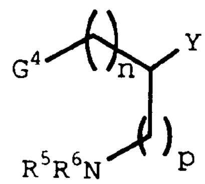

;

$G^2$ is

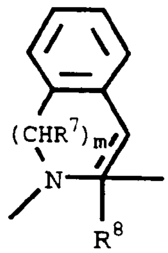

,

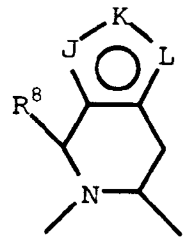

or $-NR^{10}-CH(Q^1)-$;

J, K and L are each, independently, N, $NR^9$, O, S or $CR^{10}$ with the provisos that only one of the groups J, K and L can be O or S, and at least one of the groups J or L must be N, $NR^9$, O or S to form a fused five-membered heteroring; the bond between J and K or K and L may also form one side of a phenyl ring fused to the fused five-membered heteroring;

Q is aryl;

$Q^1$, $A^1$ and $A^2$ are each, independently, H, alkyl, substituted alkyl, phenyl or substituted phenyl;

$G^3$ is $R^{11}$, $-C(O)OR^{11}$, $-C(O)NR^{11}R^{12}$, 5-tetrazolyl, $-C(O)N(R^{13})OR^{11}$, $-C(O)NHSO_2R^{14}$ or $-CH_2OR^{11}$;

$G^4$ is

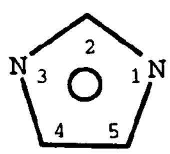

attached at the 1, 2, 4 or 5 position and optionally substituted, at any of the available position or positions on the ring, with halo, alkyl or substituted alkyl having 1 to 20 carbon atoms, alkoxy, aryl, aralkyl, hydroxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, N-hydroxycarbamyl, N-alkylcarbamyl, N-dialkylcarbamyl, alkoxycarbonyl, phenyl, substituted phenyl, or a combination of these groups;

Y and Z are each, independently, $-CH_2-$ or $-C(O)-$;

$R^1$ - $R^{14}$ are each, independently, H or alkyl having 1 to 20 carbon atoms;

$R^7$, $R^8$ and $R^{14}$ may also be aryl or aralkyl, and $R^3$, $R^9$, $R^{11}$, $R^{12}$ and $R^{13}$ may also be aralkyl;

m, n and p are each, independently, 0 or an integer from 1 to 2;

q is 0 or an integer from 1 to 4; and

the dotted line represents an optional double bond.

Listed below are definitions of various terms used to describe this invention. These definitions apply to the terms as they are used throughout this specification, unless otherwise limited in specific instances, either individually or as part of a larger group.

The term "alkyl" refers to straight or branched chain unsubstituted hydrocarbon groups of 1 to 20 carbon atoms, preferably 1 to 7 carbon atoms. The expression "lower alkyl" refers to unsubstituted alkyl groups of 1 to 4 carbon atoms.

The term "substituted alkyl" refers to an alkyl group substituted by, for example, one to four substituents such as halo, hydroxy, alkoxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, substituted carbamyl, alkoxycarbonyl, phenyl, substituted phenyl, guanidino, indolyl, imidazolyl, furyl, thienyl, thiazolyl, pyrrolidyl, pyridyl,

pyrimidyl and the like.

The term "halogen" or "halo" refers to fluorine, chlorine, bromine and iodine.

The term "alkoxy" refers to alkyl-O-.

The term "alkanoyl" refers to alkyl-C(O)-.

The term "alkanoyloxy" refers to alkyl-C(O)-O-.

The terms "alkylamino" and "dialkylamino" refer to (alkyl)NH- and $(alkyl)_2N$-, respectively.

The term "alkanoylamino" refers to alkyl-C(O)-NH-.

The term "alkylthio" refers to alkyl-S-.

The term "alkylthiono" refers to alkyl-S(O)-.

The term "alkylsulfonyl" refers to alkyl-$S(O)_2$-.

The term "carbamyl" refers to -C(O)$NH_2$.

The term "alkoxycarbonyl" refers to alkyl-O-C(O)-.

The term "aryl" refers to monocyclic or bicyclic aromatic hydrocarbon groups having 6 to 12 carbon atoms in the ring portion, such as phenyl, naphthyl, biphenyl and diphenyl groups, each of which may be substituted.

The term "aralkyl" refers to an aryl group bonded directly through an alkyl group, such as benzyl.

The term "substituted phenyl" refers to a phenyl group substituted by, for example, one to four substituents such as alkyl, halo, hydroxy, alkoxy, alkanoyl, alkanoyloxy, amino, alkylamino, dialkylamino, alkanoylamino, thiol, alkylthio, nitro, cyano, carboxy, carboxyalkyl, carbamyl, alkoxycarbonyl, alkylthiono, alkylsulfonyl, sulfonamido and the like.

The expression "substituted carbamyl" refers to a carbamyl group N-substituted by one to two substituents selected from hydroxy and alkyl, and when both the substituents are alkyl, they may be taken together to form a 5- to 7- membered saturated ring with the nitrogen atom to which they are attached, said ring being optionally substituted with halogen, hydroxy, alkoxy, oxo, nitro, cyano, -C(O)H, -C(O)OH and the like.

The compounds of formula I form salts which are also within the scope of this invention. Pharmaceutically acceptable (i.e., non-toxic, physiologically acceptable) salts are preferred, although other salts are also useful, e.g., in isolating or purifying the compounds of this invention.

The compounds of formula I may form salts with alkali metals such as sodium, potassium and lithium, with alkaline earth metals such as calcium and magnesium, with organic bases such as dicyclohexylamine, tributylamine, pyridine and amino acids such as arginine, lysine and the like. Such salts may be obtained, for example, by exchanging the carboxylic acid protons, if they contain a carboxylic acid, in compound I with the desired ion in a medium in which the salt precipitates or in an aqueous medium followed by evaporation. Other salts can be formed as known to those skilled in the art.

The compounds of formula I may form salts with a variety of organic and inorganic acids. Such salts include those formed with hydrogen chloride, hydrogen bromide, methanesulfonic acid, sulfuric acid, acetic acid, trifluoroacetic acid, maleic acid, benzenesulfonic acid, toluenesulfonic acid and various others (e.g., nitrates, phosphates, borates, tartrates, citrates, succinates, benzoates, ascorbates, salicylates and the like). Such salts may be formed by reacting compound I in an equivalent amount of the acid in a medium in which the salt precipitates or in an aqueous medium followed by evaporation.

In addition, zwitterions ("inner salts") may be formed.

A compound of the formula I may also have prodrug forms. Any compound that will be converted in vivo to provide the bioactive agent (i.e., the compound of formula I) is a prodrug within the scope and spirit of the invention. For example, compound I may be in the form of a prodrug having the formula

II

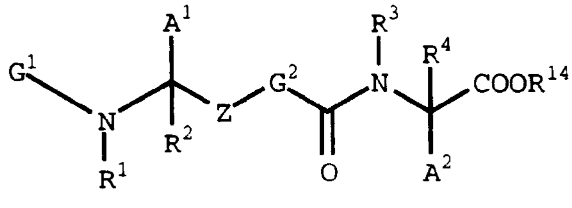

wherein $R^{14}$ is:

lower alkyl, such as methyl, ethyl and the like;

substituted lower alkyl, such as 2-(N-morpholine)ethyl and the like;

lower aralkyl, such as benzyl, biphenylmethyl and the like;

(acyloxy)alkyl, such as (pivalyloxy)methyl, 1-(propanoyloxy)-2-methyl-1-propyl and the like;

(aminoacyloxy)aroyloxyalkyl, such as para-glycyloxybenzoyloxymethyl and the like;

(aminoalkoxy)aroyloxyalkyl, such as para-2-[(N-morpholine)ethoxy]benzoyloxymethyl and the like;

substituted amides, such as N,N-di(2-hydroxyethyl) acetamido, 4-methylpiperazine-1-acetyl, 4-(2-hydroxyethyl)piperazine-1-acetyl and the like; or

a dioxolanemethyl, such as (5-methyl-2-oxo-1,3-dioxolan-4-yl)methyl and the like.

Further, for a compound of formula I where $A^2$ is substituted alkyl of the formula $-(CH_2)_wOH$ (where w is 2 or 3), and $G^3$ is -C(O)OH, $A^2$ may be joined with the carboxyl group to form a lactone ring which can be opened in vivo to give a compound of formula I where $A^2$ is $-(CH_2)_wOH$ (again where w is 2 or 3).

Various forms of prodrugs are well known in the art. For examples of such prodrug derivatives, see:

a) Design of Prodrugs, edited by H. Bundgaard, (Elsevier, 1985) and Methods in Enzymology, Vol. 42, p. 309-396, edited by K. Widder, *et al.* (Academic Press, 1985) ;

b) A Textbook of Drug Design and Development, edited by Krogsgaard-Larsen and H. Bundgaard, Chapter 5, "Design and Application of Prodrugs," by H. Bundgaard, p. 113-191 (1991) ;

c) H. Bundgaard, Advanced Drug Delivery Reviews, 8, 1-38 (1992) ;

d) H. Bundgaard, *et al.*, Journal of Pharmaceutical Sciences, 77, 285 (1988) ; and e) N. Kakeya, *et al.*, Chem Pharm Bull, 32, 692 (1984).

It should further be understood that solvates (e.g., hydrates) of the compounds of formula I are also within the scope of the present invention. Methods of solvation are generally known in the art.

## Preferred Moieties

For compounds of the formula I, the following moieties are preferred:

G is

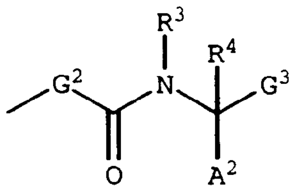 or 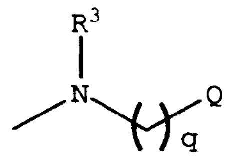 ;

$G^2$ is

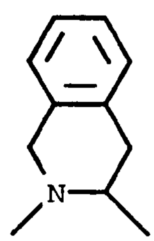 ;

$A^1$ and $A^2$ are each, independently, H or D-, L- or DL- $-CH_3$, $-CH(CH_3)_2$, $-CH_2CH(CH_3)_2$, $-CH(CH_3)CH_2CH_3$, $-C(CH_3)_3$, $-CH_2OH$, $-CH_2CH_2OH$, $-CH_2CH_2CH_2OH$, $-CH(OH)\,CH_3$,

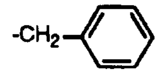 ,

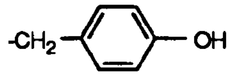 , 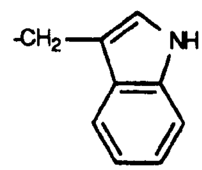 ,

$-CH_2CH_2CH_2CH_2NH_2$,

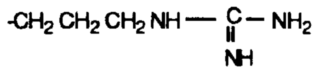

,

$-CH_2C(O)OH$, $-CH_2CH_2C(O)OH$, $-CH_2C(O)NH_2$, $-CH_2CH_2C(O)NH_2$, $-CH_2CH_2OCH_3$, $-CH_2CH_2CONR^{15}R^{16}$, $-CH_2SH$, $-CH_2CH_2SH$, $-CH_2CH_2SOCH_3$, $CH_2CH_2SO_2CH_3$ or $-CH_2CH_2SCH_3$, where $R^{15}$ and $R^{16}$ are each, independently, hydrogen or alkyl, or $R^{15}$ and $R^{16}$, taken together, form a 5- to 7-membered saturated ring with the N-atom to which they are attached, said ring being optionally substituted with halogen, hydroxy, alkoxy, oxo, nitro, cyano, -C(O)H, -C(O)OH and the like;

$G^3$ is -C(O)OH, $-C(O)OR^{11}$ or $-C(O)NHSO_2R^{14}$; and

$G^4$ is

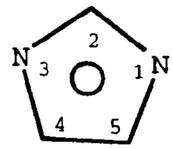

attached at the 1, 2, 4 or 5 position, and optionally substituted, at any available position or positions on the ring, with alkyl having 1 to 20 carbon atoms or aralkyl.

When $G^2$ is

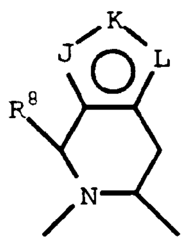

,

the fused five-membered (substituted) heteroring is preferably

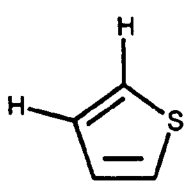

,

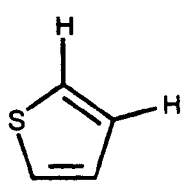

,

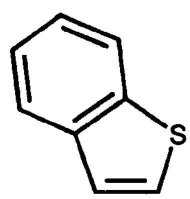

,

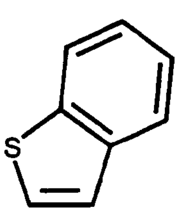

,

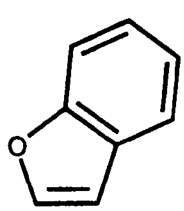

,

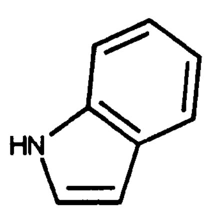

or

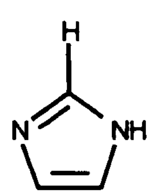

;

and when $G^2$ is $-NR^{10}-CH(Q^1)-$, $Q^1$ is preferably D-, L- or D,L-

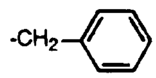 , 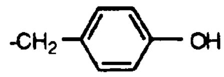 ,

or

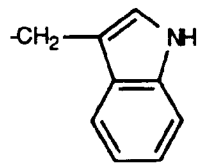 .

The following moieties are particularly preferred:

$G^2$ is

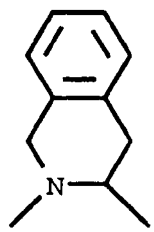 ;

$G^3$ is -C(O)OH, -C(O)OR$^{11}$ or -C(O)NHSO$_2$R$^{14}$;

$G^4$ is

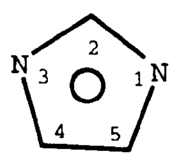

attached at the 1, 2, 4 or 5 position, and optionally substituted, at any available position or positions on the ring, with alkyl having 1 to 20 carbon atoms or aralkyl;

$A^1$ is L- $-CH_3$, $-CH(CH_3)_2$, $-CH_2CH(CH_3)_2$, $-C(CH_3)_3$,

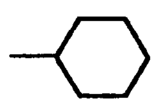 ,

$-CH(CH_3)CH_2CH_3$, $-CH_2OH$ or $-CH(OH)CH_3$;

$A^2$ is L- $-CH_2CH_2SCH_3$, $-CH_2CH_2OH$, $-CH_2CH_2CH_2OH$, $-CH_2CH_2C(O)NH_2$, $-CH_2CH_2OCH_3$, $-CH_2CH_2CONR^{15}R^{16}$, $-CH_2CH_2SH$, $-CH_2CH_2SOCH_3$ or $-CH_2CH_2SO_2CH_3$; and

$R^1$, $R^2$, $R^3$ and $R^4$ are H.

However, when

G is

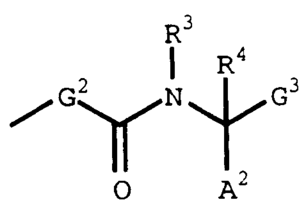 ,

$G^1$ is

is 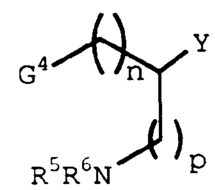 ,

$G^2$ is $-NHCH(CH_2C_6H_5)-$,
$G^3$ is $-C(O)OH$,
Y and Z are both $-C(O)-$,
$A^1$ and $A^2$ are each, independently, D-, L- or D,L- $-CH_3$, $-CH(CH_3)_2$, $-CH_2CH(CH_3)_2$, $-CH(CH_3)CH_2CH_3$, $-C(CH_3)_3$, $-CH_2OH$, $-CH_2CH_2OH$, $-CH_2CH_2CH_2OH$, $-CH(OH)CH_3$,

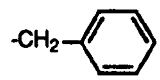 ,

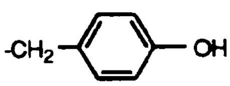 , 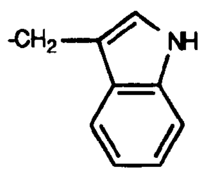 ,

$-CH_2CH_2CH_2CH_2NH_2$,

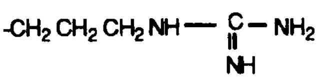 ,

$-CH_2C(O)OH$, $-CH_2CH_2C(O)OH$, $-CH_2C(O)NH_2$, $-CH_2CH_2C(O)NH_2$, $-CH_2CH_2C(O)NHOH$, $-CH_2SH$, $-CH_2CH_2SH$, $-CH_2CH_2S(O)_2NH_2$ or $-CH_2CH_2SCH_3$,
$R^1$-$R^6$ are all H,
n is 1 and
p is 0, then $G^4$ is preferably substituted.

## Use and Utility

The compounds of formula I are inhibitors of S-farnesyl protein transferase. They are thus useful in the treatment of a variety of cancers, including (but not limited to) the following:

- carcinoma, including that of the bladder, breast, colon, kidney, liver, lung, ovary, pancreas, stomach, cervix, thyroid and skin;
- hematopoietic tumors of lymphoid lineage, including acute lymphocytic leukemia, B-cell lymphoma and Burketts lymphoma;
- hematopoietic tumors of myeloid lineage, including acute and chronic myelogenous leukemias and promyelocytic leukemia;
- tumors of mesenchymal origin, including fibrosarcoma and rhabdomyosarcoma; and
- other tumors, including melanoma, seminoma, tetratocarcinoma, neuroblastoma and glioma.

The compounds of formula I are especially useful in treatment of tumors having a high incidence of Ras involvement, such as colon, lung, and pancreatic tumors. By the administration of a composition having one (or a combination) of the compounds of this invention, development of tumors in a mammalian host is reduced.

Compounds of formula I may also be useful in the treatment of diseases other than cancer that may be associated with signal transduction pathways operating through Ras, e.g., neuro-fibromatosis.

Compounds of formula I may also be useful in the treatment of diseases associated with CAAX-containing

proteins other than Ras (e.g., nuclear lamins and transducin) that are also post-translationally modified by the enzyme farnesyl protein transferase.

Compounds of formula I may also act as inhibitors of other prenyl transferases (e.g., geranylgeranyl transferase), and thus be effective in the treatment of diseases associated with other prenyl modifications (e.g., geranylgeranylation) of proteins (e.g., the rap, rab, rac and rho gene products and the like). For example, they may find use as drugs against Hepatitis delta virus (HDV) infections, as suggested by the recent finding that geranylgeranylation of the large isoform of the delta antigen of HDV is a requirement for productive viral infection [J. S. Glenn, *et al.*, Science, 256, 1331 (1992)].

The compounds of this invention may also be useful in combination with known anti-cancer and cytotoxic agents. If formulated as a fixed dose, such combination products employ the compounds of this invention within the dosage range described below and the other pharmaceutically active agent within its approved dosage range. Compounds of formula I may be used sequentially with known anticancer or cytotoxic agents when a combination formulation is inappropriate.

The compounds of this invention may be formulated with a pharmaceutical vehicle or diluent for oral, intravenous or subcutaneous administration. The pharmaceutical composition can be formulated in a classical manner using solid or liquid vehicles, diluents and additives appropriate to the desired mode of administration. Orally, the compounds can be administered in the form of tablets, capsules, granules, powders and the like. These compounds may be administered in a dosage range of about 0.05 to 50 mg/kg/day, preferably less than 50 mg/kg/day, in a single dose or in 2 to 4 divided doses.

## Process of Preparation

A compound of formula III below, wherein $Prot^1$ is an amine protecting group (e.g., *t*-butyloxycarbonyl (Boc), benzyloxycarbonyl (Cbz) and the like):

III

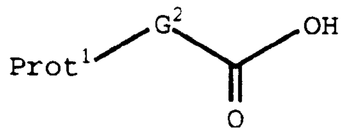

can be coupled with a carboxylic acid protected derivative of an amino acid of formula IV:

IV

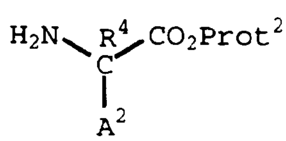

to form a compound of formula V below, wherein $Prot^2$ is a carboxylic acid protecting group (e.g., alkyl, benzyl, p-methoxybenzyl and the like) :

V

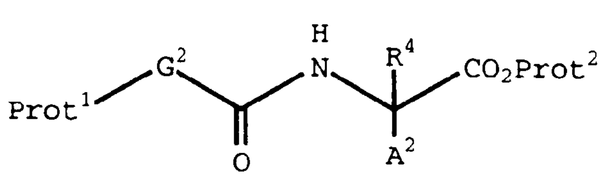

For additional examples of amino and carboxylic acid protecting groups (as well as means of formation and eventual deprotection), see T.W. Greene and P.G.M. Wuts, "Protective Groups in Organic Synthesis", Second Edition, John Wiley & Sons, New York, 1991.

A variety of coupling agents may be used for this coupling, including 1-(3-dimethylaminopropyl)-3-ethylcarbodiimide hydrochloride (EDC) with 1-hydroxybenzotriazole (HOBT), dicyclohexylcarbodiimide (DCC) with HOBT, benzotriazol-1-yloxytris(dimethylamino)phosphonium hexafluorophosphate (BOP) with or without

HOBT, carbonyldiimidazole (CDI), bis(2-oxo-3-oxazolidinyl)phosphinic chloride (BOP chloride), isopropylchloroformate (IPCF) and the like.

Compounds of the formula IV are known in the art. See, for example, R. M. Williams, "Synthesis of Optically Active α-Amino Acids," Pergamon Press, Oxford, 1989.

Compounds of formula III can be prepared by methods known in the art. For example, see J. P. Maffrand, U.S. Patent No. 4,147,787 issued April 3, 1979; H. Kawakubo, *et al.*, J. Med. Chem., 33, 3110 (1990); D. G. Harvey, *et al.*, J. Chem Soc., 153 (1941); A. Brossi, *et al.*, J. Med. Chem. 16, 418, (1973); M. Cain, *et al.*, Heterocycles, 19, 1003 (1982); S. Ueki, *et al.*, U.S. Patent No. 5,126,448 issued June 30, 1992; J. W. Skiles, *et al.*, J. Med. Chem., 29, 784 (1986); V. Schollkopf, *et al.*, Angew. Chem. Int. Ed. Engl., 26, 143 (1987); I. Huber and D. Seebach, Helvitica Chim Acta, 70, 1944 (1987); J. L. Stanton, *et al.*, J. Med. Chem., 26, 1267 (1983); and M. Okada, *et al.*, JP 02,193,971, 31 July 1990.

A compound of formula V can be treated with a suitable N-deprotecting agent to provide the corresponding free amine of formula VI:

VI

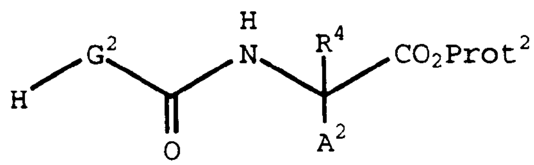

(The Prot[1] and Prot[2] protecting groups are chosen so that Prot[1] can be selectively removed in the presence of Prot[2]).

An amine of formula VI can be coupled with a suitable amine-protected amino acid of formula VII (wherein Prot[3] is an amine protecting group) :

VII

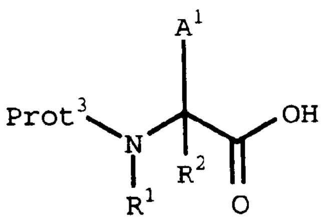

using an appropriate coupling reagent (e.g., BOP-Cl) to form a compound of formula VIII:

VIII

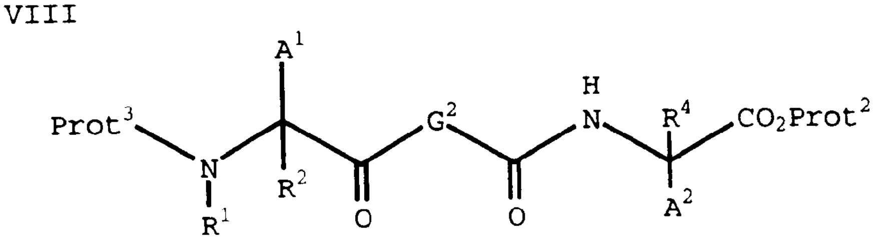

(Compounds of formula VII are known in the art. See, for example, R.M. Williams, "Synthesis of Optically Active α-Amino Acids," Pergamon Press, Oxford, 1989).

Compounds of formula VIII can also be prepared by coupling a compound of the formula IX (wherein Prot[4] is a carboxylic acid protecting group) :

IX

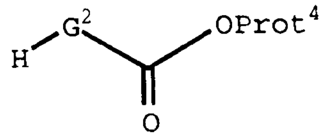

with an amine-protected amino acid of formula VII above to provide a compound of formula X:

X

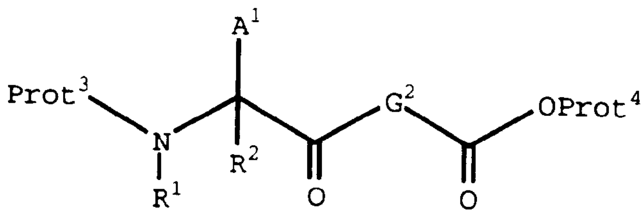

The $Prot^4$ protecting group of compound X can be selectively removed by methods known in the art to provide a compound of formula XI below:

XI

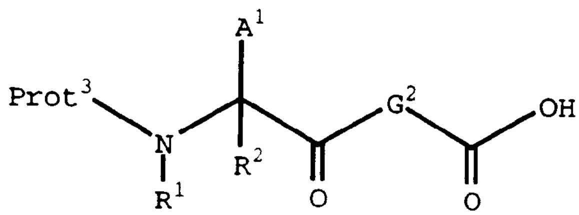

(The $Prot^3$ and $Prot^4$ protecting groups are chosen so that $Prot^4$ can be selectively removed in the presence of $Prot^3$).

Coupling of a compound of formula XI with an amino ester of formula IV would then provide compound VIII.

A compound of formula VIII can be selectively N-deprotected by methods known in the art to provide an amine of formula XII:

XII

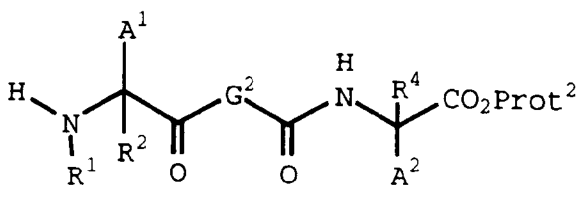

(The $Prot^2$ and $Prot^3$ protecting groups are chosen so that $Prot^3$ can be selectively removed in the presence of $Prot^2$).

Additionally, an amine of the formula VI'(a), VI'(b) or VI'(c):

VI'(a)

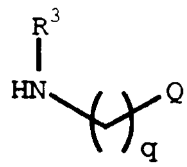

VI'(b)

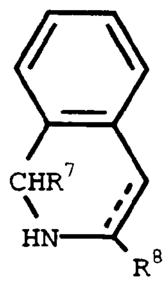

VI'(c)

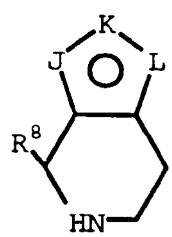

can be coupled with a suitable amino protected amino acid of the formula VII to provide a compound of the formula VIII'(a), VIII'(b) or VIII'(c), respectively:

VIII'(a)

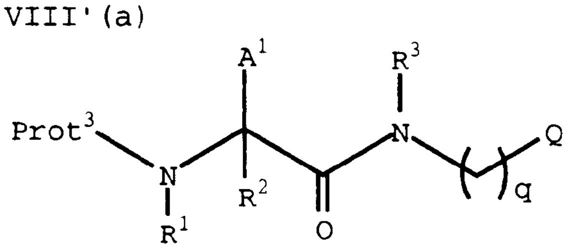

VIII'(b)

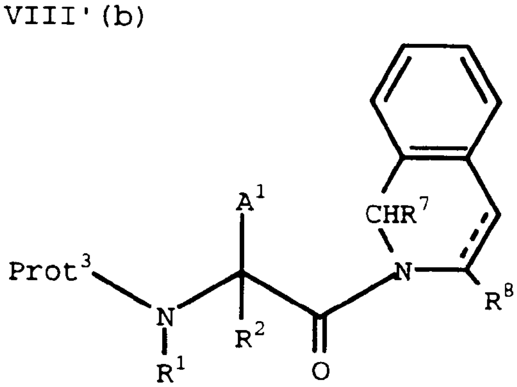

VIII'(c)

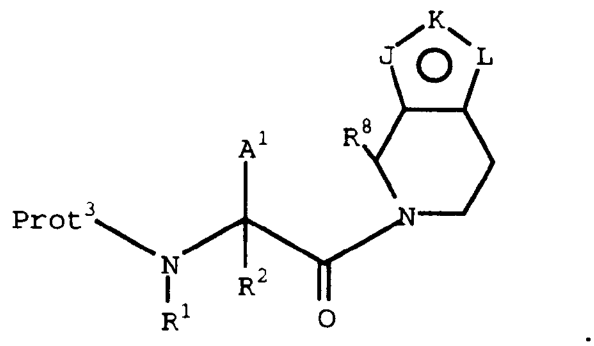

(The $Prot^3$ protecting group can then be removed by methods known in the art.)

Further, a compound of formula I where Y and Z are -C(O)- can be prepared by automated solid phase peptide synthesis using methods that are well known in the art. See, for example:

a) M. Bodansky and A. Bodansky, "The Practice of Peptide Synthesis", Springer-Verlag, Berlin/Heidelberg/New York/Tokyo, 1984; and

b) J. M. Stewart and J. D. Young, "Solid Phase Peptide Synthesis", Pierce Chemical Co., Rockport, Illinois, 1984.

In another process, an amino acid of formula VII (wherein $Prot^3$ is a suitable amino protecting group) can be converted to the N-methoxy-N-methylamide of formula XIII by methods known in the art:

XIII

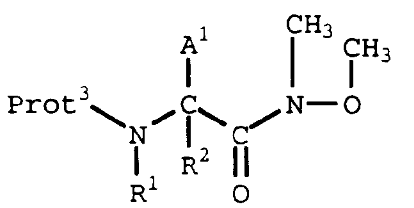

A compound of formula XIII can be reduced to an aldehyde of the formula XIV by methods known in the art. (See, e.g., Fehrentz, *et al.*, Synthesis, 676 (1983)) :

XIV

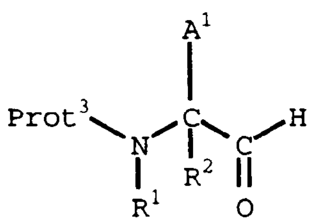

Alternatively, a compound of formula XIV can be prepared by reduction of a compound of formula VII with a reducing agent such as borane, followed by oxidation of the resulting alcohol using, for example, the Swern method of oxidation. (See, e.g., Luly, *et al.*, J. Org. Chem., 52, 1487 (1987); or Stanfield, *et al.*, J. Org. Chem., 46, 4797 (1981)). In yet another alternative, a compound of the formula XIV can be prepared by reduction of an ester analog of a compound of formula VII with a reducing agent such as diisobutylaluminum hydride. See, e.g., Rich, *et al.*, J. Org. Chem., 43, 3624 (1978).

An N-protected aldehyde of formula XIV can be reductively aminated with an amine of the formula VI to form a compound of the formula XV using methods known in the art (e.g., Borch, *et al.*, J. Am. Chem. Soc., 93, 2897 (1971)) :

XV

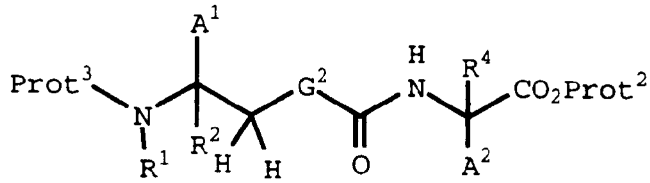

A compound of the formula XV can be treated with a suitable N-deprotecting agent to provide the corresponding free amine of the formula XVI:

XVI

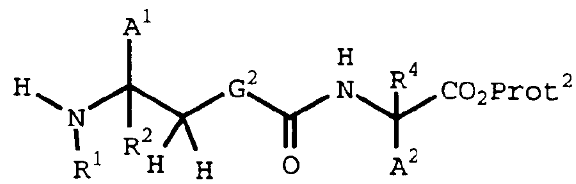

(The $Prot^2$ and $Prot^3$ protecting groups are chosen so that $Prot^3$ can be selectively removed in the presence of $Prot^2$).

A compound of the formula XII or XVI can be condensed with a compound of the formula XVII:

XVII

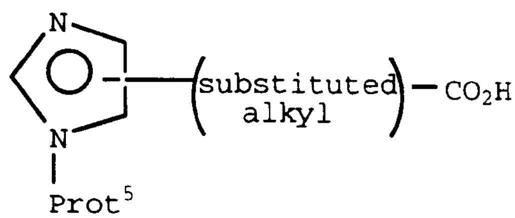

using standard conditions for making an amide bond. (The imidazole group is optionally protected ($Prot^5$) with agents known in the art. For example, trityl, Boc, Cbz, Bom, etc., can be used.) The resulting ester of formula XVIII:

XVIII

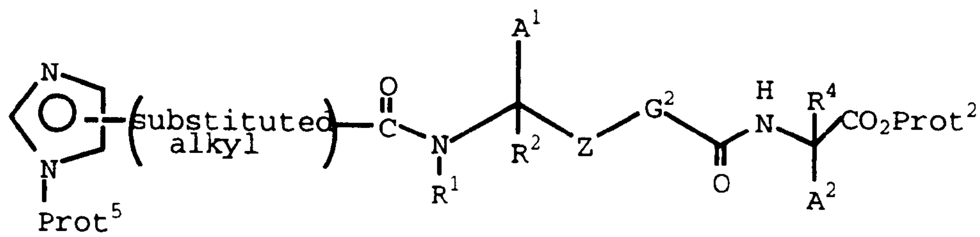

can be deprotected with base. The imidazole, if protected, can be deprotected using standard methods and reagents (for example, acid for trityl, Boc or Bom, hydrogenation for Cbz, etc).

Alternatively, a compound of formula XII or XVI can be condensed with a compound of formula XIX:

XIX

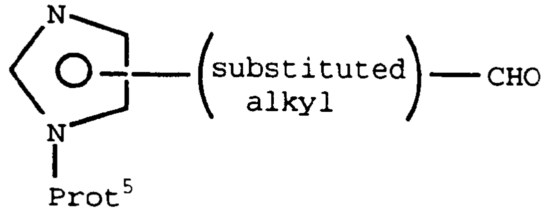

using an acid and a reducing agent (for example, $NaCNBH_3$) and deprotected as described above.

Similarly, a compound of the formula VIII'(a), VIII'(b) or VIII'(c) can be N-deprotected and condensed with a compound of the formula XVII or with a compound of the formula XIX as described above.

A protected imidazole alkanol can be prepared by methods known in the art. See, for example, F. Schneider, Z. Physiol. Chem., 3, 206 - 210 (1961) and C.P. Stewart, Biochem. Journal, 17, 130 - 133 (1923). These can then be oxidized to provide either a compound of the formula XVII or XIX by methods known in the art. Further, a compound which is used like a compound of the formula XIX, can be prepared by methods known in the art as follows:

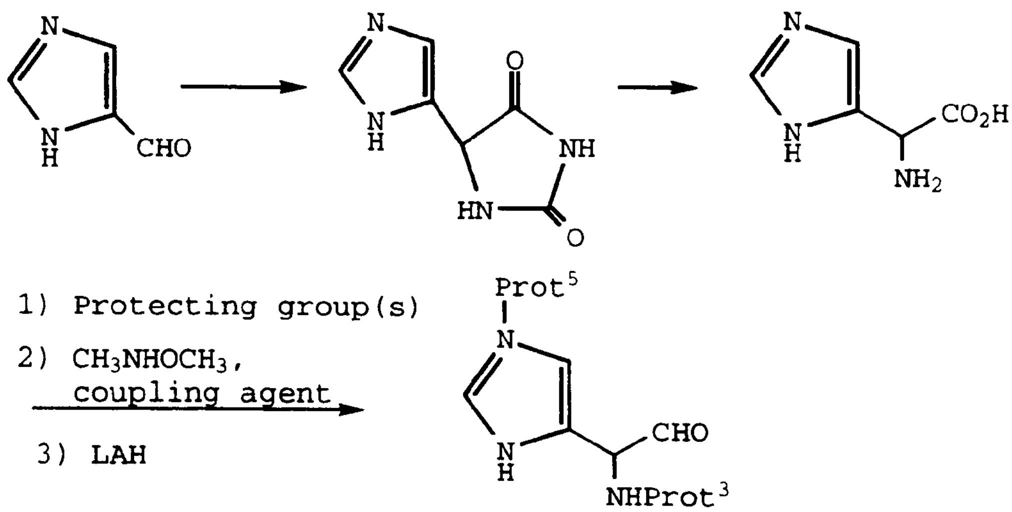


XIX'

Still further, a compound which is also used like a compound of the formula XIX can be prepared from an imidazolyl-alkanoic acid via reduction of the thioester with triethylsilane and palladium on carbon as follows:

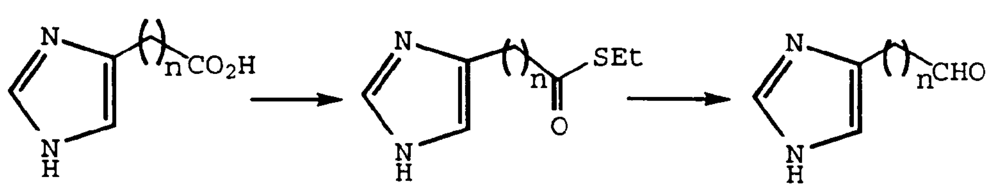

XIX"

A compound of the formula XIX, XIX' or XIX" can further be oxidized also to provide a compound of the formula XVII.)

Some imidazole alkanoic acids containing alkyl substituents on the carbon atoms of the imidazole ring can be prepared by hydrolysis of the corresponding nitriles, which can be prepared by methods known in the art. See, e.g., J. Med. Chem., 19, 923 (1976).

Many compounds of the invention also can be prepared by following one or more of the reaction schemes shown below:

SCHEME 1 (4'-imidazolyl)

I.

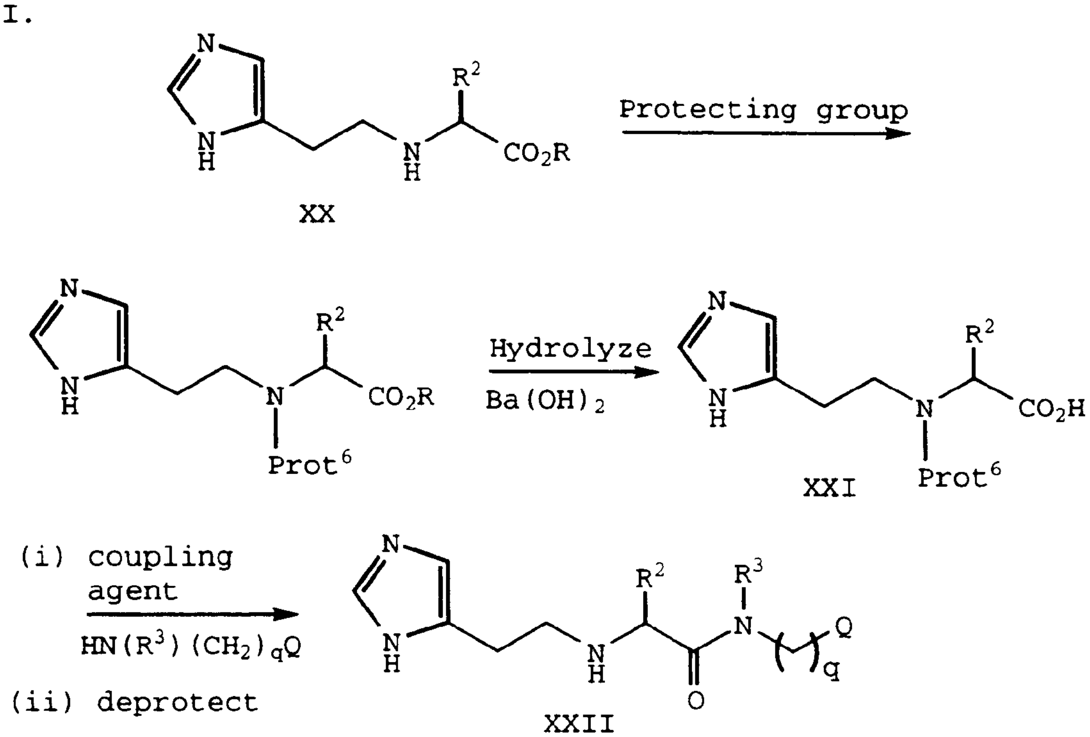


XX

XXI

XXII

Following Scheme 1(I), a compound of formula XX is protected with a protecting group such as t-butoxycarbonyl anhydride. The resulting compound is hydrolyzed with, for example, barium hydroxide ($Ba(OH)_2$) to produce a compound of the formula XXI. A compound of formula XXI is coupled with, for example, a compound of the formula $HN(R^3)(CH_2)_qQ$, and deprotected to produce the desired 4-substituted imidazole of the formula XXII.

A compound of formula XX can be prepared as follows:

a)

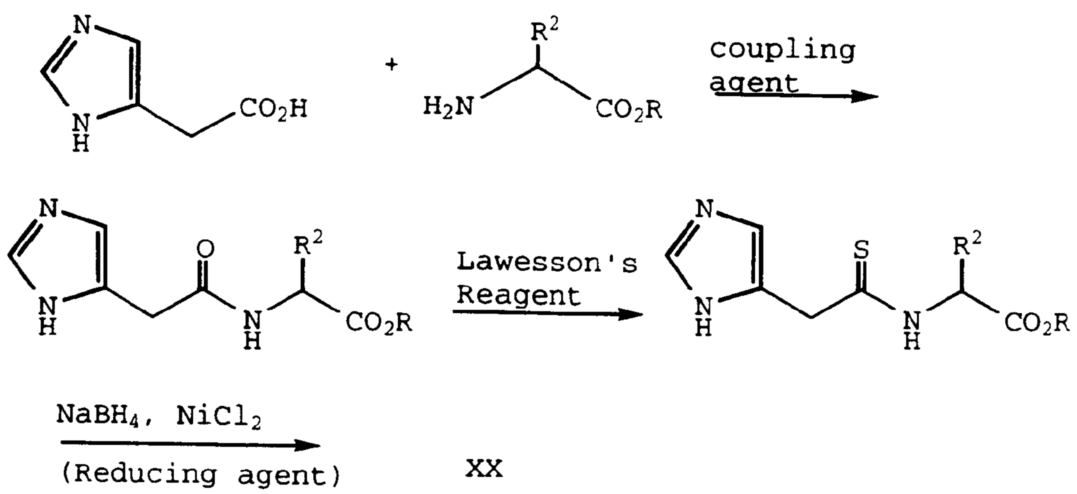


XX

In this reaction scheme 1(I)a, an amide is reduced to a methylene amine using, for example, the two-step method as shown. An example of a suitable coupling agent is EDC. (This two-step reduction scheme may also be used to prepare a compound of the formula XXII from

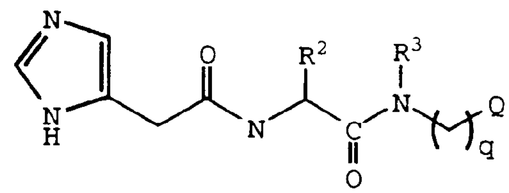

by selective reduction of the secondary amide. Similarly, a compound of the formula XXII" and a compound of the formula XXII"' can be prepared from the corresponding precursor.)

Alternatively, a compound of formula XX can be prepared from histamine in the reductive amination scheme shown below:

b)

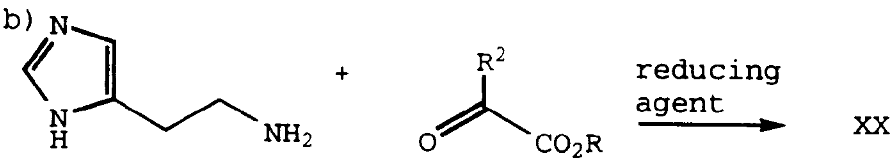


Additionally in Scheme 1, a compound of formula XXI can be further protected if necessary:

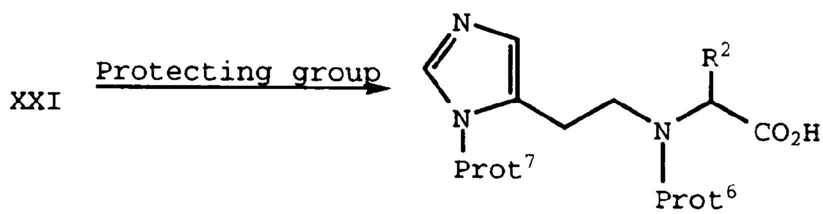


XXI'

A compound of formula XXI' can then be treated as described above for a compound of formula XXI to obtain the desired product, a compound of formula XXII.

Depending on the final product desired (see the definition of G in the general formula), a compound of formula XXI can also be treated as follows:

(1)

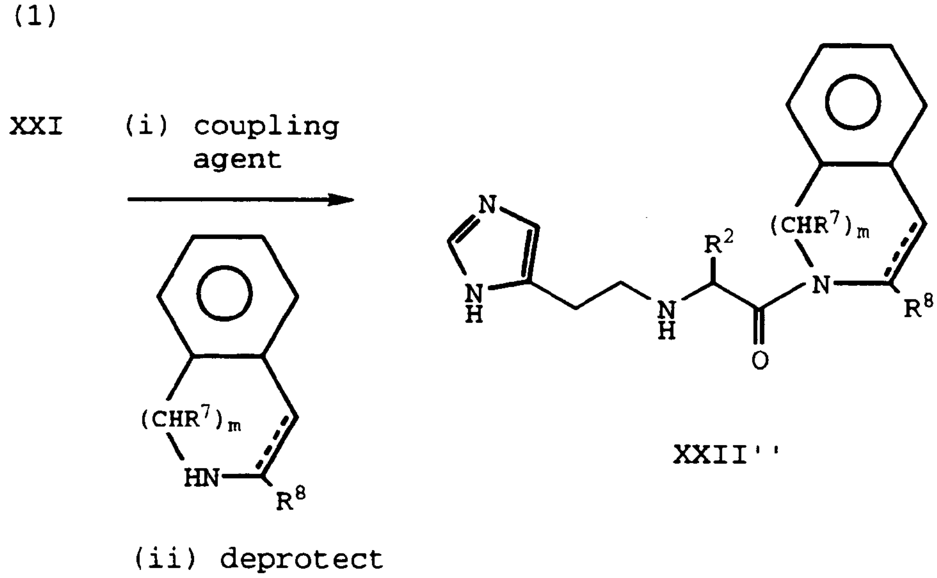


XXII''

(2)

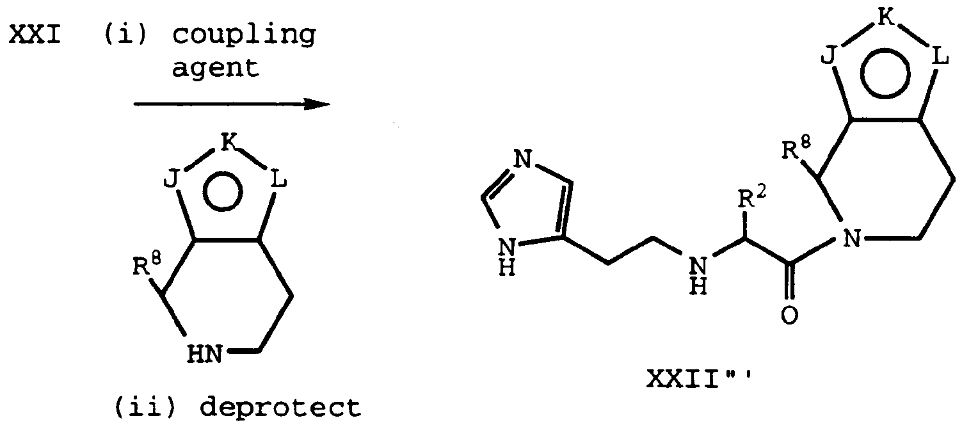


XXI

XXII"'

A 4'-imidazolyl analog may also be prepared by following the "left-to-right" synthesis as shown below in Scheme 1(II).

II.

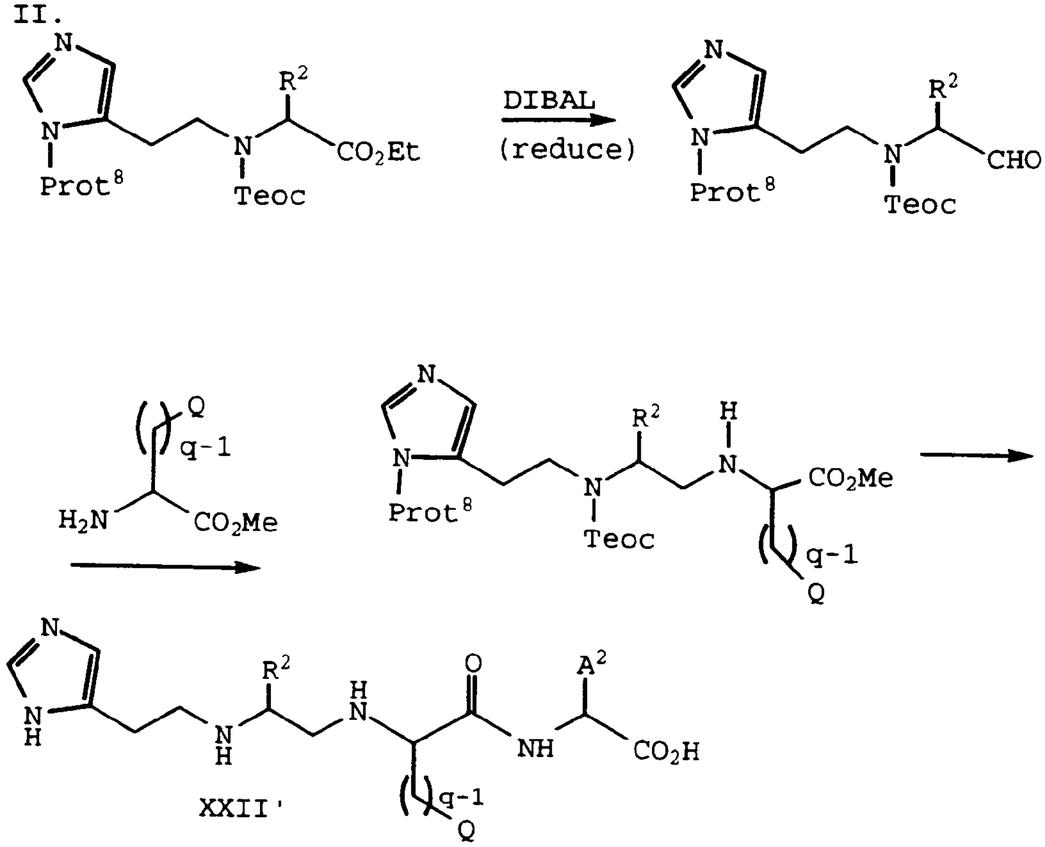


XXII'

III. Additionally, the $N^{\pi}$-alkylated 4'-imidazolyl analogs can be prepared as follows:

XXI

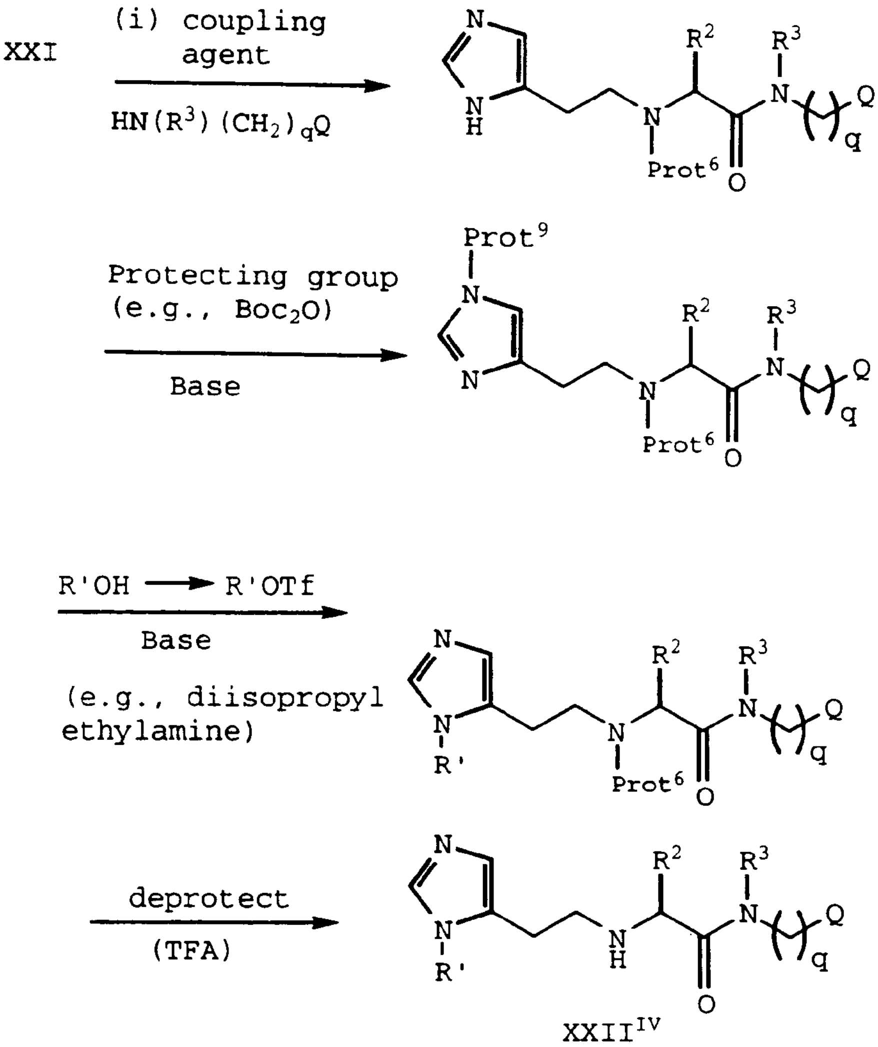


$XXII^{IV}$

IV. An amino-substituted 4'-imidazolyl analog can be prepared as follows:

XX

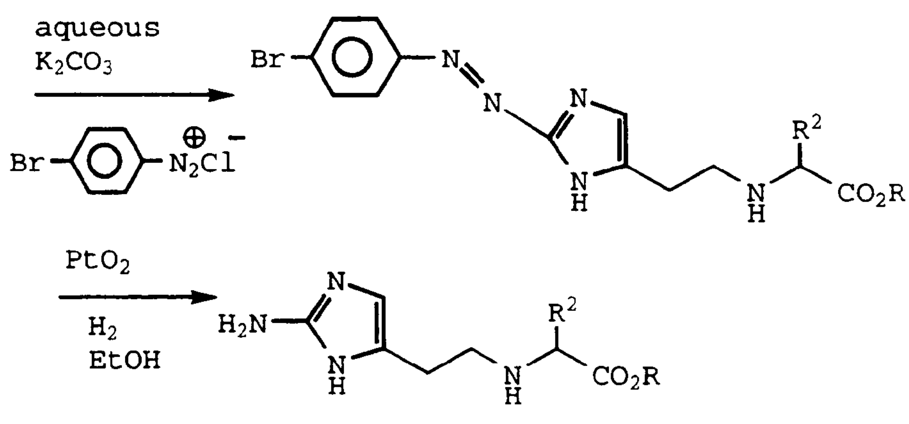


XX'

As shown, a compound of the formula XX is treated with a diazonium salt under basic conditions. The product is hydrogenated and results in a compound of the formula XX'.

Then, a compound of formula XX' can be treated as described above for a compound of the formula XX

to obtain the desired product, a compound of the formula XXII$^{v}$:

XXII$^{V}$

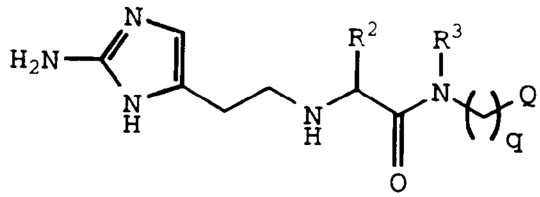

SCHEME 2 (2'-imidazolyl)

A 2'-imidazolyl analog of the formula XXIII:

XXIII

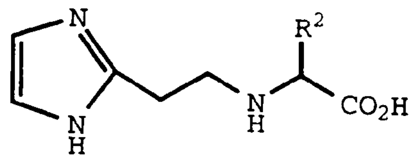

can be protected and then treated like a compound of formula XXI to obtain a compound of formula XXIV:

XXIV

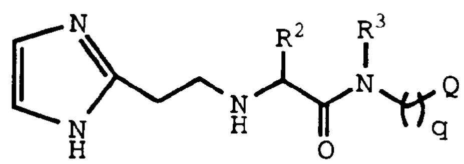

A compound of formula XXIII can be prepared as follows:

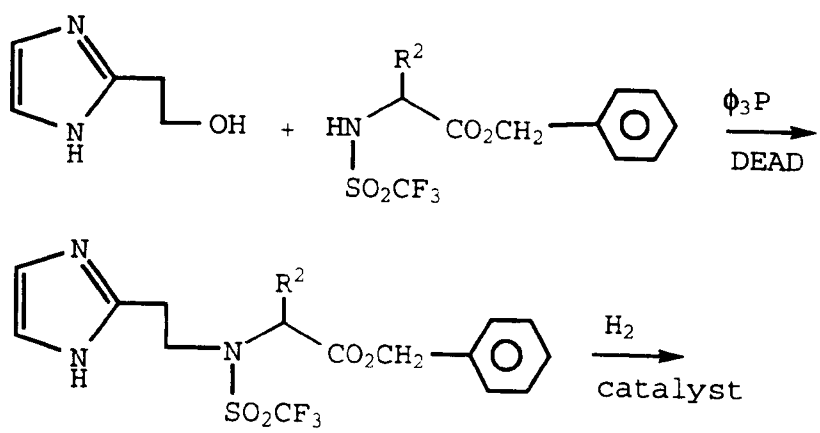

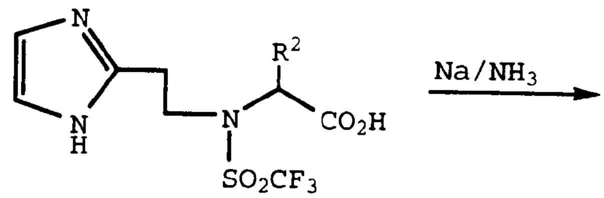


XXIII

In this scheme, the hydroxyl group in the starting material is displaced; the ester in the resulting compound is cleaved as noted; and the $-SO_2CF_3$ group is removed.

A compound of the formula XXIII':

XXIII'

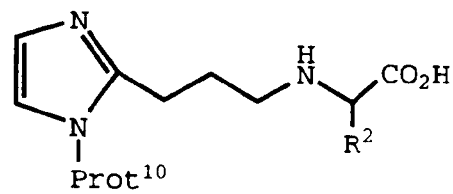

can be prepared as follows:

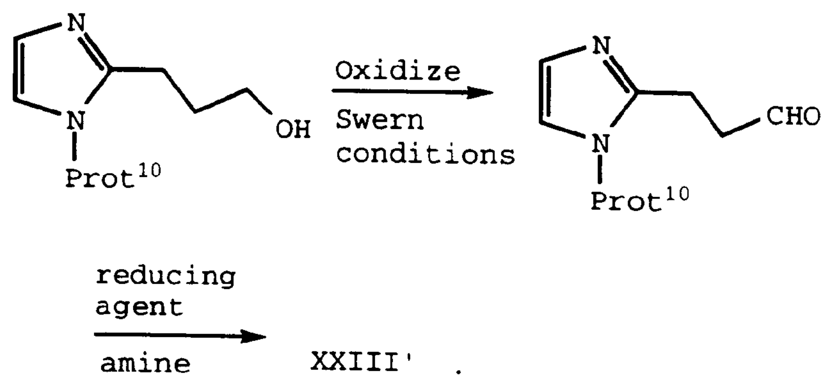


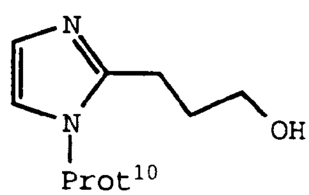

is prepared by the Wittig Reaction as follows:

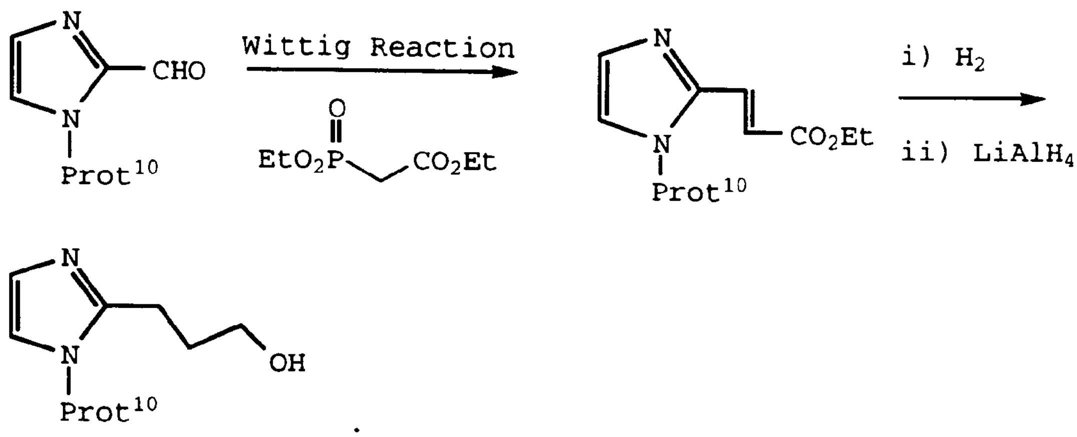


A compound of the formula XXIII' can then be treated like a compound of formula XXI or a compound of formula XXIII to obtain a compound of formula XXIV':

XXIV'

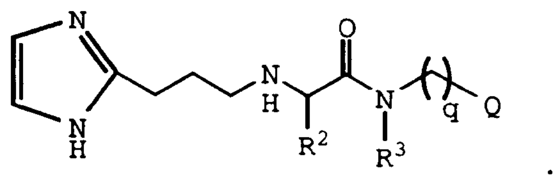

SCHEME 3 (N-imidazolyl)

In methods which are similar to the methods described in Scheme 1, the following N-imidazolyl compounds can be made:

I.

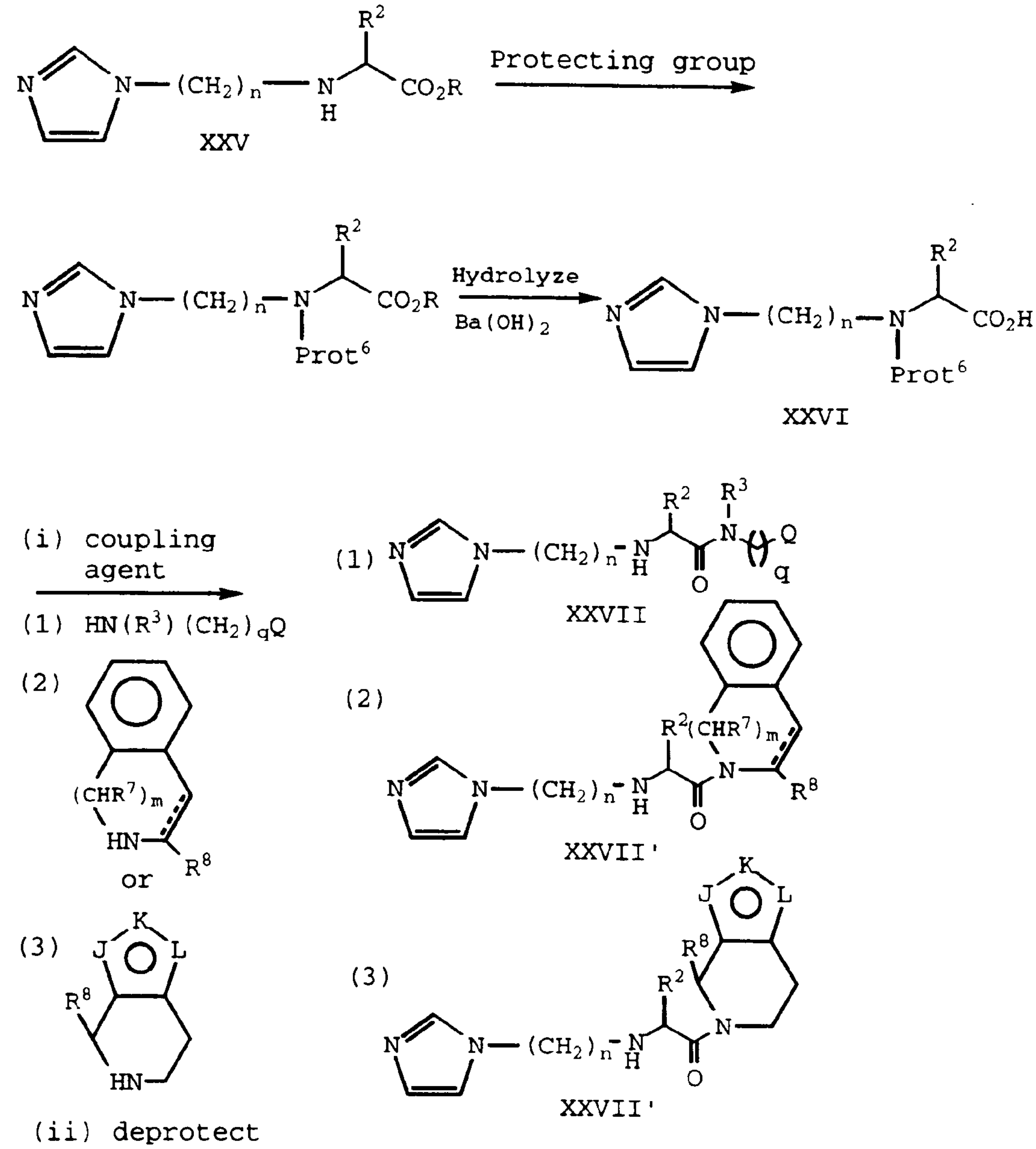


A compound of formula XXV can be prepared as follows:

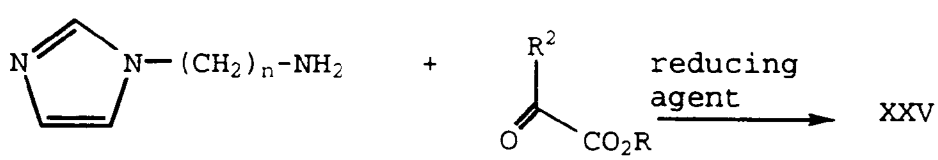


XXVIII

n = 2 or 3

1. The compound of formula XXVIII, when n is 3, is commercially available. The compound of formula XXVIII,

when n is 2, can be prepared as follows:

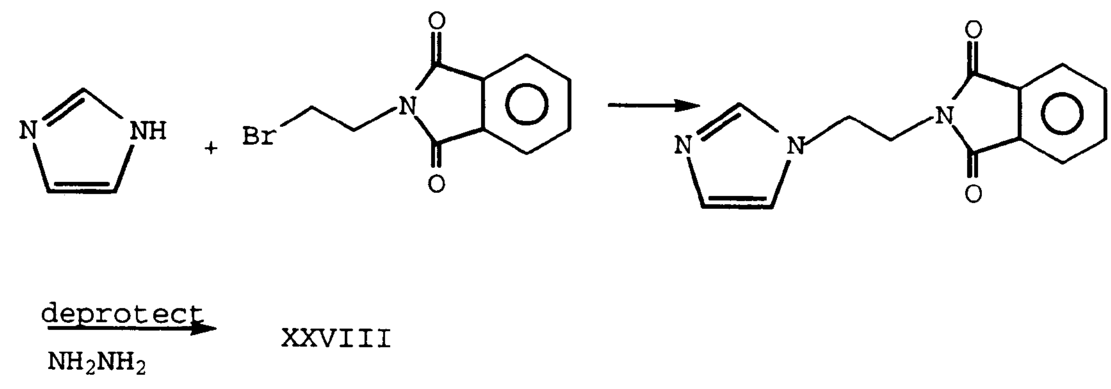


XXVIII

where n is 2

Side-chain protecting groups may be used in these processes with amino acids having reactive functionalities, such as hydroxyl, carboxyl, amino, mercapto, guanidino, imidazolyl, indolyl and the like. The particular protecting groups used for any amino acid residues depend upon the sidechains to be protected and are generally known in the art. Exemplary sidechain protecting groups include acetyl, benzoyl, benzyl, t-butyl and the like for hydroxyl; cyclohexyl, benzyl, methyl, ethyl, t-butyl and the like for carboxyl; benzyl, 4-methylbenzyl, 4-methoxybenzyl, acetyl, acetamidomethyl, triphenylmethyl (trityl) and the like for mercapto; t-butoxycarbonyl (Boc), benzyloxylcarbonyl (Cbz), N-[(9H-Fluoren-9-ylmethoxy)carbonyl] (Fmoc), phthaloyl (Pht), p-toluenesulfonyl (Tos), trifluoroacetyl, 2-(trimethylsilyl)ethoxycarbonyl (Teoc) and the like for amino; 2,4-dinitrophenyl, benzyloxymethyl, Tos, Boc, trityl and the like for imidazolyl; formyl, Cbz, Teoc, 2,2,2-trichloroethyl carbamate (TROC) and the like for indolyl; and tosyl, nitro, bis(1-adamantyloxycarbonyl) and the like for guanidino.

Side-chain protecting groups may be removed, if desired, by, for example, treatment with one or more deprotecting agents in an inert solvent or solvent mixture. For examples of protecting groups and suitable deprotecting agents, see M. Bodansky and A. Bodansky, "The Practice of Peptide Synthesis", Springer-Verlag, Inc. (1984); and T. W. Greene and P.G.M. Wuts, "Protective Groups in Organic Synthesis", Second Edition, John Wiley & Sons, New York, 1991.

The invention will now be further described by the following working example(s), which are preferred embodiments of the invention. All temperatures are in degrees Celsius (°C) unless otherwise indicated. These examples are illustrative rather than limiting.

## Example 1

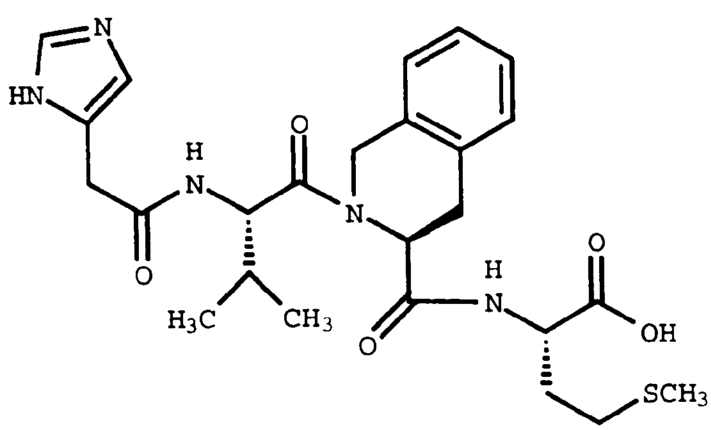

(R*)-N-[[1,2,3,4-Tetrahydro-2-[N-[2-(1H-imidazol-4-ylacetyl)]-L-valyl]3-isoquinolinyl]carbonyl]-L-methionine, trifluoroacetate salt

A. (R*)-N-[[2-[(1,1-Dimethylethoxy)carbonyl]-1,2,3,4-tetrahydro-3-isoquinolinyl]carbonyl]-L-methionine, methyl ester

A solution of (S)-3,4-dihydro-2,3(1H)-isoquinolinedicarboxylic acid, 2-(1,1-dimethylethyl) ester (2.0 g, 7.21 mmol) and L-methionine methyl ester HCl (1.44 g, 7.21 mmol) in 5:15 N-methylpyrrolidinone (NMP) -dichloromethane was stirred at 4°C. N,N-Diisopropylethylamine (DIEA, 1.23 mL, 7.21 mmol) was added, followed by N-hydroxybenzotriazole (HOBT, 974 mg, 7.21 mmol). The mixture was stirred for 5 minutes, then 1-ethyl-3-(3-dimethylaminopropyl)carbodiimide·HCl (EDC, 1.38 g, 7.21 mmol) was added. The reaction mixture was allowed to come to room temperature, stirred overnight and partitioned between dichloromethane and saturated sodium chloride. The organic phase was washed successively with aqueous citric acid, sodium bicarbonate and sodium chloride, dried over magnesium sulfate, and concentrated under vacuum to give 2.64 g (86%) of Compound A.

B. (R*)-N-[(1,2,3,4-Tetrahydro-3-isoquinolinyl)carbonyl]-L-methionine, methyl ester

A solution of Compound A (2 g, 6.22 mmol) in 10 mL dichloromethane was treated at room temperature with 10 mL trifluoroacetic acid (TFA) and 0.5 mL dimethyl sulfide, and stirred for 0.5 hour. The reaction mixture was concentrated under vacuum, dissolved in dichloromethane and concentrated. This procedure was repeated five times to yield Compound B (99%) as a clear glass.

C. (R*)-N-[[2-[N-[(1,1-Dimethylethoxy)carbonyl]-L-valyl]-1,2,3,4-tetrahydro-3-isoquinolinyl]carbonyl]-L-methionine, methyl ester

Compound B (4.73 mmol) was dissolved in dichloromethane (20 mL) and cooled to 0°C. N-t-Butyloxycarbonyl-L-valine (2.06 g, 9.47 mmol), bis(2-oxo-3-oxazolidinyl)phosphinic chloride (BOP chloride, 1.2 g, 4.7 mmol) and DIEA (1.6 mL, 9.5 mmol) were added. The mixture was stirred for 24 hours at 0°C. Additional BOP chloride (1.2 g, 4.7 mmol) and DIEA (0.8 mL, 4.73 mmol) were added and the mixture was stirred for an additional 12 hours at 0°C. The mixture was concentrated and chromatographed (silica gel, eluting with 50% ethyl acetate, 50% hexane). Appropriate fractions were collected and concentrated to yield Compound C as a clear oil (2.4 g, 97%).

D. (R*)-N-[[2-(L-Valyl)-1,2,3,4-tetrahydro-3-isoquinolinyl]carbonyl]-L-methionine, methyl ester, hydrochloride

Compound C (100 mg, 0.192 mmole) was dissolved in 4N hydrochloric acid in dioxane at 0°C for 1 hour. The solution was concentrated under vacuum to afford a pink oil which was chased with diethyl ether (3 x 20 mL) and triturated with diethyl ether to afford 81 mg (91%) of Compound D as a pink solid.

E. (R*)-N-[[1,2,3,4-Tetrahydro-2-[N-[2-(1H-imidazol-4-ylacetyl)]-L-valyl]-3-isoquinolinyl]carbonyl]-L-methionine, methyl ester

To a solution of imidazole-4-acetic acid (34 mg, 0.21 mmol), HOBT (28.3 mg, 0.21 mmol) and Compound D (81 mg, 0.18 mmol) in 2 mL of dichloromethane at 0°C was added N-methyl morpholine (NMM, 44 mL, 0.40 mmol) followed by the addition of EDC (40 mg, 0.21 mmol). The mixture was stirred at room temperature for 18 hours and partitioned between water and dichloromethane. The organic phase was washed with water and brine, and dried (magnesium sulfate, $MgSO_4$). The solvent was removed under vacuum to afford 53 mg (55%) of Compound E.

F. (R*)-N-[[1,2,3,4-Tetrahydro-2-[N-[2-(1H-imidazol-4-ylacetyl)]-L-valyl]-3-isoquinolinyl]carbonyl]-L-methionine, trifluoroacetate salt

To a cooled solution of Compound E (53 mg, 0.1 mmol) in methanol (2 mL) was added a solution of lithium hydroxide (16.5 mg, 0.4 mmol) in 3 mL of water. The mixture was stirred at 0°C for 3 hours and concentrated under vacuum. The residue was dissolved in 5 mL of (80:20) water/acetonitrile/0.1% TFA. The solution was subjected to preparative HPLC on an octadecylsilane column (S-10; 30 x 500) using a gradient system from 80% water: 20% acetonitrile: 0.1% TFA to 55% water: 45% acetonitrile: 0.1% TFA over 50 minutes at 40 mL/min. Under these conditions, Compound F eluted after approximately 37 minutes. Fractions were analyzed by analytical reversed phase HPLC, and those containing product with a minimum purity of 99% were pooled and lyophilized to afford 37 mg (62%) of the title compound as a white solid.

Ms: $(M+H)^+$ 516
Analysis for $C_{25}H_{33}N_5O_5S$ - 0.5 $H_2O$-0.75 $CF_3CO_2H$:
Calculated: C, 52.25; H, 5.58; N, 11.50; S, 5.26; F, 7.02.
Found: C, 52.27; H, 5.45; N, 11.10; S, 5.42; F, 7.05.

## Example 2

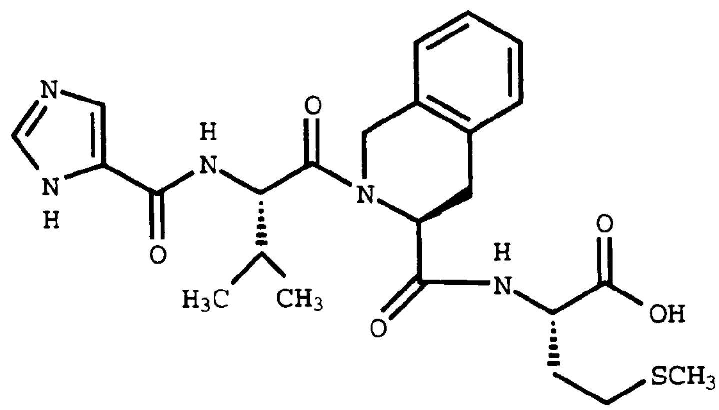

(R*)-N-[[1,2,3,4-Tetrahydro-2-[N-(1H-imidazol-4-ylcarbonyl)-L-valyl]-3-isoquinolinyl]carbonyl]-L-methionine, trifluoroacetate (1:1) salt

A. 5-Hydroxymethyl-1-triphenylmethyl-imidazole

A solution of triphenylmethyl chloride (2.2 g, 7.9 mmol) in dimethylformamide (DMF, 5 mL) was added to a mixture of 4-hydroxymethylimidazole (1 g, 7.4 mmol) and triethylamine (2.5 mL, 18 mmol) in DMF (7.5 mL). The mixture was stirred for 18 hours, filtered and the solid was washed with water and dried under vacuum to afford 2.33 g (93 %) of Compound A as a white solid.

B. (1-Triphenylmethylimidazol-5-yl)carboxaldehyde

A mixture of compound A (2.33 g, 6.85 mmol) in dioxane (100 mL) was heated to 85°C to obtain a homogeneous solution. The heat source was removed, solid manganese dioxide ($MnO_2$, 5 g, 57.5 mmol) was added all at once and the mixture was stirred until the temperature began to drop. The mixture was heated for 6 hours at 85°C, filtered through a pad of Celite® and the filtrate was concentrated. The white solid was dissolved in dichloromethane (20 mL), hexanes was added and the mixture was allowed to stand overnight. The solid was filtered and dried to afford 1.86 g (80%) of Compound B as a white solid.

C. (1-triphenylmethyl-imidazol-5-yl)carboxylic acid

Solid silver oxide ($Ag_2O$, 0.68 g, 3.0 mmol) in water (4 mL) at 55°C was treated with sodium hydroxide (NaOH, 570 mg, 14.4 mmol). Compound B (1 g, 3.0 mmol) was added and the mixture was stirred at 55°C for 15 minutes. The mixture was filtered hot through a pad of Celite® and the filtrate was acidified to pH 3.5 with 6N hydrochloric acid (HCl). The resulting tan solid was collected, rinsed with water and dried under vacuum to afford 110 mg (10 %) of Compound C.

D. (R*)-N-[[1,2,3,4-Tetrahydro-2-[N-(1-triphenylmethyl-imidazol-5-ylcarbonyl)-L-valyl]-3-isoquinolinyl]carbonyl]-L-methionine, methyl ester

Compound D was prepared from Compound C and Compound D from Example 1 as described for compound E from Example 1.

E. (R*)-N-[[1,2,3,4-Tetrahydro-2-[N-(1-triphenylmethyl-imidazol-5-ylcarbonyl)-L-valyl]-3-isoquinolinyl]carbonyl]-L-methionine

To a cooled solution of Compound D (110 mg, 0.145 mmol) in methanol (5 mL) was added a solution of

1N sodium hydroxide (5 mL). The mixture was stirred for 2 hours and concentrated to remove methanol and the residue was partitioned between water and dichloromethane. The aqueous layer was acidified to pH 4 with 6N HCl and extracted with dichloromethane (4x). The combined organic phases were washed with water and brine and dried ($MgSO_4$). The solvent was removed to afford 106 mg (98%) of crude Compound E.

F. (R*)-N-[[1,2,3,4-Tetrahydro-2-[N-(1H-imidazol-4-ylcarbonyl)-L-valyl]-3-isoquinolinyl]carbonyl]-L-methionine, trifluoroacetate (1:1) salt

To a degassed solution of triethylsilane (0.23 mL, 1.4 mmol) and TFA (5 mL) in dichloromethane (5 mL) was added Compound E. The mixture was stirred for 4 hours, concentrated and the residue was dissolved in 25 mL of (80:20) water/acetonitrile/0.1 % TFA. The mixture was centrifuged and decanted to remove insoluble materials. The solution was subjected to preparative HPLC on an octadecylsilane column (S-10; 30 x 500) using a gradient system from 80% water:20% acetonitrile:0.1% TFA to 55% water:45% acetonitrile:0.1% TFA over 50 minutes at 40 mL/minute. Under these conditions, Compound F eluted after approximately 29 minutes. Fractions were analyzed by analytical reversed phase HPLC and those containing product with a minimum purity of 99% were pooled and lyophilized to afford 14 mg (19.6%) of the title compound as a white solid.
MS: $(M+H)^+$ 502

| Analysis for $C_{24}H_{31}N_5O_5S$ - 0.57 $H_2O$-1.25 $CF_3CO_2H$: | | | |
|---|---|---|---|
| Calculated: | C, 48.52; | H, 5.15; | N, 10.67. |
| Found: | C, 48.52; | H, 4.96; | N, 10.52. |

## Example 3

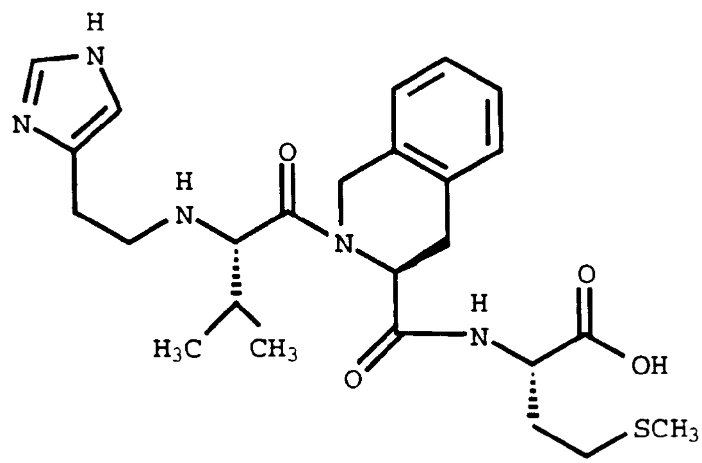

(R*)-N-[[1,2,3,4-Tetrahydro-2-[N-(2-(1H-imidazol-4-yl)ethyl]-L-valyl]-3-isoquinolinyl]carbonyl]-L-methionine, trifluoroacetate (2:5) salt

A. S-ethyl-(1-ethoxycarbonyl-imidazol-4-yl)thioacetic acid

To a mixture of 4-imidazole acetic acid (2 g, 12 mmol) and triethylamine (5.1 mL, 37 mmol) in dichloromethane (40 mL) at 0°C was added a solution of ethylchloroformate (2.5 mL, 26 mmol) in dichloromethane (10 mL) over 20 minutes to maintain the reaction temperature between 0-5°C. The mixture was stirred for 15 minutes and ethanethiol (2.0 mL, 28 mmol) and 4-(N,N-dimethylamino)pyridine (DMAP) (100 mg) were added consecutively. The mixture was stirred for 30 minutes at 0°C, warmed to room temperature, washed with water and brine, dried ($MgSO_4$) and evaporated. The residue was absorbed on a plug of silica gel and the plug was eluted with dichloromethane to remove the unreacted ethanethiol followed by 10% methanol:dichloromethane. Fractions containing product were combined and evaporated to yield 0.55 g of Compound A as an oil.

B. (1-ethoxycarbonyl-imidazol-4-yl)acetaldehyde

To a degassed solution of Compound A (0.5 g, 2.1 mmol) and triethylsilane (1.64 mL, 10.3 mmol) in acetone (15 mL) was added 10% palladium on carbon (100 mg). The mixture was stirred 1 hour, filtered through a pad of Celite® and the filtrate concentrated to afford 0.37 g (98%) of Compound B as an oil.

C. (R*)-N-[[1,2,3,4-Tetrahydro-2-[N-(2-(1H-imidazol-4-yl)ethyl]-L-valyl]-3-isoquinolinyl]carbonyl]-L-methionine

To a solution of Compound D from Example 1 (83 mg, 0.18 mmol) and Compound B (100 mg) in methanol (5 mL) was added solid sodium cyanoborohydride ($NaBH_3CN$, 23 mg, 0.36 mmol). After 10 minutes, additional amounts of $NaBH_3CN$ (23 mg, 0.36 mmol) and acetic acid (21 μL, 0.36 mmol) were added and the mixture was stirred 30 minutes. Two additional portions of Compound B (150 mg), $NaBH_3CN$ (46 mg, 0.72 mmol) and acetic acid (41 μL, 0.72 mmol) were added over 1 hour. The mixture was stirred for 18 hours and evaporated, and the residue was dissolved in methanol (5 mL). NaOH (1N, 4 mL) was added, the solution was stirred 1 hour and the methanol was evaporated. The aqueous phase was diluted to 10 mL with water, and 1 mL of TFA was added. The mixture was sitrred 10 minutes, concentrated, and the residue was dissolved in 25 mL of (80:20) water:acetonitrile containing 0.1% TFA. The mixture was centrifuged and decanted to remove insoluble materials. The solution was subjected to preparative HPLC on an octadecylsilane column (S-10; 30 x 500) using a gradient system from 80% water: 20% acetonitrile: 0.1% TFA to 55% water: 45% acetonitrile: 0.1% TFA over 50 minutes at 40 mL/minute. Under these conditions, the product eluted after 32 minutes. Fractions containing product with a minimum purity of 99% were pooled and lyophilized to afford 22 mg of the title compound (24%) as a white solid.
MS: $(M+H)^+$ 502

| Analysis for $C_{25}H_{35}N_5O_4S$ - 0.3 $H_2O$-2.5 $CF_3CO_2H$: | | | |
|---|---|---|---|
| Calculated: | C, 45.49; | H, 4.84; | N, 8.84. |
| Found: | C, 45.49; | H, 4.68; | N, 8.77. |

**Example 4**

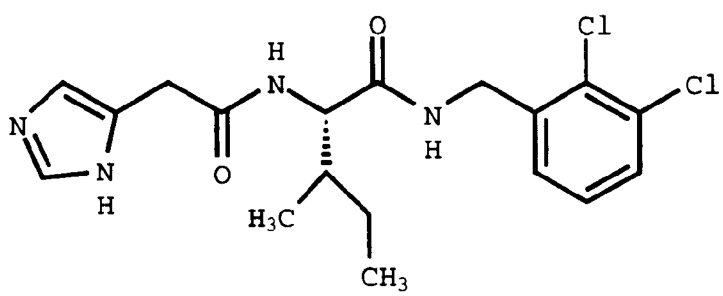

N-[(2,3-Dichlorophenyl)methyl]-N²-(1H-imidazol-4-ylacetyl)-L-isoleucinamide

A. N-[(1,1-Dimethylethoxy)-carbonyl]-L-isoleucine, 2,3-dichlorobenzylamide

A suspension of Boc-isoleucine hemihydrate (0.61 g, 2.5 mmol), 2,3-dichlorobenzylamine hydrochloride (0.36 g, 1.75 mmol), NMM (0.22 mL, 2.0 mmol), EDC (0.48 g, 2.5 mmol) and HOBT (0.34 g, 2.5 mmol) in 10 mL of 9:1 THF:DMF was stirred 13 hours. Half saturated sodium hydrogen carbonate and ethyl acetate were added, the mixture was partitioned, the organic phase was washed again with aqueous sodium hydrogen carbonate, twice with aqueous 5% potassium hydrogen sulfate and once with brine, dried ($MgSO_4$) and evaporated to afford 0.90 g of foamy gum. Flash chromatography on silica with 25% ethyl acetate/hexanes provided 0.61 g (92%) of Compound A as a white solid.

B. L-isoleucine, 2,3-dichlorobenzylamide, hydrochloride

To Compound A (0.60 g, 1.5 mmol) was added 2 mL of 4N hydrogen chloride in dioxane. The solution was stirred for 1.5 hours, evaporated and chased with ether, and the resulting white solid was suspended in ether, filtered, rinsed with ether and dried to afford 0.47 g (94%) of Compound B as a white solid.

C. N-[(2,3-Dichlorophenyl)methyl]-$N^2$-(1H-imidazol-4-ylacetyl)-L-isoleucinamide

A mixture of Compound B (0.12 g, 0.35 mmol), imidazole-4-acetic acid hydrochloride (0.12 g, 0.71 mmol), NMM (0.12 mL, 1.0 mmol), EDC (0.138 g, 0.71 mmol) and HOBT (0.095 g, 0.71 mmol) in 3 mL of DMF was stirred overnight. The DMF was removed under vacuum with gentle warming, half saturated sodium hydrogen carbonate was added, and the mixture was extracted twice with 10% isopropanol/methylene chloride. The combined organic phases were washed with brine, dried ($MgSO_4$) and evaporated to afford 0.18 g of off-white gelatinous solid which was subjected to flash chromatography on silica with 5% methanol/methylene chloride/0.5% ammonium hydroxide followed by 10% methanol/methylene chloride/1% ammonium hydroxide. Fractions containing clean product were evaporated, the residue was taken up in 10% isopropanol/methylene chloride, dried ($MgSO_4$) and evaporated to afford 0.13 g (94%) of white solid, which was rinsed with ether to provide the title compound, mp 167-169°C.

| Analysis calculated for $C_{18}H_{22}N_4O_2Cl_2$-0.20 $H_2O$-0.07 $C_4H_{10}O$: | | | | |
|---|---|---|---|---|
| Calculated: | C, 54.07; | H, 5.73; | N, 13.80; | Cl, 17.46. |
| Found: | C, 54.06; | H, 5.58; | N, 13.86; | Cl, 17.06. |

**Example 5**

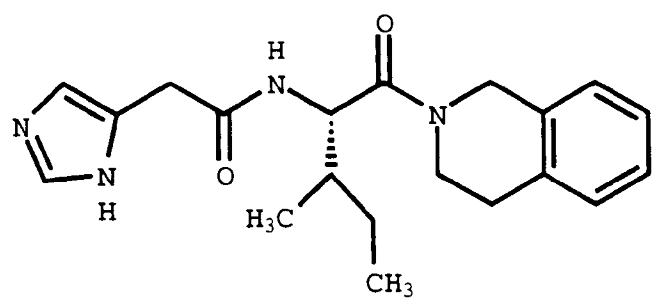

(S)-N-[2-Methyl-1-[(1,2,3,4-tetrahydro-2-isoquinolinyl)carbonyl]butyl]-1H-imidazole-4 acetamide

A. 2-[N-[(1,1-Dimethylethoxy)-carbonyl]-L-isoleucyl]-1,2,3,4-tetrahydroisoquinoline

Compound A was prepared as a clear gum from Boc-isoleucine hemihydrate and 1,2,3,4-tetrahydroisoquinoline as described for Compound A of Example 4.

B. 2-(L-isoleucyl)-1,2,3,4-tetrahydroisoquinoline, hydrochloride

To Compound A (1.60 g, 4.62 mmol) was added 6 mL of 4N HCl in dioxane. The solution was stirred 2.25 hours, evaporated and chased with ether twice to afford 1.29 g (98%) of Compound B as a foamy white solid.

C. (S)-N-[2-Methyl-1-[(1,2,3,4-tetrahydro-2-isoquinolinyl)carbonyl]butyl]-1H-imidazole-4 acetamide

The title compound was prepared as an off-white solid from Compound B and imidazole-4-acetic acid hydrochloride as described for Example 4, mp 73-78°C.

| Analysis calculated for $C_{20}H_{26}N_4O_2$-0.4 $H_2O$: | | | |
|---|---|---|---|
| Calculated: | C, 66.42; | H, 7.47; | N, 15.49. |
| Found: | C, 66.35; | H, 7.59; | N, 15.14. |

**Example 6**

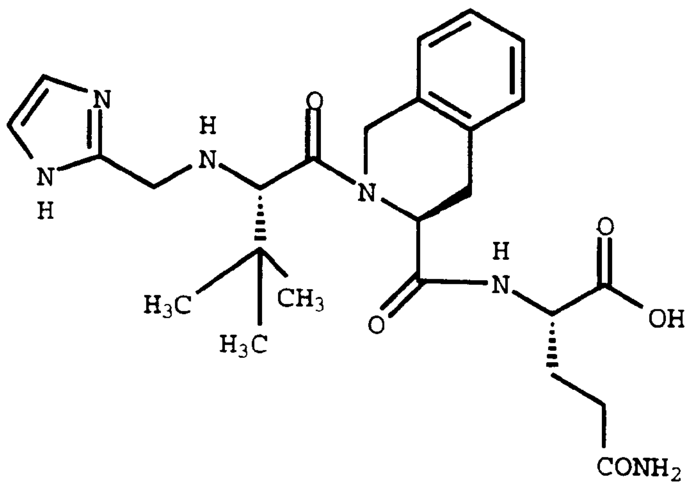

(R*)-N-2-[[1,2,3,4-Tetrahydro-2-[N-(1H-imidazol-2-ylmethyl)-3-methyl-L-valyl]-3-isoquinolinyl]carbonyl]-L-glutamine, trifluoroacetate (1:2) salt

A. 1-Triphenylmethyl-imidazole-2-carboxaldehyde

To a solution of imidazole-2-carboxaldehyde (700 mg, 7.28 mmol) and DIEA (3.05 mL, 17.5 mmol) in DMF (8 mL) was added a solution of triphenylmethyl chloride (2.23 g, 8.00 mmol) in DMF (25 mL) dropwise. The mixture was stirred for 16 hours, poured into water and extracted 3 times with ethyl acetate. The combined organic layers were washed twice with water and once with brine, dried ($MgSO_4$) and concentrated, and the residue was chromatographed (silica gel, 50% ethyl acetate/hexane) to yield Compound A as a slightly yellow solid (800 mg, 39 %), $(M+H)^+$ $339^+$.

B. 3-Methyl-N-[(phenylmethoxy)carbonyl]-L-valine

To a solution of L-tert-leucine (10.2 g, 77.6 mmol) in aqueous sodium hydroxide (2N, 40 mL) at 0°C was added benzyl chloroformate (12.1 mL, 85.4 mmol) and sodium hydroxide (2N, 40 mL) alternatively in 5 portions over 30 minutes. The cold bath was removed and after 1 hour the pH of the mixture was adjusted to 10. The mixture was extracted twice with ether. The aqueous layer was cooled to 0°C and acidified (pH 2) with 5 N hydrochloric acid and extracted 3 times with ether. The organic layer was dried (magnesium sulfate) and concentrated to afford Compound B as a thick oil (18.2 g, 88%). MS (CI) : $(M+H)^+ = 266^+$.

C. (S)-1,2,3,4-Tetrahydro-2-[3-methyl-N-[(phenylmethoxy)carbonyl]-L-valyl]-3-isoquinolinecarboxylic acid, methyl ester

To a solution of Compound B (5.31 g, 20 mmol) in dichloromethane (80 mL) at 0°C under argon were sequentially added DIEA (10.6 mL, 60 mmol), benzotriazol-1-yloxytris-(dimethylamino)phosphonium hexafluorophosphate (BOP, 5.08 g, 20 mmol) and (S)-1,2,3,4-tetrahydroisoquinoline-3-carboxylic acid, methyl ester, hydrochloride (5.68 g, 25 mmol). The reaction mixture was allowed to warm to 5°C over 2 hours and stirred 16 hours. The mixture was washed with 1N hydrochloric acid, saturated sodium bicarbonate and brine. The organic layer was dried (magnesium sulfate), filtered and concentrated to afford an oil. Purification by flash silica gel column chromatography eluting with 20% ethyl acetate in hexanes afforded Compound C (4.0 g, 45%). MS: (CI) $(M+H)^+ = 439^+$.

D. (S)-1,2,3,4-Tetrahydro-2-[3-methyl-N-[(phenylmethoxy)carbonyl]-L-valyl]-3-isoquinolinecarboxylic acid

To a solution of Compound C (830 mg, 1.9 mmol) in THF/methanol (4 mL/5 mL) at room temperature was added lithium hydroxide (1N, 1.9 mL). After 1 hour, additional lithium hydroxide (1N, 1.5 mL) was added. After 2 hours, sodium hydroxide (1N, 1.9 mL) was added. After another hour, solvent was removed and the residue was partitioned between 1N hydrochloric acid and ethyl acetate. The organic layer was dried (magnesium sulfate) and concentrated to afford Compound D (820 mg).

E. (R*)-$N^2$-[[1,2,3,4-Tetrahydro-2-[3-methyl-N-[(phenylmethoxy)carbonyl]-L-valyl]-3-isoquinolinyl]carbonyl]-L-glutamine, 1,1-dimethylethyl ester

To a solution of Compound D (0.9 g, 2.1 mmol), L-glutamine t-butyl ester hydrochloride (0.50 g, 2.1 mmol), and BOP (0.93 g, 2.1 mmol) in 3:1 $CH_3CN$:DMF (26 mL) was added DIEA (1.1 mL, 6.3 mmol). The solution was stirred for 16 hours, quenched with 1N HCl (100 mL) and extracted 4 times with ethyl acetate, and the combined organic extracts were washed 3 times with 10% LiCl, dried ($MgSO_4$), filtered and concentrated. The residue was purified by flash chromatography (1/1 hexane/acetone) to afford Compound E (1.1 g, 87%) as a white solid, mp 60-68°C. MS: $(M+H)^+$ 609.

F. (R*)-$N^2$-[[1,2,3,4-Tetrahydro-2-(3-methyl-L-valyl)-3-isoquinolinyl]-carbonyl]-L-glutamine, 1,1-dimethylethyl ester, hydrochloride

Palladium hydroxide on carbon (10%, 91 mg) was added to a solution of Compound E (0.91 g, 1.5 mmol) in THF (9.1 mL) with 1N HCl (1.5 mL). A balloon containing hydrogen was attached to the flask and the mixture was stirred for 3 hours. The mixture was filtered through Celite® and the filtrate concentrated under vacuum to afford Compound F (0.77 g, 100%) which was used without further purification. MS: $(M+H)^+$ 475 (free base).

G. (R*)-N-2-[[1,2,3,4-Tetrahydro-2-[N-(1-triphenylmethyl-imidazol-2-ylmethyl)-3-methyl-L-valyl]-3-isoquinolinyl]carbon-yl]-L-glutamine, 1,1-dimethylethyl ester

Compound A (332 mg, 0.97 mmol) and Compound F (250 mg, 0.489 mmol) were dissolved in methanol (20 mL) and glacial acetic acid (0.4 mL). Sodium cyanoborohydride (31 mg, 0.489 mmol) was added portionwise over 15 minutes. The mixture was stirred for 16 hours and cooled to 0°C, and saturated sodium hydrogen carbonate (30 mL) was slowly added. The mixture was concentrated and extracted with ethyl acetate. The ethyl acetate layer was washed with water and brine, dried ($MgSO_4$), concentrated and chromatographed (silica gel, 95% methylene chloride, 5% methanol). Fractions containing the desired compound were collected, concentrated and purified by preparative HPLC (YMC S-10 ODS column, 30 X 500 mm; 18% to 63% methanol:water containing 0.1% TFA over 40 minutes at 20 mL/min). Fractions containing the desired product were combined, concentrated, extracted twice with methylene chloride, dried ($MgSO_4$) and concentrated to yield Compound G as a clear oil (130 mg, 33%), $(M+H)^+$ $797^+$.

H. (R*)-N-2-[[1,2,3,4-Tetrahydro-2-[N-(1H-imidazol-2-ylmethyl)-3-methyl-L-valyl]-3-isoquinolinyl]carbon-yl]-L-glutamine, trifluoroacetate (1:2) salt

Compound G (130 mg, 0.34 mmol) was dissolved in methylene chloride (10 mL), TFA (10 mL) and triethylsilane (0.5 mL), and the mixture was stirred for 1 hour. The mixture was concentrated, dissolved in methylene chloride (60 mL) and concentrated. This procedure was repeated five times to yield the crude product as a white sticky solid in quantitative yield. The crude solid was purified by preparative HPLC (YMC S-10 ODS column, 30 X 500 mm; 18% to 63% methanol:water containing 0.1% TFA over 40 minutes at 20 mL/min). Fractions containing the desired product were combined and lyophilized to provide 53 mg (17 %) of the title compound, mp 89-90°C.
$[\alpha]_D 25°$ = - 34.0° (c = 0.1, methanol)

| Analysis calculated for $C_{25}H_{34}N_6O_5$-3.20 $CF_3CO_2H$-2.60 $H_2O$: | | | |
|---|---|---|---|
| Calculated: | C, 41.43; | H, 4.69; | N, 9.23. |
| Found: | C, 41.43; | H, 4.25; | N, 8.92. |

Example 7

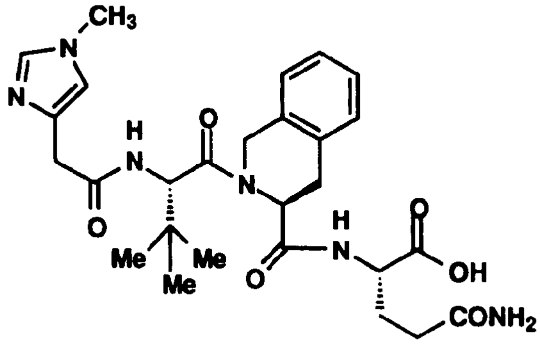

(R*)-N2-[[1,2,3,4-Tetrahydro-2-[N-[(1-methyl-1H-imidazol-4-yl)acetyl]-3-methyl-L-valyl]-3-isoquinolinyl]carbonyl]-L-glutamine, trifluoroacetate (1:2) salt

A. (R*)-N2-[[1,2,3,4-Tetrahydro-2-[N-[(1-methyl-1H-imidazol-4-yl)acetyl]-3-methyl-L-valyl]-3-isoquinolinyl] carbonyl]-L-glutamine, 1,1-dimethylethyl ester

Compound A was prepared from Compound F of Example 6 and 1-methylimidazole-4-acetic acid hydrochloride as described for Compound E of Example 1, $(M+H)^+$ $596^+$.

B. (R*)-N2-[[1,2,3,4-Tetrahydro-2-[N-[(1-methyl-1H-imidazol-4-yl)acetyl]-3-methyl-L-valyl]-3-isoquinolinyl]carbonyl]-L-glutamine, trifluoroacetate (1:2) salt

A mixture of TFA (2 mL) and Compound A (100 mg, 0.168 mmol) in dichloromethane (5 mL) was stirred two hours and concentrated. The residue was dissolved in 25 mL of (80:20) water/acetonitrile/0.1% TFA and the mixture was centrifuged and decanted to remove insoluble materials. The solution was subjected to preparative HPLC on an octadecylsilane column (S-10; 30 x 500) using a gradient system from 80% water:20% acetonitrile:0.1% TFA to 55% water:45% acetonitrile:0.1% TFA over 50 minutes at 50 mL/minute. Under these conditions, the peptide eluted after approximately 18.2 minutes. Fractions were analyzed by analytical reverse phase HPLC and those containing enriched product were pooled and lyophilized. The residue was dissolved in water (5mL), 1N NaOH was added to pH 10 and the solution was stirred 15 minutes and acidified to pH 2.5 with TFA. The solution was applied to a C-18 sep-pak cartridge (2g), which was prewashed with 2 column volumes of acetonitrile and 10 column volumes of water. The cartridge was eluted with water followed by 10% aqueous acetonitrile:0.1% TFA (10 mL) and then 30% aqueous acetonitrile:0.1% TFA. Fractions containing clean product were combined to afford 27 mg (30%) of the title compound as a white solid.
MS: M+H = 556, M+ Li = 562.

| Analysis calculated for $C_{27}H_{36}N_6O_6Li$-1.0 $CH_3OH$: 1.33 $H_2O$: | | | |
|---|---|---|---|
| Calculated: | C, 54.45; | H, 6.96; | N, 15.87. |
| Found: | C, 54.55; | H, 6.78; | N, 15.48. |

Example 8

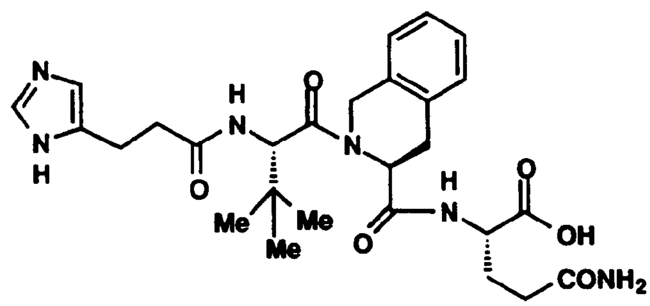

(R*)-$N^2$-[[1,2,3,4-Tetrahydro-2-[N-[3-(1H-imidazol-4-yl)-1-oxopropyl]-N-methyl-L-valyl]-3-isoquinolinyl]-carbonyl]-L-glutamine, trifluoroacetate (1:2) salt

A. (R*)-$N^2$-[[1,2,3,4-Tetrahydro-2-[N-[3-(1-triphenylmethyl-imidazol-4-yl)-1-oxopropyl]-N-methyl-L-valyl]-3-isoquinolinyl]-carbonyl]-L-glutamine, 1,1-dimethylethyl ester

Compound A was prepared from Compound F of Example 6 and (N-triphenylmethylimidazol-4-yl)propionic acid as described for Compound E of Example 1, $(M+H)^+$ $596^+$.

B. (R*)-$N^2$-[[1,2,3,4-Tetrahydro-2-[N-[3-(1H-imidazol-4-yl)-1-oxopropyl]-N-methyl-L-valyl]-3-isoquinolinyl]-carbonyl]-L-glutamine, trifluoroacetate (1:2) salt

The title compound was prepared as a white solid from Compound A as described for Compound F of Example 2.
MS: M+H = 541.

Analysis calculated for $C_{27}H_{36}N_6O_6$-2.13 $CF_3CO_2H$ : 0.31 $H_2O$:

Calculated: C, 47.92; H, 4.91; N, 10.73.

Found: C, 47.58; H, 5.20; N, 11.11.

Example 9

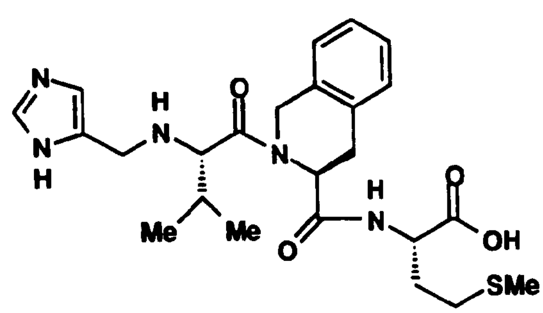

(R*)-N-[[1,2,3,4-Tetrahydro-2-[N-(1H-imidazol-4-ylmethyl)-L-valyl]-3-isoquinolinyl]carbonyl]-L-methionine, trifluoroacetate (1:1) salt

A. (R*)-N-[[2-[N-[(1,1-Dimethylethoxy)carbonyl]-L-valyl]-1,2,3,4-tetrahydro-3-isoquinolinyl]carbonyl]-L-methionine

A mixture of Compound C of Example 1 (1.0 g, 1.91 mmol) in methanol (20 mL) and 5N NaOH (5 mL) was stirred for 3 hours, neutralized to pH 7 using 5N HCl (4.8 mL) and concentrated. The residue was dissolved in ethyl acetate (100 mL) and water (100 mL), the layers were separated, and the aqueous layer was acidified to pH 2 using 5N HCl and re-extracted with ethyl acetate (2 X 50 mL). The combined organic layers were washed with brine (100 mL), dried ($MgSO_4$) and concentrated to yield a clear oil. This oil was dissolved in methylene chloride (10 mL), TFA (10 mL) and triethylsilane (0.5 mL) were added and the mixture was stirred for 40 minutes and concentrated. The residue was dissolved in methylene chloride (50 mL) and concentrated five times to yield the crude product as a white sticky solid (800 mg). Half of this crude solid (400 mg) was purified by preparative HPLC (YMC-C18 column, 2.2 X 25 cm, 5 micron, 120 angstrom; solvent A, 0.1% TFA in water; solvent B, 0.1% TFA in acetonitrile: 25-35% B in 40 minutes, flow rate 9 mL/minute). Fractions containing the desired product were combined and lyophilized to provide Compound A (230 mg, 54%), mp 129-130°C.

B. (R*)-N-[[1,2,3,4-Tetrahydro-2-[N-(1-triphenylmethyl-imidazol-4-ylmethyl)-L-valyl]-3-isoquinolinyl]carbonyl]-L-methionine

Compound B was prepared in 56% yield from Compound A and Compound B of Example 2 as described for Compound G of Example 6. It was carried on without purification.

C. (R*)-N-[[1,2,3,4-Tetrahydro-2-[N-(1H-imidazol-4-ylmethyl)-L-valyl]-3-isoquinolinyl]carbonyl]-L-methionine, trifluoroacetate (1:1) salt

The title compound was prepared as a white solid from Compound B as described for Compound H of Example 6.

MS: M+H = 488.

| Analysis calculated for $C_{24}H_{33}N_5O_4S$-1.4 $CF_3CO_2H$ : 0.21 $H_2O$: | | | |
|---|---|---|---|
| Calculated: | C, 49.84; | H, 5.21; | N, 10.84. |
| Found: | C, 49.52; | H, 5.22; | N, 11.10. |

Example 10

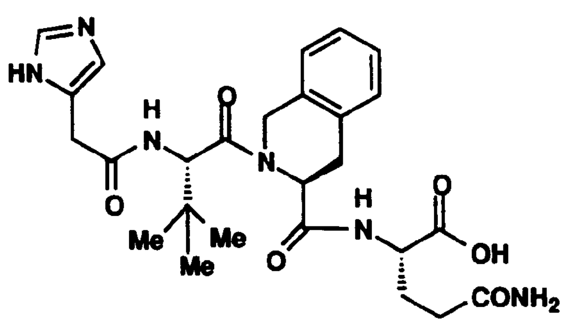

(S)-N-[[1,2,3,4-Tetrahydro-2-[N-(1H-imidazol-4-ylacetyl)-3-methyl-L-valyl]-3-isoquinolinyl]carbonyl]-L-glutamine, trifluoroacetate (1:2) salt

A. (S)-N-[[1,2,3,4-Tetrahydro-2-[N-(1H-imidazol-4-ylacetyl)-3-methyl-L-valyl]-3-isoquinolinyl]carbonyl]-L-glutamine, 1,1-dimethylethyl ester

Compound A was prepared in 78% yield from Compound F of Example 6 and 4-imidazoleacetic acid hydrochloride as described for Compound E of Example 1, $(M+H)^+$ $587^+$.

B. (S)-N-[[1,2,3,4-Tetrahydro-2-[N-(1H-imidazol-4-ylacetyl)-3-methyl-L-valyl]-3-isoquinolinyl]carbonyl]-L-glutamine, trifluoroacetate (1:2) salt

A solution of Compound B (135 mg, 0.23 mmol) and TFA (5 mL) in dichloromethane (5 mL) was stirred for four hours and concentrated. The residue was dissolved in 30 mL of (80:20:0.1) water / acetonitrile / TFA. The mixture was centrifuged and decanted to remove insoluble materials. The solution was subjected to preparative HPLC on an octadecylsilane column (S-10; 30 x 500) using a gradient system from 80% water: 20% acetonitrile: 0.1% TFA to 55% water: 45% acetonitrile: 0.1% TFA over 50 minutes at 50 mL/minute. Under these conditions, two materials eluted at approximately 18.3 and 21.9 minutes. Fractions were analyzed by analytical reversed phase HPLC and those containing the early retention time peak with a minimum purity of 99% were pooled and lyophilized to afford 11 mg (10%) of the title compound from Example 11 as a white solid. Similarly, fractions containing the late retention time peak with a minimum purity of 99% were pooled and lyophilized to afford 30 mg (31%) of the title compound of Example 10 as a white solid.
MS: M+H = 527.

| Analysis calculated for $C_{26}H_{34}N_6O_6$-2.0 $CF_3CO_2H$: | | | |
|---|---|---|---|
| Calculated: | C, 47.75; | H, 4.81; | N, 11.14. |
| Found: | C, 48.10; | H, 4.86; | N, 11.57. |

Example 11

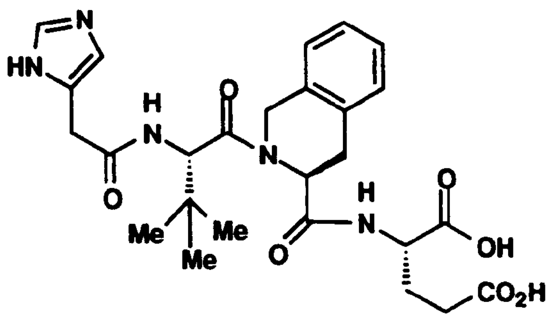

(S)-N-[[1,2,3,4-Tetrahydro-2-[N-(1H-imidazol-4-ylacetyl)-3-methyl-L-valyl]-3-isoquinolinyl]carbonyl]-L-glutamic acid, trifluoroacetate (1:2) salt

The title compound of Example 11 was prepared as described in Example 10.
MS: M+H = 527.

| Analysis calculated for $C_{26}H_{33}N_5O_7$-1.7 $CF_3CO_2H$: | | | |
|---|---|---|---|
| Calculated: | C, 49.02; | H, 4.99; | N, 11.67. |
| Found: | C, 49.25; | H, 4.69; | N, 11.24. |

Example 12

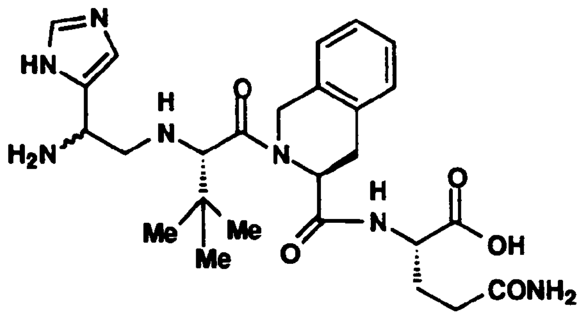

N$^2$-[[(S)-2-[N-[2-amino-2-(1H-imidazol-4-yl)ethyl]-3-methyl-L-valyl]-1,2,3,4-tetrahydro-3-isoquinolinyl]carbonyl]-L-glutamine, trifluoroactate (1:3) salt

A. N-Methyl-N-methoxy-[2-amino-[N-[(1,1-dimethylethoxy)-carbonyl]-2-(N-[(1,1-dimethylethoxy)-carbonyl]-imidazol-4-yl)]acetamide

To a solution of 2-amino-[N-[(1,1-dimethylethoxy)-carbonyl]]-2-(N-[(1,1-dimethylethoxy) -carbonyl]-imidazol-4-yl)acetic acid (330 mg, 0.97 mmol; prepared as described in Schneider F. Z. Physiol. Chem., 3, 206-210 (1961); Janusz, J.M., Young, P.A., Blum, R.B., Riley, C.M. J. Med Chem. 33, 1676-1682 (1990)), N,O-dimethyl hydroxylamine hydrochloride (97 mg, 1 mmol) and bromo-tris-pyrrolidino-phosphonium hexafluorophosphate (PyBrop) (466 mg, 1 mmol) in methylene chloride (10 mL) at 0°C was added DIEA (0.523 mL, 3 mmol) and DMAP (12.2 mg, 0.1 mmol). The solution was allowed to stir at ambient temperature for 2 hours and concentrated. The residue was dissolved in ethyl acetate (40 mL) and the solution was washed with 1N potassium bisulfate ($KHSO_4$) (3 x 30 mL) followed by water (1 x 30 mL). The organic layer was dried (magnesium sulfate), filtered and concentrated. The residue was chromatographed (flash, silica 7.1 x 20 cm, 1:1 ethyl acetate:hexanes). Fractions containing the product were pooled and concentrated to afford 260 mg (70%) of Compound A as a white solid.
MS: $(M+H)^+$ $385^+$.

B. N$^2$-[[(S)-2-[N-[2-amino-[N-[(1,1-dimethylethoxy)-carbonyl]]-2-(N-[(1,1-dimethylethoxy)-carbonyl]-(imidazol-4-yl)ethyl]-3-methyl-L-valyl]-1,2,3,4-tetrahydro-3-isoquinolinyl]carbonyl]-L-glutamine, 1,1-dimethylethyl ester

To a solution of Compound A (0.18 g, 0.47 mmol) in THF (20 mL) cooled to 0°C was added 1 M lithium aluminum hydride in THF (0.47 mL, 0.47 mmol) dropwise. The solution was stirred under argon for 0.5 hour followed by dropwise addition of 1M potassium bisulfate to pH 4. The mixture was stirred for 1 hour at 0 C, ether (40 mL) and water (40 mL) were added and the layers were separated. The aqueous layer was washed with ether and the organic layers were pooled, dried ($MgSO_4$) and concentrated. The highly unstable residue was immediately dissolved in dry methanol (20 mL), along with 3Å molecular sieves (0.5 g), acetic acid (0.2 mL) and Compound F of Example 6 (300 mg, 0.58 mmol). The solution was stirred for 10 minutes followed by the portionwise addition of $NaBH_3CN$ (29 mg, 0.47 mmol) over 1 hour. The solution was stirred for 4 hours and concentrated, and the residue was chromatographed (flash, silica, 19:1 chloroform:methanol). Fractions containing the product were pooled and concentrated to afford 90 mg (25%) of Compound B as a slightly yellow oil.
MS: $(M+H)^+$ $784^+$.

C. N$^2$-[[(S)-2-[N-[2-amino-2-(1H-imidazol-4-yl)ethyl]-3-methyl-L-valyl]-1,2,3,4-tetrahydro-3-isoquinolinyl]carbonyl]-L-glutamine, trifluoroactate (1:3) salt

A solution of Compound B (40 mg, 0.05 mmol) in TFA (5 mL) and methylene chloride (5 mL) was stirred for 2.5 hours and concentrated. The residue was dissolved in 3 mL of a 50/50 mixture of 0.1% TFA in methanol and 0.1% TFA in water, and subjected to HPLC purification (YMC C18 column (S-10, ODS 30 x 500 mm); Solvent A; 0.1% TFA in 90% water, 10% methanol, Solvent B; 0.1% TFA in 90% methanol, 10% water; 10-35% B in A over 60 minutes). Fractions containing the major peak were pooled and lyophilized to yield 20 mg (45%)

of the title compound as a fluffy white solid.
MS: $(M+H)^+$ $528^+$.
$^1$H-NMR ($CDCl_3$, 400 MHz) δ 8.40 (1H, m), 7.46 (1H, m), 7.22 (4H, m), 5.10 (1H, m), 4.8-4.3 (5H, m), 4.19 (1H, m), 3.3-3.0 (2H, m), 2.28 (1H, m),1.92 (2H, m), 1.80 (1H, m), 1.10 (9H, d).

Example 13

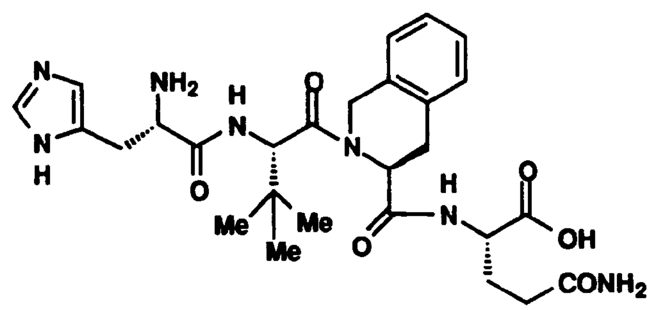

(S,S)-$N^2$-[[2-[[2-Amino-3-(1H-imidazol-4-yl)-1-oxopropyl]-3-methyl-L-valyl]-1,2,3,4-tetrahydro-3-isoquinolinyl]-carbonyl]-L-glutamine, monolithium salt

A. (S,S)-$N^2$-[[2-[[2-Amino-[N-[(1,1-dimethylethoxy)-carbonyl]]-3-(1H-imidazol-4-yl)-1-oxopropyl]-3-methyl-L-valyl]-1,2,3,4-tetrahydro-3-isoquinolinyl]carbonyl]-L-glutamine, 1,1-dimethylethyl ester

Compound A was prepared in 71% yield from Compound F of Example 6 and Boc-L-histidine as described for Compound E of Example 1.
$(M+H)^+$ $712^+$.

B. (S,S)-$N^2$-[[2-[[2-Amino-3-(1H-imidazol-4-yl)-1-oxopropyl]-3-methyl-L-valyl]-1,2,3,4-tetrahydro-3-isoquinolinyl]-carbonyl]-L-glutamine, monolithium salt

A mixture of Compound A (100 mg, 140 mmol) and TFA (1 mL) in dichloromethane (10 mL) was stirred for two hours and concentrated. The residue was retreated with TFA (1 mL) in 5 mL of dichloromethane for 30 minutes and evaporated. The residue was dissolved in 25 mL of (80:20:0.1) water / acetonitrile / TFA. The mixture was centrifuged and decanted to remove insoluble materials. The solution was subjected to preparative HPLC on an octadecylsilane column (S-10; 30 x 500) using a gradient system from 85% water: 15% acetonitrile: 0.1% TFA to 50% water: 50% acetonitrile: 0.1% TFA over 50 minutes at 50 mL/minute. Under these conditions, the product eluted at approximately 22 minutes. Fractions were analyzed by analytical reversed phase HPLC and those containing product with a minimum purity of 90% were pooled and lyophilized. The residue was dissolved in 25 mL of water and the pH was adjusted to 7.5 with 1N lithium hydroxide (LiOH). The solution was applied to a column of HP-20 (25 x 200 mm), which was prewashed with water. The column was eluted with water (200 mL). The product was eluted with a gradient system from 100% water to 50% aqueous acetonitrile over 2 hours at 2 mL/minute. Clean product was combined and lyophilized to afford 14.8 mg (19%) of the title compound as a white solid.
MS: M+H = 556.

| Analysis calculated for $C_{27}H_{36}N_7O_6Li$-1.0 $CH_3OH$ : 1.33 $H_2O$: | | | |
|---|---|---|---|
| Calculated: | C, 54.45; | H, 6.96; | N, 15.87. |
| Found: | C, 54.55; | H, 6.78; | N, 15.48. |

Example 14

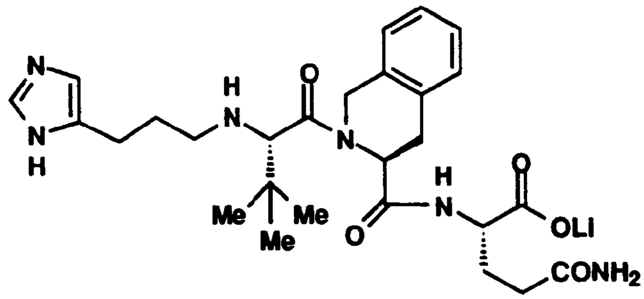

(S)-N-[[1,2,3,4-tetrahydro-2-[N-[2-(1H-imidazol-4-yl)propyl]-3-methyl-L-valyl]-3-isoquinolinyl]carbonyl]-L-glutamine, monolithium salt

A. 3-(N-Triphenylmethylimidazol-4-yl)-thiopropionic acid, ethyl ester

To a heterogenous solution of (N-triphenylmethylimidazol-4-yl)-propionic acid (0.8 g, 2.09 mmol), ethanethiol (0.42 mL, 5.65 mmol) and DMAP (50mg) in dichloromethane (10 mL) at 0°C was added EDC (0.52 g, 2.7 mmol). The mixture was stirred at room temperature for 2 hours. The homogeneous solution was washed twice with 5% $KHSO_4$, saturated sodium bicarbonate ($NaHCO_3$) and brine, and dried over $MgSO_4$. The drying agent was filtered and the filtrate was evaporated to afford 0.85g (95%) of Compound A.
MS: $(M+Na)^+ = 449^+$.

B. 3-(N-Triphenylmethylimidazol-4-yl)-propanal

To a degassed solution of Compound A (0.8 g, 1.88 mmol) and triethylsilane (1.0 mL, 6.33 mmol) in acetone (5 mL) was added 10% palladium on carbon (100 mg). The mixture was stirred 30 minutes, additional amounts of triethylsilane (0.25 mL, 1.58 mmol) and 10% palladium on carbon (100 mg) were added, and stirring was continued for 1 hour. The mixture was filtered through a pad of Celite®, the pad was rinsed with acetone and the combined filtrates were evaporated to afford 0.7 g (100%) of crude Compound B.
MS: (M+Na)+ = 389.

C. (S)-N-[[1,2,3,4-tetrahydro-2-[N-[2-(1-triphenylmethyl-imidazol-4-yl)propyl]-3-methyl-L-valyl]-3-isoquinolinyl]-carbonyl]-L-glutamine, 1,1-dimethylethyl ester

Compound C was prepared in 100% yield from Compound B and Compound F of Example 6 as described for Compound G of Example 6. It was carried on without purification.
MS: (M+H) = 825.

D. (S)-N-[[1,2,3,4-tetrahydro-2-[N-[2-(1H-imidazol-4-yl)propyl]-3-methyl-L-valyl]-3-isoquinolinyl]-carbonyl]-L-glutamine, monolithium salt

The title compound was prepared from Compound C as described for Compound F of Example 2. Following preparative HPLC, the purified material was dissolved in 25 mL of water and the pH was adjusted to 7.5 with 1N LiOH. The solution was applied to a column of HP-20 (25 x 200 mm), which was prewashed with water. The column was eluted with water (200 mL). The product was eluted with a gradient system from 100% water to 50% aqueous acetonitrile over 2 hours at 2 mL/minute. Clean product was combined and lyophilized to afford 53.3 mg (34%) of the title compound as a white solid.
MS: M+H = 526.

| Analysis for $C_{27}H_{37}N_6O_5Li$ - 3.43 $H_2O$: | | | |
|---|---|---|---|
| Calculated: | C, 54.56; | H, 7.43; | N, 14.13. |
| Found: | C, 54.56; | H, 7.32; | N, 13.79. |

Example 15

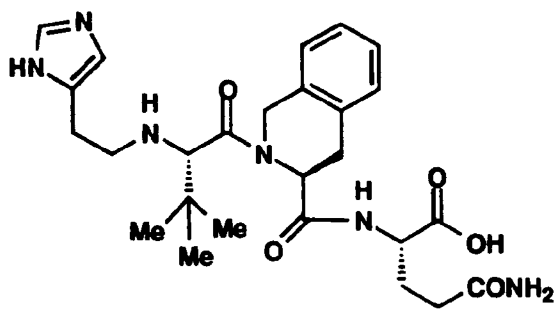

(S)-N-[[1,2,3,4-tetrahydro-2-[N-[2-(1H-imidazol-4-yl)ethyl]-3-methyl-L-valyl]-3-isoquinolinyl]carbonyl]-L-glutamine, monolithium salt

A. (Imidazol-4-yl)-thioacetic acid, ethyl ester

To a heterogenous solution of 4-imidazoleacetic acid hydrochloride (0.5 g, 3.08 mmol) in dichloromethane (10 mL) at 0°C was added DIEA (0.50 mL, 3.08 mmol) followed by ethanethiol (0.56 mL, 7.68 mmol) and EDC (0.65 g, 3.40 mmol). DMF (1 mL) was added after 1 hour to increase the solubility of the acid. The mixture was stirred at room temperature for 18 hours and partitioned between saturated $NaHCO_3$ and dichloromethane. The organic phase was washed with water and brine, and dried over $MgSO_4$. The drying agent was filtered and the filtrate evaporated to afford 0.52g (100%) of Compound A.
MS: $(M+H)^+ = 171^+$.

B. (N-Triphenylmethylimidazol-4-yl)-thioacetic acid, ethyl ester

Compound B was prepared from triphenylmethylchloride and Compound A as described for Compound A of Example 2. Flash chromatography on silica gel with (2:1) hexanes/ethyl acetate afforded a 54% yield of Compound B.
MS: $(M+H)^+ = 413^+$.

C. (N-Triphenylmethylimidazol-4-yl)-acetaldehyde

Compound C was prepared from Compound B as described for Compound B of Example 14.

D. (S)-N-[[1,2,3,4-tetrahydro-2-[N-[2-(1-triphenylmethyl-imidazol-4-yl)ethyl]-3-methyl-L-valyl]-3-isoquinolinyl]-carbonyl]-L-glutamine, 1,1-dimethylethyl ester

Compound D was prepared from Compound C and Compound F of Example 6 as described for Compound G of Example 6, using dichloromethane as the extraction solvent. It was carried on without purification.
MS: (M+H) = 811.

E. (S)-N-[[1,2,3,4-tetrahydro-2-[N-[2-(1H-imidazol-4-yl)ethyl]-3-methyl-L-valyl]-3-isoquinolinyl]-carbonyl]-L-glutamine, monolithium salt

The title compound was prepared as a white solid from Compound D as described for Compound D of Example 14 (21% yield from Compound C).
MS: M+H = 513.

Analysis for $C_{26}H_{35}N_6O_5Li$ - 0.5 $CH_3OH$; 1.48 $H_2O$:

| | | | |
|---|---|---|---|
| Calculated: | C, 56.70; | H, 7.17; | N, 14.97. |
| Found: | C, 57.00; | H, 6.97; | N, 14.57. |

Example 16

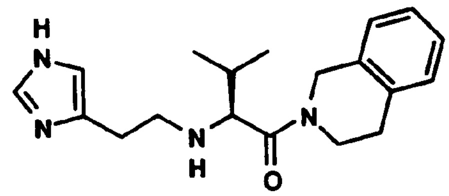

(S)-1,2,3,4-Tetrahydro-2-[2-[[2-(1H-imidazol-4-yl)ethyl]amino]-3-methyl-1-oxobutyl]isoquinoline, 1:1 hydrochloride/trifluoroacetate salt

A. ((Imidazol-4-yl)-1-oxoethyl)-L-valine, phenylmethyl ester

To a solution of valine benzyl ester • HCl (2.0 g, 8.2 mmol), imidazole-4-acetic acid • HCl (1.46 g, 9 mmol), HOBT (1.22 g, 9 mmol) and NMM (3 mL, 27 mmol) in 10 mL of dry DMF at 0°C under argon was added EDC (1.73 g, 9 mmol). The slurry was stirred with cooling for 0.5 hour and at room temperature for 3 hours. The milky reaction was evaporated to dryness (30°C, high vac), and the residue was diluted with saturated $NaHCO_3$ and extracted with ethyl acetate (three times). The combined extracts were washed with brine (twice) and dried ($MgSO_4$), and the solvent was removed to give 3.6 g of clear orange viscous oil. Flash chromatography (dichloromethane:methanol:ammonium hydroxide (95:5:0.5)) afforded 2.0 g (78%) of compound A as a viscous foam.

B. ((Imidazol-4-yl)-1-thioxoethyl)-L-valine, phenylmethyl ester

A suspension of compound A (0.8 g, 2.5 mmol) and Lawesson's Reagent (1.0 g, 2.5 mmol) in 30 mL of dry toluene was heated at 90°C for 1 hour. A clear yellow solution was obtained during the heating period. A viscous oil separated on cooling to room temperature. The mixture, without workup, was subjected to flash chromatography on a 2.5 x 30 cm column of silica gel. (The toluene solution was decanted directly onto the column, the precipitate was dissolved in dichloromethane and this solution was also added to the column). Elution with trichloromethane:methanol:ammonium hydroxide (95:5:0.5) afforded 0.75 g (90%) of compound B as a clear viscous oil.

C. ((Imidazol-4-yl)-ethyl)-L-valine, phenylmethyl ester

To a solution of compound B (0.75 g, 2.27 mmol) and $NiCl_2$ • 6 $H_2O$ (1.08 g, 4.54 mmol) in 15 mL each of methanol and THF in an oversized flask, at -40°C and under argon, was added in portions as a solid, sodium borohydride ($NaBH_4$) (515 mg, 13.62 mmol). An immediate black precipitate, along with vigorous hydrogen evolution, was obtained on addition of the borohydride. Each addition of borohydride was done after reaction from the previous addition had essentially ceased. After the addition, stirring was continued for 2 hours, during which time the reaction was allowed to warm to approximately 0°C. To the dark slurry was then added sodium sulfide nonahydrate ($Na_2S$ • 9 $H_2O$) (1.09 g, 4.54 mmol). Stirring was continued for an additional 0.5 hours, after which the solids were removed by filtration through Celite® and the black filter cake washed well with additional methanol. The clear yellow filtrate was evaporated to dryness and the semi-solid residue extracted exhaustively with dichloromethane. The combined extracts were evaporated to dryness to give approximately 0.8 g of oil. Flash chromatography (2.5 x 30 cm, silica gel, 5% methanol - trichloromethane) afforded 513 mg (75%) of compound C as a clear viscous oil.

D. N-[(1,1-Dimethylethoxy)-carbonyl]-N-[1-[(1,1-dimethylethoxy)-carbonyl]-imidazol-4-yl-ethyl]-L-valine, phenylmethyl ester

A solution of compound C (4.0 g, 13.3 mmol) and t-butoxycarbonyl (BOC) anhydride (8.7 g, 40 mmol) in 100 mL of dichloromethane, under argon, was allowed to stir at room temperature for 2 days, after which time an additional 2 g of BOC anhydride was added. Stirring was continued for an additional day. The mixture was reduced in volume to approx 50 mL and the remaining solution subjected to flash chromatography (5 x 30 cm, silica gel, 30% ethyl acetate-hexane) to afford 4.2 g (63%) of compound D as a clear viscous oil.

E. N-[(1,1-Dimethylethoxy)-carbonyl]-N-[imidazol-4-yl-ethyl]-L-valine

A solution of compound D (2.0 g, 4 mmol) and barium hydroxide octahydrate ($Ba(OH)_2 \bullet 8 H_2O$) (3.15 g, 10 mmol) in 15 mL each of water and methanol was heated at 50°C under argon for 1 hour, during which time a white precipitate formed. After cooling to room temperature, the methanol was removed under reduced pressure and to the remaining white slurry was added 960 mg (10 mmol) of ammonium carbonate ($(NH_4)_2CO_3$) as a solid. After stirring for 0.5 hour, the solids were removed by filtration and the filter cake washed well with additional water. The clear colorless filtrate was washed once with ether and evaporated to dryness to give a white powder. This material was suspended into water and the suspension again evaporated to dryness to yield 1.1g (88%) of compound E as a fine white powder.

F. (S)-1,2,3,4-Tetrahydro-2-[[N-[(1,1-dimethylethoxy)-carbonyl][2-(1H-imidazol-4-yl)ethyl]amino]-3-methyl-1-oxobutyl]-isoquinoline

1-Hydroxy-7-azo-benzotriazole (HOAT) (0.066 g, 0.48 mmol) and EDC (0.093 g, 0.48 mmol) were added to a solution of Compound E (0.10 g, 0.32 mmol) in DMF (1.5 mL) and the mixture was stirred for 30 minutes. 1,2,3,4-Tetrahydroisoquinoline (0.121 mL, 0.97 mmol) and DIEA (0.101 mL, 0.64 mmol) were added and the mixture was stirred for 16 hours. The reaction was quenched with 10% lithium chloride (LiCl) (30 mL) and saturated $NaHCO_3$ (10 mL), and the solution was extracted with ethyl acetate (2 X 30 ml). The combined organic extracts were washed with 10 % LiCl (2 X 30 ml), dried ($MgSO_4$), filtered and concentrated under vacuum. The residue was purified by flash chromatography (eluting with 19/1 chloroform / methanol) to afford Compound F (0.094 g, 67%) as a foam. MS: $(M-H)^-$ 425.

G. (S)-1,2,3,4-Tetrahydro-2-[2-[[2-(1H-imidazol-4-yl)ethyl]amino]-3-methyl-1-oxobutyl]isoquinoline, 1:1 hydrochloride / trifluoroacetate salt

TFA (2.0 mL, 26 mmol) was added to a solution of Compound F (0.090 g, 0.21 mmol) in dichloromethane (2.0 mL), and the reaction was stirred for 1 hour and concentrated under vacuum. The residue was dissolved in water (10 mL), millipore filtered and lyophilized to afford the title compound (0.012 g, 100 %) as a glassy solid.
MS: $(M+H)^+$ 327.

| Analysis for $C_{19}H_{26}N_4O$-1.0 HCl-2.11 $H_2O$-1.0 $CF_3CO_2H$: | | | |
|---|---|---|---|
| Calculated: | C, 48.99; | H, 6.31; | N, 10.88. |
| Found: | C, 48.99; | H, 5.90; | N, 10.81. |

Example 17

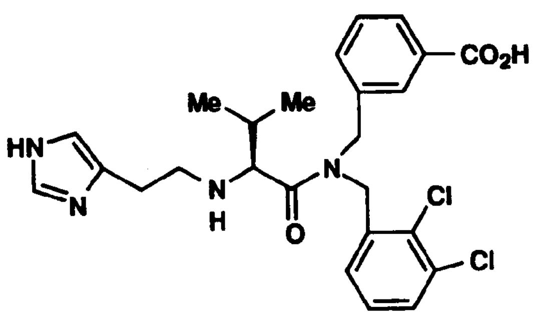

(S)-3-[[[(2,3-Dichlorophenyl)methyl][2-[[2-(1H-imidazol-4-yl)ethyl]amino]-3-methyl-1-oxobutyl]amino]methyl] benzoic acid, dihydrochloride

A. N-[(2,3-dichlorophenyl)methyl]-N-((3-methoxycarbonyl)-phenylmethyl)-amine, hydrochloride

Acetic acid (0.57 mL, 10 mmol) was added to a solution of 3-carboxybenzaldehyde (1.0 g, 6.7 mmol) and

2,3-dichlorobenzylamine (1.3 mL, 10 mmol) in dichloromethane (33 mL). The mixture was stirred for 1 hour, sodium triacetoxyborohydride (2.1 g, 10 mmol) was added and the solution was stirred for 16 hours and concentrated under vacuum. The residue was dissolved in methanol (50 mL), the solution was cooled to 0°C, and anhydrous HCl was bubbled into the mixture. The solution was warmed to room temperature, heated to reflux for 6 hours and concentrated under vacuum. The residue was dissolved in 10% $NaHCO_3$ (50 mL) and the aqueous solution was extracted with ethyl acetate (3 X 100 mL). The combined organic extracts were dried ($MgSO_4$), filtered and concentrated under vacuum. The residue was dissolved in diethyl ether (20 mL), treated with 4M HCl in dioxane (5 mL), and the solid was filtered. The solid material was washed with diethyl ether (3 X 10 mL) and dried under vacuum to afford Compound A (1.95 g, 81%) as a white solid. MS:$(M+H)^+$ 324.

B. (S)-3-[[[(2,3-Dichlorophenyl)methyl][2-[N-[(1,1-dimethylethoxy)-carbonyl][2-(1-triphenylmethyl-imidazol-4-yl)ethyl]amino]-3-methyl-1-oxobutyl]amino]methyl]benzoic acid, methyl ester

DIEA (0.32 mL, 1.8 mmol) was added to a solution of Compound A (0.33 g, 0.90 mmol) and Compound A of Example 30 (0.25 g, 0.45 mmol) in dichloromethane (2.3 mL). The solution was cooled to 0°C, DMAP (0.083 g, 0.68 mmol) and PyBrop (0.32 g, 0.68 mmol) were added. The mixture was warmed to room temperature, stirred for 72 hours, quenched with 1N HCl (50 mL) and extracted with ethyl acetate (3 X 50 mL). The combined organic extracts were washed with 10% $NaHCO_3$ (1 X 50 mL), dried ($MgSO_4$), filtered and concentrated under vacuum to afford Compound B (0.78 g, 100%) as a sticky solid.
MS: $(M+H)^+$ 859.

C. (S)-3-[[[(2,3-Dichlorophenyl)methyl][2-[N-[(1,1-dimethylethoxy)-carbonyl][2-(1-tri phenylmethyl-imidazol-4-yl)ethyl]amino]-3-methyl-1-oxobutyl]amino]methyl]bezoic acid

Sodium hydroxide (1N, 10 mL, 10 mmol) was added to a solution of Compound B (0.78 g, 0.90 mmol) in methanol (30 mL). The mixture was stirred for 16 hours and concentrated under vacuum, and the residue was dissolved in 1N HCl (50 mL). The solution was extracted with dichloromethane (3 X 50 mL) and the combined organic extracts were dried ($MgSO_4$), filtered and concentrated under vacuum to afford Compound C (0.76 g, 100%) as a sticky solid.
MS: $(M+H)^+$ 845.

D. (S)-3-[[[(2,3-Dichlorophenyl)methyl][2-[[2-(1H-imidazol-4-yl)ethyl]amino]-3-methyl-1-oxobutyl]amino]methyl]benzoic acid, dihydrochloride

Triethylsilane (2.2 mL, 13.6 mmol) followed by TFA (10 mL, 130 mmol) were added to a solution of Compound C (0.764 g, 0.904 mmol) in dichloromethane (7 mL). The mixture was stirred for 16 hours and concentrated under vacuum, and the residue was triturated with hexane (3 X 10 mL), discarding the hexane layers. The residue was purified by preparative HPLC (YMC S10 ODS 30 X 500 mm, 10-90% aqeous methanol with 0.1% TFA, 60 minute gradient, 20 mL/minute). The appropriate fractions were concentrated under vacuum. The residue was dissolved in methanol (10 mL) and 1N HCl (2 mL) was added. The mixture was stirred 5 minutes and concentrated under vacuum. This latter procedure was repeated two times and the residue was dissolved in water (10 mL), millipore filtered and lyophilized to afford the title compound (0.070g, 13%).
MS: $(M+H)^+$ 503.

Example 18

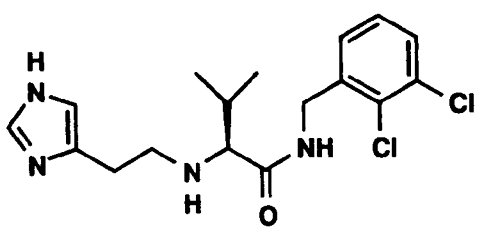

N-[(2,3-Dichlorophenyl)methyl]-$N^2$-[2-(1H-imidazol-4-yl)ethyl]-L-valinamide, hydrochloride

A suspension of Compound E of Example 16 (15.6 mg, 0.05 mmol), triethylamine (8 μl, 0.055 mmol), 2,3-dichlorobenzylamine (200 μl of 0.25 M solution in methylene chloride, 0.05 mmol), and BOP (220 μl of 0.25 M

solution in methylene chloride, 0.055 mmol) was stirred at 25°C for 18 hours. TFA (1 mL) was added to the mixture and stirring was continued for an additional 2 hours. The mixture was concentrated *in vacuo*, the residue was combined with material from 4 other similar reactions, and the combined material was purified by preparative HPLC (YMC S-10 ODS column, 30 x 500 mm, eluting with 50 mL/minute of a linear gradient from 10% to 90% aqueous methanol, containing 0.1% TFA, over 30 minutes). Fractions from the third (of four) major peak were combined and concentrated. The residue was purified again by the same method. Fractions containing the major peak were combined and concentrated. The residue was dissolved in 50 mL of 1N hydrochloric acid and reconcentrated twice. The residue was lyophilized to give the title compound as a white solid (70 mg, 75%).
MS: $(M+H)^+$ 369.

| Analysis for $C_{17}H_{22}N_4OCl_2$-1.00 $H_2O$-2.06 HCl: | | | | |
|---|---|---|---|---|
| Calculated: | C, 44.16; | H, 5.68; | N, 12.12; | F, 31.13. |
| Found: | C, 44.53; | H, 5.94; | N, 11.41; | F, 31.24. |

Example 19

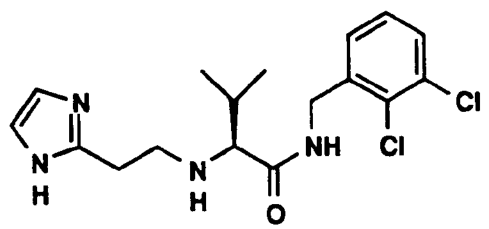

N-[(2,3-Dichlorophenyl)methyl]-N²-[2-(1H-imidazol-2-yl)ethyl]-L-valinamide, trifluoroacetate

A. 2-Hydroxyethyl-N-triphenylmethylimidazole

To a solution of N-triphenylmethylimidazole (15.5 g, 50 mmol) at 0°C in THF was added normal butyl lithium (nBuLi) (34.4 mL, 55 mmol) dropwise over 30 minutes. The mixture was stirred for 1 hour followed by gentle warming to 30°C over 30 minutes. The solution was cooled to 0°C, and ethylene oxide (24.4 mL, 50 mmol) was added to the mixture via a cannula. The mixture was stirred at 0°C for 2 hours and gradually warmed to ambient temperature over 16 hours. The solution was concentrated and the residue was chromatographed (flash silica, 19:1; chloroform:methanol) to yield 14.5g (82%) of compound A as a white solid. MS $(M+Na)^+$ $377^+$.

B. N²-[(Trifluoromethanesulfonyl)]-N²-[2-(1-triphenylmethyl-imidazol-2-yl)ethyl]-L-valine benzyl ester

To a solution of compound A (880 mg, 2.5 mmol), N-trifluoromethanesulfonyl-L-valine, benzyl ester (842 mg, 2.5 mmol) and triphenylphosphine (655 mg, 2.5 mmol) under nitrogen was added diisopropylazodicarboxylate(0.49 mL, 2.5 mmol) dissolved in THF (2 mL) dropwise. The solution was stirred for 16 hours and concentrated, and the residue was chromatographed (flash silica, 1:1; ethyl acetate:hexanes) to yield 1.4g (83%) of compound B as a white crystalline solid. $(M+H)^+$ $676^+$.

C. N²-[(Trifluoromethanesulfonyl)]-N²-[2-(1-triphenylmethyl-imidazol-2-yl)ethyl]-L-valine

A solution of compound B (3 g, 4.4 mmol) dissolved in methanol (30 mL) and THF (40 mL) was hydrogenated on palladium on carbon (10%, 0.3 g) at atmospheric pressure. After 2.5 hours, the solution was filtered through Celite® and the filtrate concentrated to give 2.6 g (100 %) of Compound C as a white crystalline solid. $(M+H)^+$ $586^+$.

D. [Imidazol-2-yl)ethyl]-L-valine

To a solution of Compound C (2.5g, 4.3 mmol) and t-butanol (25 mL) in THF (25 mL) at -70°C was added

freshly distilled ammonia (150 mL). Freshly cut sodium chunks were added until a blue color persisted for 40 minutes. Ammonium chloride was added until the blue color dissipated. The solution was concentrated to dryness and the residue was dissolved in 15 mL of water and applied to a cation exchange column in the following manner: 10 equivalents of resin was washed with 0.1N HCl (100 mL) followed by water (100 mL); the sample was loaded and the column was washed with water (200 mL); the column was washed with 300 mL of 1M ammonium hydroxide ($NH_4OH$) and fractions were collected. The appropriate fractions were pooled and nitrogen was bubbled through to remove dissolved ammonia. The solution was concentrated to give 833 mg (85%) of Compound D as an off-white powder. $(M+H)^+$ $212^+$.

E. N-[(2,3-Dichlorophenyl)methyl]-$N^2$-[2-(1H-imidazol-2-yl)ethyl]-L-valinamide, trifluoroacetate

A solution of compound D (100 mg, 0.44 mmol) 2,3-dichlorobenzylamine (77 mg, 0.44 mmol), BOP (190 mg, 0.44 mmol) and DIEA (0.77 mL, 0.44 mmol) in DMF (3 mL) and DMSO (0.1 mL) was stirred for 3 hours under nitrogen. The mixture was concentrated and the residue was dissolved in a 50/50 mixture of 0.1%TFA in methanol and 0.1%TFA in water. This was applied to a YMC C18 column (S-10, ODS 30 x 500 mm) and HPLC purification was performed under the following conditions; Solvent A; 0.1%TFA in 90% water, 10% methanol, Solvent B; 0.1%TFA in 90% methanol, 10% water; 10-35% B in A over 60 minutes. Fractions containing the product were pooled and lyophilized to afford the title compound as a white solid.
MSs $(M+H)^+$ 369.

| Analysis for $C_{18}H_{24}N_4OCl_2$-0.98 $H_2O$-2.19 $CF_3CO_2H$: | | | | |
|---|---|---|---|---|
| Calculated: | C, 40.34; | H, 4.14; | N, 8.80; | F, 19.61. |
| Found: | C, 40.34; | H, 3.85; | N, 8.55; | F, 19.59. |

Example 20

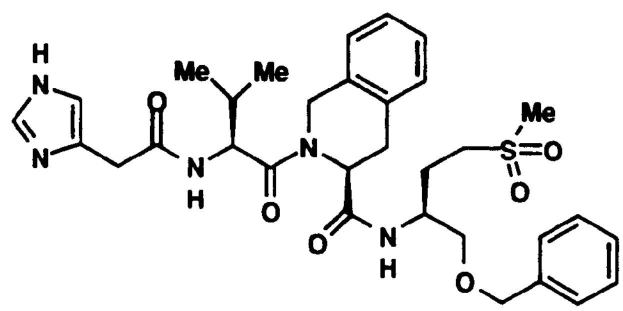

[S-(R*,R*)]-1,2,3,4-Tetrahydro-2-[N-(1H-imidazol-4-ylacetyl)-L-valyl]-N-[3-(methylsulfonyl)-1-[(phenyl-methoxy)methyl]propyl]-3-isoquinoline-carboxamide, trifluoroacetate

A. [S-[2-[(1,1-dimethylethoxy)-carbonyl]amino]-4-methylsulfonyl-O-phenylmethyl]-butan-1-ol

A mixture of [S-[2-[(1,1-dimethylethoxy)carbonyl]amino]-4-methylsulfonyl]-butan-1-ol (212 mg, 0.79 mmol), sodium hydride (40 mg of 60% dispersion in oil, 1.0 mmol) and benzyl bromide (0.12 mL, 1.0 mmol) in DMF (2 mL) was stirred at 25°C for 18 hours. The mixture was diluted with methylene chloride, washed with saturated aqueous sodium bicarbonate solution, dried over $MgSO_4$ and concentrated. The residue was purified by flash chromatography on silica gel (60 g), eluting with 2:1 hexanes : ethyl acetate, to give Compound A (165 mg, 58%).

B. [S-[2-amino]-4-methylsulfonyl-O-phenylmethyl]-butan-1-ol, trifluoroacetate

A mixture of Compound A (165 mg, 0.46 mmol), methylene chloride (1 mL) and TFA (2 mL) was stirred for 6 hours and concentrated *in vacuo* to give compound B (125 mg, 90%).

C. [S-(R*,R*)]-1,2,3,4-Tetrahydro-2-[N-L-valyl]-3-isoquinoline-carboxylic acid, phenylmethyl ester, hydrochloride

Compound C was prepared from tetrahydroisoquinolin-3-carboxylic acid, phenylmethyl ester and Boc-L-valine in a 2 step procedure as described for Compounds C and D of Example 1.

D. [S-(R*,R*)]-1,2,3,4-Tetrahydro-2-[N-(1H-imidazol-4-ylacetyl)-L-valyl]-3-isoquinoline-carboxylic acid, phenylmethyl ester

To a suspension of the sodium salt of imidazole acetic acid (2.26 g, 12.3 mmol) in DMF (15 mL) at 0° under nitorgen was added BOP (5.42 g, 12.3 mmol). After stirring for 30 minutes, Compound C (4.12, 10.2 mmol) was added followed by NMM (1.12 mL, 10.2 mmol). After 5 hours, the mixture was concentrated *in vacuo*. The residue was dissolved in ethyl acetate (150 mL) and the solution was washed with 10% sodium bicarbonate (2 x 70 mL) and 10% LiCl (70 mL). The aqueous LiCl layer was extracted with ethyl acetate (3 x 100 mL). The organic layers were combined, dried over sodium sulfate, filtered and concentrated *in vacuo* to give Compound D (5.32 g) which was 71% pure by HPLC. This material was combined with material from similar reactions (6.30 g total) and purified by silica gel chromatography (dichloromethane:methanol, 98:2 to 88:12) to give Compound D (3.83 g, 59% yield).

E. [S-(R*,R*)]-1,2,3,4-Tetrahydro-2-[N-(1H-imidazol-4-ylacetyl)-L-valyl]-3-isoquinoline-carboxylic acid, sodium salt

To a solution of Compound D (4.19 g, 8.84 mmol) in methanol (50 mL) was added 10% palladium on carbon (1.2 g). The suspension was stirred under a hydrogen atmosphere for 2 hours, filtered through Celite® and the filter cake was washed with methanol (10 mL). The filtrate was concentrated *in vacuo*, the residue was dissolved in methanol (20 mL), and 1N sodium hydroxide (9.46 ml, 9.46 mmol) was added. The methanol was removed *in vacuo* and the aqueous slurry was purified by reverse phase column chromatography on CHP20P (water:acetonitrile gradient, 100:0 to 0:100) to give Compound E (2.48 g, 69%).
MS: $(M+H)^+$ 385.

F. [S-(R*,R*)]-1,2,3,4-Tetrahydro-2-[N-(1H-imidazol-4-ylacetyl)-L-valyl]-N-[3-(methylsulfonyl)-1-[(phenylmethoxy)methyl]propyl]-3-isoquinoline-carboxamide, trifluoroacetate

A mixture of Compound E (122 mg, 0.30 mmol), Compound B (80 mg, 0.27 mmol), BOP (155 mg, 0.35 mmol), triethylamine (50 μL, 0.35 mmol), methylene chloride (0.5 mL) and DMF (0.5 mL) was stirred at 25°C for 18 hours. The mixture was diluted with ethyl acetate, washed with saturated aqueous sodium bicarbonate solution and brine, dried over $MgSO_4$, and concentrated. The residue was purified by preparative HPLC (YMC S5 ODS column, 30 x 250 mm, eluting with a gradient from 10% to 90% aqueous methanol, containing 0.1% TFA, over 30 minutes). Fractions containing the major product were combined and concentrated. The residue was lyophilized to give the title compound as a white solid (53 mg, 24%).
MS: M+H = 624.
Analysis calculated for $C_{32}H_{41}N_6O_6S$-1.5 $CF_3CO_2H$ : 2.5 $H_2O$:
Calculated : C, 50.06; H, 5.70; N, 8.34; S, 3.82; F, 10.18.
Found : C, 49.92; H, 5.33; N, 8.01; S, 3.75; F, 9.92.

Example 21

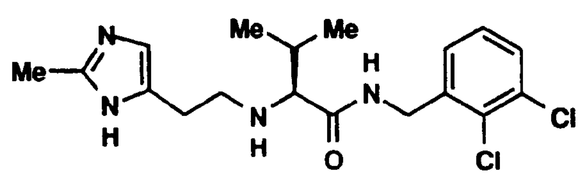

N-[(2,3-Dichlorophenyl)methyl]-N2-[2-(2-methyl-1H-imidazol-4-yl)ethyl]-L-valinamide, dihydrochloride

A. 2-Methyl-4-chloromethylimidazole, hydrochloride

To 15 mL of thionyl chloride was added, in portions, 3.0 g (27 mmol) of 2-methyl-4-hydroxymethylimidazole (prepared according to the method of Durant et al., J. Med. Chem., 19, 923 (1976)). A vigorous reaction was obtained on addition. The resulting black solution was heated at 75°C for 0.5 hours and evaporated to dryness, and the residue was evaporated once from THF to remove residual thionyl chloride. The resulting brown solid was recrystallized from ethanol-ether. Standing under refrigeration afforded 3.3 g (19.7 mmoles, 73 %) of Compound A as brown crystals.

B. 2-Methyl-4-cyanomethylimidazole

To an ice cooled solution of 11 g of potassium cyanide (KCN) in 20 mL of water was added, dropwise over 0.5 hours a solution of 3.3 g (19.7 mmol) of Compound A in 60 mL of ethanol. A voluminous precipitate was obtained on addition. Stirring was continued for an additional 0.5 hours with cooling. The solids were filtered and the filter cake washed well with ethanol. To the filtrate was added 20 mL of saturated $NaHCO_3$, and the solution was evaporated to dryness to give a dark solid residue. This material was extracted twice with hot ethyl acetate. The combined extracts were dried ($MgSO_4$) and the solvent removed to yield 1.9 g of dark solid. This material was triturated with methylene chloride and the insolubles filtered to afford 1.2 g (50 %) of Compound B as a light tan powder.

C. (2-Methyl-imidazol-4-yl)acetic acid

A suspension of 1.2 g (9.9 mmol) of Compound B and 6.3 g (20 mmol) of $Ba(OH)_2 \bullet 8H_2O$ in 25 mL of water was heated at reflux, under argon, for 3 hours. The precipitate which formed on cooling was removed by filtration and discarded. To the filtrate was added 1.9 g (20 mmol) of $(NH_4)_2CO_3$ and the resulting white slurry stirred an additional 1 hour. The solids were removed by filtration and the filter cake washed well with water. The filtrate was evaporated to dryness, and the residue evaporated twice more from water, to afford 1.3 g (9.3 mmoles, 93 %) of Compound D as an oil which crystallized on standing.

D. $N^2$-[2-(2-methyl-1H-imidazol-4-yl)-1-oxoethyl]-L-valine benzyl ester

Compound D was prepared from Compound C and valine benzyl ester • HCl as described for Compound A of Example 4. Flash chromatography (silica gel, 5% methanol - chloroform) afforded Compound D as a viscous foam.

E. $N^2$-[2-(2-methyl-1H-imidazol-4-yl)-1-thioxoethyl]-L-valine benzyl ester

Compound E was prepared as a viscous yellow oil from Compound D as described for Compound B of Example 16.

F. $N^2$-[2-(2-methyl-1H-imidazol-4-yl)ethyl]-L-valine benzyl ester

Compound F was prepared as a pale yellow oil from Compound E as described for Compound C of Example 16.

G. $N^2$-[2-(2-methyl-1H-imidazol-4-yl)ethyl]-L-valine

Compound G was prepared as a pale yellow solid from Compound F as described for Compound E of Example 16.

H. N-[(2,3-Dichlorophenyl)methyl]-$N^2$-[2-(2-methyl-1H-imidazol-4-yl)ethyl]-L-valinamide, dihydrochloride

To a suspension of 100 mg (0.44 mmmol) of Compound G in 1.5 mL of DMF under argon was added 194 mg (0.44 mmol) of BOP, 77 mg (0.44 mmol) of 2,3-dichlorobenzylamine and 87 μL (0.5 mmol) of DIEA. The suspension was allowed to stir overnight and the resulting pale yellow solution was evaporated to dryness. The residue was diluted with ethyl acetate and washed with saturated $NaHCO_3$ and brine, dried ($MgSO_4$), and the solvent removed to give a clear yellow oil residue which was subjected to flash chromatography on a 44 cc column of silica gel. Elution with trichloromethane:methanol:ammonium hydroxide (95:5:0.5) afforded 122

mg of the free base of Example 21 as a white foam. This material was subjected to prep. HPLC on a YMC S-10 120A ODS column using a gradient elution of 40-100 % B (A: 10 % methanol-water + 0.1 % TFA, B: 10 % water-methanol + 0.1 % TFA) to afford an oily residue which was taken into 1 N HCl and again evaporated to dryness. The residue was evaporated from water twice more to yield a white solid. This material was triturated with ether and the solid filtered to afford 87 mg (0.19 mmole, 43 %) of the title compound as a white powder, mp 239-241° C.
MS: $(M+H)^+$ 383.
$[\alpha]_D$ -3.0° (c 0.87, methanol).

| Analysis for $C_{18}H_{26}N_4OCl_4$-0.50 $H_2O$: | | | |
|---|---|---|---|
| Calculated: | C, 46.57; | H, 5.65; | N, 12.07. |
| Found: | C, 46.39; | H, 5.48; | N, 11.82. |

Example 22

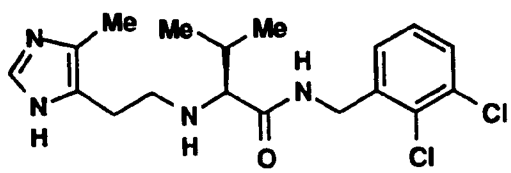

N-[(2,3-Dichlorophenyl)methyl]-$N^2$-[2-(5-methyl-1H-imidazol-4-yl)ethyl]-L-valinamide, dihydrochloride

A. 5-Methyl-4-hydroxymethylimidazole

To a suspension of lithium aluminum hydride (5.4 g, 142 mmol) in THF (200 mL) at 0°C was added ethyl 4-methylimidazole-5-carboxylate (15.4 g, 100 mmol) in portions as a slurry in THF (100 mL). The solution was stirred 24 hours at room temperature, concentrated HCl was added and the organic solvent was evaporated. Absolute ethanol was added, and the mixture was heated to boiling and filtered hot through Celite®. The filtrate was evaporated to afford 23 g of Compound 1 (>>100%) as a brown semisolid.
$^{13}C$ ($CD_3OD$) 9.27, 53.94, 127.92, 130.10, 133.67 ppm.

B. 5-Methyl-4-chloromethylimidazole

To 1 mL of thionyl chloride was added 0.35 g (2.35 mmol) of Compound A. The resulting solution was heated at 65°C for 1 hour, stirred at room temperature for 3 days, evaporated to dryness and the residue washed twice with ether to afford 0.37 g (94 %) of Compound B as a white solid.

C. 5-Methyl-4-cyanomethylimidazole

Compound C was prepared as a tan solid from Compound B as described for Compound B of Example 21.

D. (5-Methyl-imidazol-4-yl)acetic acid, ethyl ester

A solution of Compound C (2.58 g, 21.3 mmol) in 2 N NaOH (40 mL) was heated at reflux for 3.5 hours. The solution was acidified with concentrated HCl and filtered of a small amount of insoluble material. The filtrate was evaporated, the residue was dissolved in water and the solution was evaporated. The residue was extracted twice with boiling absolute ethanol and the combined extracts were evaporated to afford a mixture of the free acid and the ethyl ester of the product. The residue was dissolved in absolute ethanol (50 mL), concentrated HCl was added (2 mL) and the solution was stirred 16 hours and evaporated. The residue was suspended in 5% aqueous sodium hydrogen carbonate and the mixture was extracted 3 times with 10% isopropanol/methylene chloride. The combined organic extracts were dried (magnesium sulfate), filtered and evaporated to afford 1.82 g (48%) of Compound D as a tan oil which crystallized on standing.

$^{13}C$ ($CDCl_3$) 10.28, 14.17, 32.14, 60.93, 133.33, 171.43 ppm.

E. (5-Methyl-imidazol-4-yl)acetic acid

Compound E was prepared as a crystalline solid from Compound D as described for Compound C of Example 21.

F. $N^2$-[2-(5-methyl-1H-imidazol-4-yl)-1-oxoethyl]-L-valine benzyl ester

Compound F was prepared from Compound E and valine benzyl ester • HCl as described for Compound A of Example 4. Flash chromatography (silica gel, 5% methanol - chloroform) afforded Compound D as a viscous foam.

G. $N^2$-[2-(5-methyl-1H-imidazol-4-yl)-1-thioxoethyl]-L-valine benzyl ester

Compound G was prepared as a viscous oil from Compound F as described for Compound B of Example 16.

H. $N^2$-[2-(5-methyl-1H-imidazol-4-yl)ethyl]-L-valine, benzyl ester

Compound H was prepared as a pale yellow solid from Compound G as described for Compound G of Example 16.

I. $N^2$-[2-(5-methyl-1H-imidazol-4-yl)ethyl]-L-valine

Compound I was prepared as a white solid from Compound H as described for Compound E of Example 16.

J. N-[(2,3-Dichlorophenyl)methyl]-$N^2$-[2-(5-methyl-1H-imidazol-4-yl)ethyl]-L-valinamide, dihydrochloride

The title compound was prepared as a white lyophilate from Compound I as described for Compound H of Example 21.
MS: $(M+H)^+$ 383.
$[\alpha]_D$ -3.2° (c 0.85, methanol).

| Analysis for $C_{18}H_{26}N_4OCl_4$-2.19 $H_2O$: | | | |
|---|---|---|---|
| Calculated: | C, 43.62; | H, 6.18; | N, 11.30. |
| Found: | C, 43.73; | H, 5.88; | N, 11.19. |

Example 23

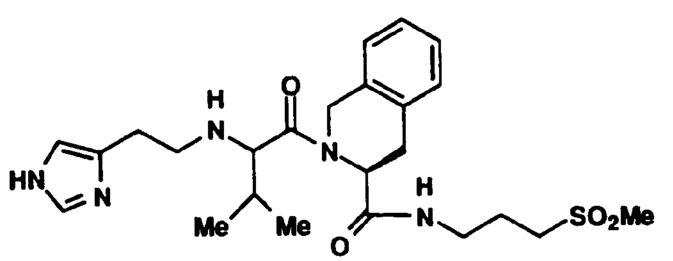

(3S)-1,2,3,4-Tetrahydro-2-[N-[2-(1H-imidazol-4-yl)ethyl]-D,L-valyl]-N-[3-(methylsulfonyl)propyl]-3-isoquinolinecarboxamide, trifluoroacetate (1:2)

A. [(Imidazol-4-yl)ethyl]-D,L-valine, ethyl ester

To a solution of histamine dihydrochloride (5.96 g, 32.4 mmol) and ethyl 2-oxo-3-methylbutyrate (5.14 g, 35.6 mmol) in 160 mL of dry methanol was added 8 g of 3Å molecular sieves. The mixture was stirred for 15 minutes. To this was added 800 mg of 10% palladium on carbon, and the resulting mixture was stirred under hydrogen atmosphere (balloon) for 24 hours and filtered, and the solids were washed with 50 mL of 1:1 methanol-water. The filtrate was concentrated *in vacuo*. The residue was chased several times with toluene/methanol to give 9.43 g (93%) of white solid. The free amine was obtained as follows. A suspension of 5 g of the dihydrochloride salt in 250 mL of 10% isopropanol/dichloromethane was washed with 100 mL of saturated aqueous $NaHCO_3$. The aqueous layer was extracted with 100 mL of 10% isopropanol/dichloromethane. The aqueous layer was then saturated with sodium chloride and re-extracted with 10% isopropanol/dichloromethane (2 x 100 mL). The combined organic extracts were dried over sodium sulfate, filtered, and concentrated *in vacuo* to afford 3.43 g (85% overall) of Compound A as a white solid.

B. [(N-Triphenylmethyl-imidazol-4-yl)ethyl]-D,L-valine, ethyl ester

Compound B was prepared from Compound A as described for Compound A of Example 6, using acetonitrile as solvent and using 25-100% ethyl acetate/hexanes for chromatography.
$(M+H)^+$ 482.

C. $N^2$-[(2,2,2-Trichloroethoxycarbonyl)]-$N^2$-[2-(1-triphenylmethyl-imidazol-4-yl)ethyl]-D,L-valine, ethyl ester

To a solution of Compound B (1.88 g, 3.90 mmol) in 20 mL of THF at 0°C was added 20 mL of saturated aqueous $NaHCO_3$. 2,2,2-Trichloroethyl chloroformate (0.96 g, 81% by wt., 4.29 mmol) in 4 mL of THF was added dropwise over 5 minutes with stirring. The resulting mixture was stirred at 0°C for 30 minutes, warmed to room temperature and concentrated *in vacuo* to remove the THF. The aqueous mixture was extracted with ethyl acetate, and the organic layer was washed with 25 mL each of saturated aqueous $NaHCO_3$ and brine. The organic layer was dried over sodium sulfate, filtered, and concentrated *in vacuo*. The residue (2.15 g) was chromatographed (eluting with 20-40% ethyl acetatehexane) to provide 1.52 g (62%) of Compound C.

D. $N^2$-[(2,2,2-Trichloroethoxycarbonyl)]-$N^2$-[2-(1-triphenylmethyl-imidazol-4-yl)ethyl]-D,L-valine

To a solution of Compound C (1.00 g, 1.60 mmol) in 10 mL of ethanol was added 5 mL of 1M lithium hydroxide. The cloudy mixture was heated at 50°C for 12 hours. An additional 1.6 mL of 1M LiOH was added to the reaction mixture and the mixture was heated at 50°C for 6 hours, cooled to room temperature and treated with 6.6 mL of 1M HCl. The mixture was concentrated *in vacuo* to remove the ethanol. The aqueous mixture was extracted with dichloromethane (3 x 50 mL). The combined organic layers were dried over sodium sulfate, filtered, and concentrated *in vacuo* to afford 911 mg (95%) of Compound D.

E. (3S)-1,2,3,4-Tetrahydro-2-[$N^2$- [(2,2,2-trichloroethoxycarbonyl)]-$N^2$-[2-(1-triphenylmethyl-imidazol-4-yl)ethyl]-D,L-valyl]-3-isoquinolinecarboxylic acid, methyl ester

To a mixture of Compound C (660 mg, 1.10 mmol), methyl L-1,2,3,4-tetrahydroisoquinoline-3-carboxylate (421 mg, 2.20 mmol), and HOBT (297 mg, 2.20 mmol) in 5 mL of dichloromethane were added 0.2 mL of DIEA and EDC (317 mg, 1.65 mmol). The mixture was stirred for 24 hours, additional EDC (211 mg, 1.10 mmol) and 1 mL of DMF were added, and the mixture was stirred for 48 hours. The mixture was diluted with ethyl acetate (100 mL) and washed with 50 mL each of water, pH 4 phosphate buffer, saturated aqueous $NaHCO_3$ and brine. The organic layer was dried over sodium sulfate, filtered, and concentrated *in vacuo*. The residue (855 mg) was chromatographed (20-60% ethyl acetate-hexane) to provide 428 mg (50%) of Compound E. $(M+H)^+$ 771.

F. (3S)-1,2,3,4-Tetrahydro-2-[$N^2$-[(2,2,2-trichloroethoxycarbonyl)]-$N^2$-[2-(1-triphenylmethyl-imidazol-4-yl)ethyl]-D,L-valyl]-3-isoquinolinecarboxylic acid

Compound F was prepared from Compound E as described for Compound D of Example 23, with the reaction run at 0°C and workup with dilute HCl.

G. (3S)-1,2,3,4-Tetrahydro-2-[$N^2$- [(2,2,2-trichloroethoxycarbonyl)]-$N^2$-[2-(1-triphenylmethyl-imidazol-4-yl)ethyl]-D,L-valyl]-N-[3-(methylsulfonyl)propyl]-3-isoquinolinecarboxamide

To a solution of Compound F (168 mg, 0.222 mmol) in 2 mL of 3:1 acetonitrile-DMF were added methyl 3-aminopropylsulfone (58 mg, 0.33 mmol), BOP reagent (150 mg, 0.33 mmol) and 0.12 mL of DIEA. The mixture was stirred for 18 hours, concentrated *in vacuo*, diluted with ethyl acetate (50 mL) and washed with 25 mL each of 10% citric acid, saturated aqueous $NaHCO_3$ and brine. The organic layer was dried over sodium sulfate, filtered and concentrated *in vacuo*. The residue (236 mg) was chromatographed (50-100% ethyl acetate-hexane, then 5% methanol-ethyl acetate) to provide 186 mg (96%) of Compound G. $(M+H)^+$ 876.

H. (3S)-1,2,3,4-Tetrahydro-2-[N-[2-(1H-imidazol-4-yl)ethyl]-D,L-valyl]-N-[3-(methylsulfonyl)propyl]-3-isoquinolinecarboxamide, trifluoroacetate (1:2)

To a solution of Compound G (170 mg, 0.194 mmol) in 1.5 mL of dichloromethane were added triethylsilane (0.19 mL, 1.2 mmol) and 1.5 mL of TFA. The mixture was stirred for 5 hours and concentrated *in vacuo*. The residue was triturated with hexane (2 x 2 mL) and diethyl ether (2 x 2 mL). The remaining oil (160 mg) was purified by preparative HPLC (YMC S-10 ODS 30 x 500 mm, 20 mL/minute, 20-100% aqueous methanol with 0.1% TFA, 60 minutes) and the concentrated fractions were freeze-dried to provide 113 mg (83%) of the title compound as a white solid.
MS: $(M+H)^+$ 490

Example 24

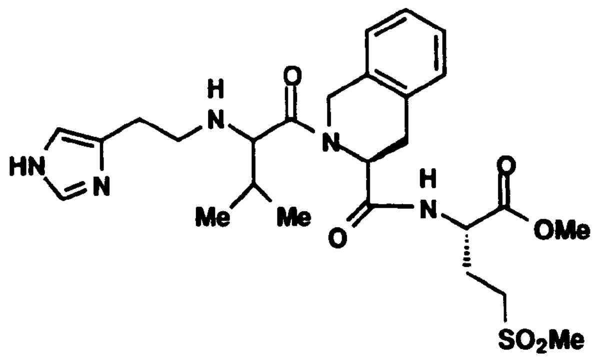

(3S)-N-[[1,2,3,4-Tetrahydro-2-[N-[2-(1H-imidazol-4-yl)ethyl]-D,L-valyl]-3-isoquinolinyl]carbonyl]-3-[(methylsulfonyl)methyl]-L-alanine, methyl ester, trifluoroacetate (1:2)

A. (3S)-N-[[1,2,3,4-Tetrahydro-2-[$N^2$-[(2,2,2-trichloroethoxycarbonyl)]-$N^2$-[2-(1-triphenylmethyl-imidazol-4-yl) ethyl]-D,L-valyl]-3-isoquinolinyl]carbonyl]-3-[(methylsulfonyl)methyl]-L-alanine, methyl ester

Compound A was prepared from Compound F of Example 23 and L-methionine methyl ester hydrochloride as described for Compound G of Example 23. $(M+H)^+$ 934.

B. (3S)-N-[[1,2,3,4-Tetrahydro-2-[N-[2-(1H-imidazol-4-yl)ethyl]-D,L-valyl]-3-isoquinolinyl]carbonyl]-3-[(methylsulfonyl)methyl]-L-alanine, methyl ester, trifluoroacetate (1:2)

The title compound was prepared as a white solid from Compound A as described for Compound H of Example 23.
MS: $(M+H)^+$ 548

Example 25

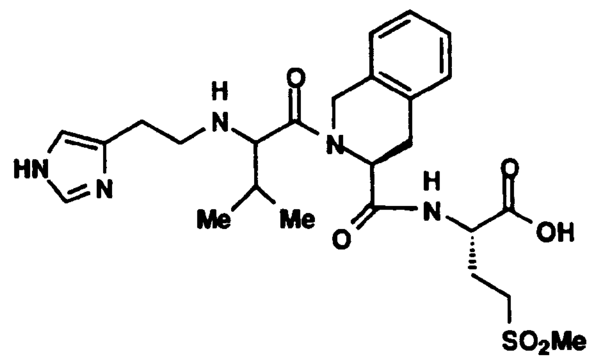

(3S)-N-[[1,2,3,4-Tetrahydro-2-[N-[2-(1H-imidazol-4-yl)ethyl]-D,L-valyl]-3-isoquinolinyl]carbonyl]-3-[(methylsulfonyl)methyl]-L-alanine, trifluoroacetate (1:2)

A. (3S)-N-[[1,2,3,4-Tetrahydro-2-[$N^2$-[(2,2,2-trichloroethoxycarbonyl)]-$N^2$-[2-(1-triphenylmethyl-imidazol-4-yl) ethyl]-D,L-valyl]-3-isoquinolinyl]carbonyl]-3-[(methylsulfonyl) methyl]-L-alanine

To a solution of Compound A of Example 24 (100 mg, 0.107 mmol) in 1.0 mL of methanol was added 0.30 mL of 1M LiOH. The mixture was stirred for 12 hours, treated with 0.3 mL of 1M HCl and concentrated *in vacuo*. The mixture was diluted with 25 mL of dichloromethane and washed with 1:1 1M HCl-brine. The aqueous layer was extracted with 100 mL of dichloromethane. The combined organic layer was dried over sodium sulfate, filtered and concentrated *in vacuo* to afford 95 mg (97%) of Compound A. $(M+H)^+$ 920.

B. (3S)-N-[[1,2,3,4-Tetrahydro-2-[N-[2-(1H-imidazol-4-yl)ethyl]-D,L-valyl]-3-isoquinolinyl]carbonyl]-3-[(methylsulfonyl)methyl]-L-alanine, trifluoroacetate (1:2)

To a solution of Compound A (95 mg, 0.103 mmol) in 1.0 mL of dichloromethane were added triethylsilane (0.10 mL) and 1.0 mL of TFA. The mixture was stirred at room temperature for 3.5 hours and concentrated *in vacuo*. The residue was triturated with 1 mL of hexane. The remaining oil was purified by preparative HPLC (YMC S-10 ODS 30 x 500 mm, 20 mL/minute, 20-100% aqueous methanol with 0.1% TFA) and the concentrated fractions were freeze-dried to provide 47 mg (59%) of the title compound as a white solid.
MS: $(M+H)^+$ 534

Example 26

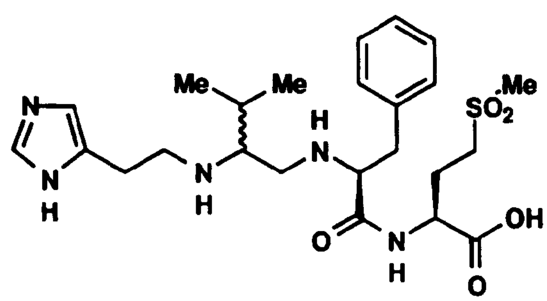

N-[(S)-2-[[2-[[2-(1H-imidazol-4-yl)ethyl]amino]-3-methylbutyl]amino]-1-oxo-3-phenylpropyl]-3-[(methylsulfonyl)methyl]-L-alanine, trifluoroacetate

A. $N^2$-[(2,2,2-Trichloroethoxycarbonyl)]-$N^2$-[2-(1-triphenylmethyl-imidazol-4-yl)ethyl]-D,L-valinal

To a solution of Compound C of Example 23 (507 mg, 0.81 mmol) in toluene (5 mL) at -78°C was added a solution of diisobutylaluminum hydride (2.7 mL of 1.5 M in toluene, 4.05 mmol) at such a rate that the reaction temperature was maintained below -65°C. The mixture was stirred at -78°C for 4 hours, quenched with methanol (2 mL) below -65°C, warmed to room temperature, and aqueous $NaHCO_3$ solution (2 mL) was added. The mixture was filtered through a Celite® pad and the pad was washed with ethyl acetate (20 mL). The filtrate was washed with brine, dried (sodium sulfate) and concentrated. The residue was purified by flash column chromatography on silica eluting with hexanes : ethyl acetate (1 : 1) to afford Compound A (268 mg, 57%) as a gel. MS $(M+H)^+$582.

B. N-[(S)-2-[N-[(2,2,2-Trichloroethoxycarbonyl)-[2-(1-triphenylmethyl-imidazol-4-yl)ethyl]amino]-3-methylbutyl]amino]-3-phenylpropanoic acid, methyl ester

A mixture of Compound A (260 mg, 0.45 mmol) and phenylalanine methyl ester HCl salt (116 mg, 0.54 mmol) in methanol (2.5 mL) and acetic acid (0.2 mL) was stirred for 15 minutes. To this solution was added sodium cyanoborohydride (30 mg, 0.48 mmol) in portions over 10 minutes. After stirring for 1 hour, the mixture was concentrated and partitioned between ethyl acetate (20 mL) and aqueous $NaHCO_3$ solution (20 mL). The organic layer was washed with brine, dried ($Na_2SO_4$) and concentrated. The residue was purified by flash column chromatography (silica, hexanes : ethyl acetate 1: 1) to yield Compound B (230 mg, 69%) as a foam. MS $(M+H)^+$ 745.

C. N-[(S)-2-[N-[(2,2,2-[Trichloroethoxycarbonyl)-[2-(1-triphenylmethyl-imidazol-4-yl)ethyl]amino]-3-methylbutyl]amino]-3-phenylpropanoic acid

A mixture of Compound B (230 mg, 0.31 mmol) in methanol (2 mL) and aqueous lithium hydroxide solution (0.62 mL of 1 N, 0.62 mmol) was stirred for 24 hours. Most of the solvent was removed and the residue was triturated with hexanes twice. The residue was cooled to 0°C and acidified with aqueous HCl solution (0.62 mL of 1 N). The resulting mixture was partitioned between ethyl acetate (20 mL) and brine (20 mL). The organic layer was dried ($Na_2SO_4$) and concentrated to afford Compound C (230 mg, 100%) as a foam. MS $(M+H)^+$ 731.

D. N-[(S)-2-[[2-[N-[(2,2,2-Trichloroethoxycarbonyl)[2-(1-triphenylmethyl-imidazol-4-yl)ethyl]amino]]-3-methylbutyl]amino]-1-oxo-3-phenylpropyl]-3-[(methylsulfonyl)methyl]-L-alanine, methyl ester

To a mixture of Compound C (230 mg, 0.31 mmol), methionine sulfone methyl ester HCl salt (86 mg, 0.37 mmol) and HOBT (47.4 mg, 0.31 mmol) in DMF (1 mL) was added NMM (55 mg, 0.55 mmol), followed by EDC (71.3 mg, 0.372 mmol) at 0°C. The mixture was stirred overnight at room temperature and partitioned between ethyl acetate (20 mL) and water (10 mL). The organic layer was washed with brine, dried over $MgSO_4$ and concentrated. The residue was purified by flash column chromatography on silica eluting with ethyl acetate to afford Compound D (181 mg, 64%) as a foam. MS $(M+H)^+$ 908.

E. N-[(S)-2-[[2-[N-(2,2,2-Trichloroethoxycarbonyl)[2-(1-triphenylmethylimidazol-4-yl)ethyl]amino]]-3-methylbutyl]amino]-1-oxo-3-phenylpropyl]-3-[(methylsulfonyl)methyl]-L-alanine

Compound E was prepared as a foam from Compound D as described for Compound A of Example 25. MS $(M+H)^+$ 894.

F. N-[(S)-2-[[2-[[2-(1H-imidazol-4-yl)ethyl]amino]-3-methylbutyl]amino]-1-oxo-3-phenylpropyl]-3-[(methylsulfonyl)methyl]-L-alanine, trifluoroacetate

The title compound was prepared as a solid from Compound E as described for Compound B of Example 25.
M.P. 55-60 °C (softened).
MS $(M+H)^+$ 301.

| Analysis for $C_{24}H_{37}N_5O_5S$-1.0 $H_2O$-3.4 $CF_3CO_2H$: | | | | |
|---|---|---|---|---|
| Calculated: | C, 40.50; | H, 4.68; | N, 7.67; | F, 21.22. |
| Found: | C, 40.50; | H, 4.76; | N, 7.64; | F, 21.31. |

Example 27

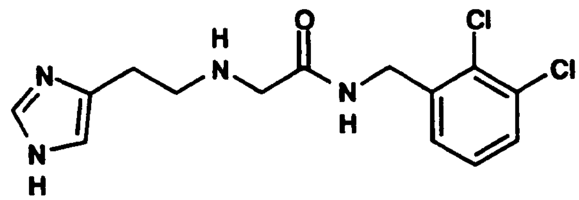

N-[(2,3-Dichlorophenyl)methyl]-2-[[2-(1H-imidazol-4-yl)ethyl]amino]acetamide

A. [2-(Imidazol-4-yl)ethyl]aminoacetic acid, methyl ester

To a stirred solution of histamine dihydrochloride (2.0 g, 10.8 mmol) and glyoxylic acid (1.10 g, 11.9 mmol) in methanol (40 mL), 5 g of 3Å molecular sieves were added. After stirring for 15 minutes, 10% palladium on carbon (0.2 g) was added and the slurry was stirred under hydrogen atmosphere (balloon) for 16 hours. The mixture was filtered through a pad of Celite® and the pad was washed with methanol several times. HCl gas was bubbled through the filtrate and the solution was stirred overnight. The solution was concentrated, the residue was suspended in 10% isopropanol/dichloromethane and the solution was washed with $NaHCO_3$ (35 mL). The aqueous layer was reextracted with 10% isopropanol/dichloromethane (2 x 35 mL) and the combined extracts were dried over $MgSO_4$, filtered and concentrated to afford Compound A (0.43 g, 24 %).

B. [2-(N-Triphenylmethyl-imidazol-4-yl)ethyl]aminoacetic acid, methyl ester

A solution of Compound A (0.55 g, 2.1 mmol) and chlorotriphenylmethane (0.60 g, 2.1 mmol) was stirred in acetonitrile 14 hours. The mixture was concentrated, the residue was diluted with methylene chloride (75 mL) and the solution was washed with $NaHCO_3$ (25 mL), brine (25 mL), dried over $MgSO_4$, filtered and concentrated. Purification on silica gel eluting with a step gradient of 25 % ethyl acetate/hexane, ethyl acetate, and 1% methanol/trichloromethane afforded Compound B.

C. N-[[(1,1-Dimethylethoxy)-carbonyl]-[2-(N-triphenylmethyl-imidazol-4-yl)ethyl]]aminoacetic acid, methyl ester

A solution of Compound B (0.5 g, 1.2 mmol), DMAP (0.05 g), Boc-anhydride (0.33 g, 1.52 mmol) and triethylamine (0.13 g, 0.17 mL, 1.29 mmol) was stirred in dichloromethane (6 mL) under argon for 14 hours and partitioned between chloroform (30 mL) and brine (10 mL). The aqueous layer was extracted (2 x 30 mL) and the combined organic extracts were washed with 10 % $KHSO_4$ (2 x 10 mL) and brine (2 x 10 mL), dried over MgSO4, filtered and concentrated to afford Compound C (0.578 g, 94%).

D. N-[[(1,1-Dimethylethoxy)-carbonyl]-[2-(N-triphenylmethyl-imidazol-4-yl)ethyl]]aminoacetic acid

1N NaOH (0.28 mL, 0.28 mmol) was added to a solution of Compound C (0.10 g, 0.19 mmol) in methanol (1mL). After 15 hours, the reaction was diluted with ethyl acetate (20 mL) and neutralized with 10 % HCl (10 mL), the layers were separated and the aqueous layer was extracted with ethyl acetate (2 x 20 mL). The combined organic layers were washed with brine (1 x 20 mL), dried over $MgSO_4$, filtered and concentrated to afford Compound D as a glassy solid (0.80 g, 83 %).

E. N-[(2,3-Dichlorophenyl)methyl]-$N^2$-[[(1,1-dimethylethoxy)-carbonyl]-[2-(N-triphenyl methyl-imidazol-4-yl) ethyl]]aminoacetamide

A solution of Compound D (0.080 g, 0.156 mmol), HOBT (0.026 g, 0.17 mmol) 3,4 dichlorobenzylamine (0.027 g, 0.15 mmol), EDC (0.032 g, 0.17 mmol) and DIEA (0.022 g, 0.030 mL, 0.17 mmol) was stirred in THF/DMF (4 mL/1.5 mL) under argon for 13 hours. The mixture was partitioned between 10% HCl (10 mL) and ethyl acetate (20 mL), the aqueous layer was extracted with ethyl acetate (2 x 20 mL) and the combined organic layers were washed with water (20 mL), $NaHCO_3$ (2 x 20mL mL), brine (2 x 20 mL) dried over $MgSO_4$, filtered and concentrated. The product was purified on silica gel, eluting with a step gradient of trichloromethane and trichloromethane / methanol (95/5) to afford Compound E (0.078 g, 75%).

F. N-[(2,3-Dichlorophenyl)methyl]-2-[[2-(1H-imidazol-4-yl)ethyl]amino]acetamide

A solution of triethylsilane (0.035 mL, 0.22 mmol), TFA (0.5 mL) and Compound E (0.075 g, 0.11 mmol) were stirred in dichloromethane (2 mL) under argon for 16 hours and concentrated. The residue was triturated with hexanes/ethyl acetate and lyophilized to afford the title compound (43.1 mg, 69 %) :
MS $(M+H)^+ = 327^+$, $(M-H)^- = 325^-$.

Example 28

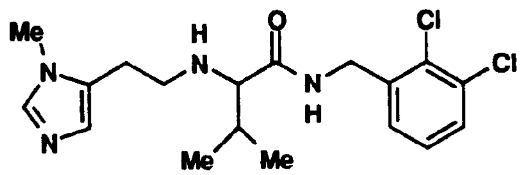

N-[(2,3-Dichlorophenyl)methyl]-$N^2$-[2-(1-methyl-1H-imidazol-5-yl)ethyl]-D,L-valinamide, dihydrochloride

A. [(1-Methyl-imidazol-5-yl)ethyl]-D,L-valine, ethyl ester

Compound A was prepared from 3-methylhistamine dihydrochloride and ethyl 2-oxo-3-methylbutyrate as described for Compound A of Example 23.

B. [(1-Methyl-imidazol-5-yl)ethyl]-D,L-valine

Compound B was prepared from Compound A as described for Compound E of Example 16, using water as the reaction solvent.

C. N-[(2,3-Dichlorophenyl)methyl]-$N^2$-[2-(1-methyl-1H-imidazol-5-yl)ethyl]-D,L-valinamide, dihydrochloride

Compound C was prepared as a white solid from Compound B as described for Compound E of Example 19, with the coupling reaction stirred for 24 hours.
MS $(M+H)^+ = 383^+$.
$^1$H NMR ($CD_3OD$) δ 1.06 (d, J=6.8, 3H), 1.12 (d, J=7.3, 3H), 2.25-2.35 (m, 1H), 3.15-3.45 (m, 4H), 3.89 (s, 3H), 3.91 (d, J=5.1, 1H), 4.5-4.7 (m, 2H), 7.29 (m, 1H), 7.40-7.55 (m, 3H), 8.90 (s, 1H), 9.0 (m, 1H).

Example 29

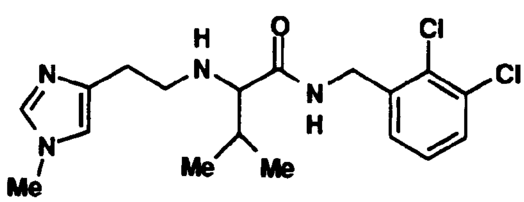

N-[(2,3-Dichlorophenyl)methyl]-$N^2$-[2-(1-methyl-1H-imidazol-4-yl)ethyl]-D,L-valinamide, trifluoroacetate
The title compound was prepared from 1-methylhistamine dihydrochloride using the three step procedure described in the preparation of Example 28.
MS $(M+H)^+ = 383^+$.
$^1$H NMR ($CD_3OD$) δ 1.02 (d, J=6.8, 3H), 1.08 (d, J=7.3, 3H), 2.20-2.30 (m, 1H), 3.1-3.4 (m, ~4H), 3.78 (d, J=5.1, 1H), 3.90 (s, 3H), 4.59 (ABq, J=15.0, Δδ=0.05, 2H), 7.29 (m, 1H), 7.40 (m, 2H), 7.50 (d, J=7.7, 1H), 8.81 (s, 1H).

Example 30

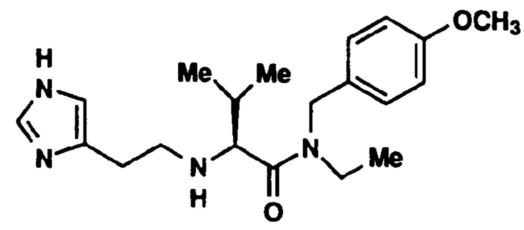

N-Ethyl-$N^2$-[2-(1H-imidazol-4-yl)ethyl]-N-[(4-methoxyphenyl)methyl]-L-valinamide

A. N-[(1,1-Dimethylethoxy)-carbonyl]-N-[1-triphenylmethyl-imidazol-4-yl-ethyl]-L-valine

A slurry of 10 g (32.2 mmol) of Compound E of Example 16 in 150 mL of dry methylene chloride was treated with 4.4 mL (40.1 mmol) of NMM, and the slurry cooled in an ice bath under a blanket of argon. A 1M solution of trimethylsilyl chloride in methylene chloride (40mL) was added over 15 minutes, and the ice bath was removed. After stirring for another 2.5 hours, a second portion of NMM was added (3.85 mL, 35 mmol) followed by 9.75 g (35 mmol) of triphenylmethyl chloride. The mixture was stirred overnight at room temperature, treated with 150 mL of methanol and stirred an additional 3 hours. Evaporation *in vacuo* gave a solid. This was slurried in 500 mL of methylene chloride and poured on to a pad of 600 mL of dry K-60 silica. The pad was washed with 6 X 500 mL of methylene chloride, then with 4 X 100 mL of 5% methanol in methylene chloride. The first 5% wash contained material which was pure by TLC (Rf = 0.29, 5% methanol in methylene chloride). Evaporation *in vacuo* gave 10.8 g of Compound A as a light yellow foam. Less pure material was also obtained, which was chromatographed again under identical conditions to afford a sticky foam. This material was covered with 200 mL of heptane and 15 mL of diethyl ether. Standing overnight at room temperature gave a solid which was pulverized, filtered, washed with heptane and dried *in vacuo* to give an additional 4.5 g of Compound A as a powder (86% total).

B. N-Ethyl-$N^2$-[2-(1H-imidazol-4-yl)ethyl]-N-[(4-methoxyphenyl)methyl]-L-valinamide

A mixture of Compound A (29 mg, 0.05 mmol), a solution of N-ethyl-p-methoxybenzylamine in methylene chloride (200 μL of 0.25 M solution, 0.05 mmol), a solution of HOAt in DMF (200 μL of 0.3 M solution, 0.06 mmol), and a solution of diisopropylcarbodiimide in methylene chloride (200 μL of 0.3 M solution, 0.06 mmol) was allowed to stand in a sealed vessel for 2 days. TFA (1 mL) and triethylsilane (0.1 mL) were added and the mixture was allowed to stand for an additional 2 hours. The mixture was concentrated *in vacuo* and the residue was dissolved in 1:1 methylene chloride : methanol (3 mL). The solution was applied to a solid phase extraction cartridge containing 1 g of SCX resin (Varian). The cartridge was washed sequentially with the following: 10 mL of 1:1 methylene chloride : methanol, 10 mL of methanol, 10 mL of 0.01N ammonia in methanol, and 10 mL of 0.1N ammonia in methanol. The above washes were discarded. The cartridge was then eluted with 10 mL of 1.0 N ammonia in methanol. The eluant was collected and concentrated *in vacuo* to give the title compound as a colorless oil (20 mg, 100%).
MS: $(M+H)^+$ 359.

Example 31

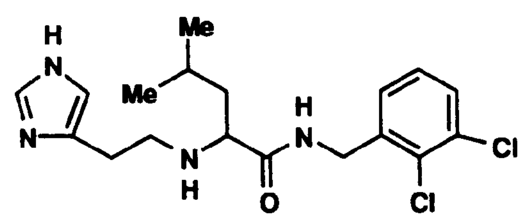

N-[(2,3-Dichlorophenyl)methyl]-2-[[2-(1H-imidazol-4-yl) ethyl]amino]-3-methylpentanamide, dihydrochloride

A. [2-[(1H-imidazol-4-yl)ethyl]amino]-3-methylpentanoic acid

To a stirred solution of histamine dihydrochloride (2.0 g, 10.8 mmol) and 4-methyl-2 oxopentanoic acid sodium salt (1.81 g, 11.9 mmol) in methanol (40 mL), 5 g of 3Å molecular sieves were added . After stirring for 30 minutes, 10% palladium on carbon (0.2 g) was added and the slurry was stirred under hydrogen atmosphere (balloon) for 14 hours. The mixture was filtered through a pad of Celite®, the pad was washed with methanol several times and the combined filtrates were concentrated to afford Compound A as a solid (3.52 g, >100%).

B. N-[(2,3-Dichlorophenyl)methyl]-2-[[2-(1H-imidazol-4-yl)ethyl]amino]-3-methylpentanamide, dihydrochloride

The title compound was prepared from Compound A and 2,3-dichlorobenzylamine as described for Compound E of Example 27. Purification by preparative HPLC (YMC, S -10, ODS, 30 x 500 mm, gradient from 10-90 % aqueous methanol containing 0.1 % TFA) afforded the title compound (0.11 g, 13%).
MS: M+H = 383.

| Analysis calculated for $C_{18}H_{24}N_4OCl_2$-2.2 HCl : 1.0 $H_2O$: | | | |
|---|---|---|---|
| Calculated: | C, 44.90; | H, 5.90; | N, 11.63. |
| Found: | C, 44.64; | H, 5.78; | N, 11.42. |

Example 32

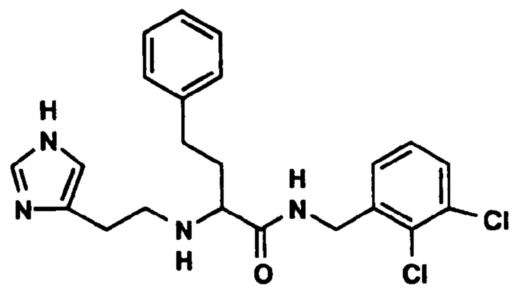

N-[(2,3-Dichlorophenyl)methyl]-2-[[2-(1H-imidazol-4-yl)ethyl]amino]-4-phenylbutanamide, trifuoroacetate

A. [[2-(1H-imidazol-4-yl)ethyl]amino]-4-phenylbutanoic acid ethyl ester

Compound A was prepared from ethyl 2-oxo-4-phenylbutyrate as described for Compound A of Example 31.

B. [[2-(1H-imidazol-4-yl)ethyl]amino]-4-phenylbutanoic acid

NaOH (8.28 mL, 8.28 mmol) was added to a solution of Compound A (1.0 g, 3.48 mmol) in methanol (15 mL). After stirring for 15 hours, the reaction was diluted with ethyl acetate (20 mL) and neutralized with 1N HCl (8.3 mL), the layers were separated, and the aqueous layer was extracted with 10 % isopropyl alcohol in dichloromethane (3 x 30 mL). The combined organic layers were concentrated to afford Compound B as a white solid (1.04 g, 115 %). MS : M+H+ = 274.

C. N-[(2,3-Dichlorophenyl)methyl]-2-[[2-(1H-imidazol-4-yl)ethyl]amino]-4-phenylbutanamide, trifuoroacetate

The title compound was prepared from Compound B as described for Compound B of Example 31.
MS: M+H = 431.

| Analysis calculated for $C_{22}H_{24}N_4OCl_2$-2.2 $CF_3CO_2H$ : 1.0 $H_2O$: | | | |
|---|---|---|---|
| Calculated: | C, 45.24; | H, 4.05; | N, 7.99. |
| Found: | C, 45.25; | H, 3.74; | N, 7.95. |

Example 33

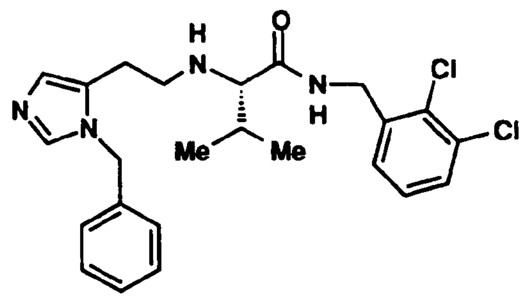

N-[(2,3-Dichlorophenyl)methyl]-$N^2$-[2-[1-(phenylmethyl)-(1H-imidazol-5-yl)]ethyl]-L-valinamide, dihydrochloride

A. $N^2$-[(1,1-Dimethylethoxy)-carbonyl]-$N^2$-[2-(1H-imidazol-4-yl)ethyl]-N-(2,3-dichlorophenylmethyl)-L-valinamide

DIEA (0.674 ml, 3.87 mmol) was added to a solution of Compound E of Example 16 (1.0 g, 3.23 mmol,), 2,3-dichlorobenzylamine (0.625 g, 3.87 mmol) and BOP (1.71 g, 3.87 mmol) in 1:1 dichloromethane/DMF (20 mL). The mixture was stirred for 16 hours, quenched with 10% $NaHCO_3$ (20 mL) and extracted with ethyl acetate (3 X 70 mL). The combined organic extracts were washed with 10% LiCl (2 X 50 mL), dried ($MgSO_4$), filtered and concentrated under vacuum. The residue was purified by flash chromatography (eluting 19/1 chloroform / methanol) to afford Compound A (1.45 g, 96%).
MS: $(M+H)^+$ 469.

B. $N^2$-[(1,1-Dimethylethoxy)-carbonyl]-$N^2$-[2-(1-[(1,1-dimethylethoxy)-carbonyl]imidazol-4-yl)ethyl]-N-(2,3-dichlorophenylmethyl)-L-valinamide

Triethylamine (0.486 mL, 3.49 mmol) was added to a solution of Compound A (0.47 g, 1.0 mmol) and Boc anhydride (0.39 g, 1.79 mmol) in dichloromethane (30 mL). The mixture was stirred 19 hours and quenched with water (50 ml). The organic layer was separated and the aqueous layer extracted with dichloromethane (2 X 50 mL). The organic layer was dried ($MgSO_4$), filtered and concentrated under vacuum to afford Compound B (0.466 g, 82%).
MS: $(M+H)^+$ 569.

C. N-[(2,3-Dichlorophenyl)methyl]-$N^2$-[2-[1-(phenylmethyl)-(1H-imidazol-5-yl)]ethyl]-L-valinamide, dihydrochloride

Benzyl alcohol (0.014 mL, 0.135 mmol) was added to a -78°C solution of triflic anhydride (0.023 mL, 0.135 mmol) and DIEA (0.024 mL, 0.135 mmol) in dichloromethane (1 mL). The mixture was stirred for 30 minutes at -78°C. A solution of Compound B (0.070 g, 0.123 mmol) was added and the mixture was stirred an additional 4 hours at -78°C and 4 hours at room temperature. The mixture was diluted with dichloromethane (1 mL) and TFA (1 mL) was added. After stirring at room temperature for 3 hours the solution was concentrated under vacuum, purified by preparative HPLC (YMC S10 ODS 30 X 500 mm, 10-90% aqeous methanol with 0.1% TFA, 60 minute gradient, 20 mL/minute) and the appropriate fractions were concentrated under vacuum. The residue was dissolved in methanol (2 mL) and 1N HCl (2 mL) was added. The mixture was stirred 5 minutes and concentrated under vacuum. This latter procedure was repeated two times and the residue was dissolved in water (2 ml), millipore filtered and lyophilized to afford the title compound (0.037 g, 53%), mp: 140-150°C.

MS: $(M+H)^+$ 459.

| Analysis for $C_{24}H_{28}N_4OCl_2$-1.0 $H_2O$-2.0 HCl: | | | |
|---|---|---|---|
| Calculated: | C, 52.59; | H, 5.84; | N, 10.22. |
| Found: | C, 52.59; | H, 6.01; | N, 9.83. |

Example 34

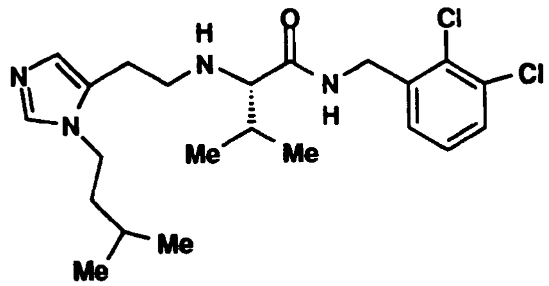

N-[(2,3-Dichlorophenyl)methyl]-N2-[2-[1-(3-methylbutyl)-1H-imidazol-5-yl]ethyl]-L-valine amide, dihydrochloride

A. N2-[(1,1-Dimethylethoxy)-carbonyl]-N2-[2-(1-(3-methylbutyl)-1H-imidazol-5-yl)ethyl]-N-(2,3-dichlorophenylmethyl)-L-valinamide

Isoamylalcohol (0.019 mL, 0.176 mmol) was added to a -78°C solution of triflic anhydride (0.030 mL, 0.176 mmol) and DIEA (0.031 mL, 0.176 mmol) in dichloromethane (0.25 mL). The mixture was stirred for 15 minutes at -78°C. A solution of Compound B of Example 33 (0.10 g, 0.176 mmol) in dichloromethane (0.25 mL) was added and the mixture was stirred an additional 4 hours at -78°C and 16 hours at room temperature. The reaction was quenched with pH buffer 7.0 (2 mL) and extracted with dichloromethane (3 X 30 mL). The combined organic extracts were dried ($MgSO_4$), filtered and concentrated under vacuum. The residue was purified by flash chromatography (19/1 chloroform / methanol) and the appropriate fractions concentrated under vacuum to afford Compound A (0.054 g, 57%), MS: $(M+H)^+$ 539.

B. N-[(2,3-Dichlorophenyl)methyl]-N2-[2-[1-(3-methylbutyl)-1H-imidazol-5-yl]ethyl]-L-valine amide, dihydrochloride

Anhydrous HCl (4 M in dioxane, 2 mL, 8.0 mmol) was added to Compound A (0.05 g, .093 mmol) and the mixture was stirred for 2 hours and concentrated under vacuum. The residue was purified by preparative HPLC (YMC S10 ODS 30 X 500 mm, 10-90% aqueous methanol with 0.1% TFA, 60 minute gradient, 20 mL/minute) and the appropriate fractions were concentrated under vacuum. The residue was dissolved in methanol (10 mL) and 1N HCl (2 mL) was added. The mixture was stirred 5 minutes and concentrated under vacuum. This latter procedure was repeated two times and the residue was dissolved in water (10 mL), millipore filtered and lyophilized to afford the title compound (39 mg, 82%), mp: 110-120°C.
MS: M+H = 439.

| Analysis for calculated for $C_{22}H_{32}N_4OCl_2$-2 HCl : 1.83 $H_2O$: | | | |
|---|---|---|---|
| Calculated: | C, 48.46; | H, 6.96; | N, 10.27. |
| Found: | C, 48.46; | H, 6.74; | N, 10.03. |

Example 35

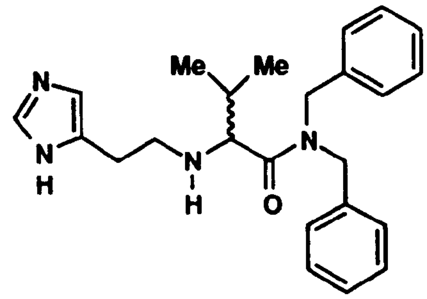

N²-[2- (1H-Imidazol-4-yl)ethyl]-N,N-bis(phenylmethyl)-D,L-valinamide, trifluoroacetate

A. [(Imidazol-4-yl)ethyl]-D,L-valine

Compound A was prepared from Compound A of Example 23 as described for Compound E of Example 16.

B. N²-[2-(1H-Imidazol-4-yl)ethyl]-N,N-bis(phenylmethyl)-D,L-valinamide, trifluoroacetate

The title compound was prepared as a white lyophilate from Compound A as described for Compound H of Example 21, except that it was isolated as a trifluoroacetic acid salt directly after preparative HPLC.
MS: $(M+H)^+$ $391^+$.

Example 36

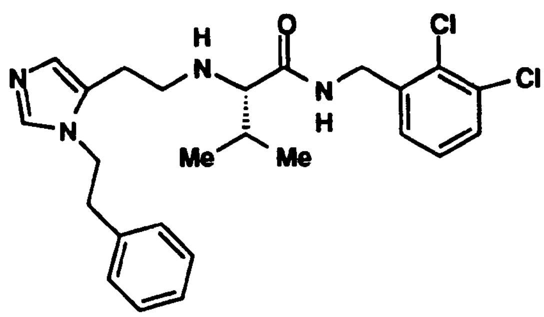

N-[(2,3-Dichlorophenyl)methyl]-N²-[2-[1-(2-phenylethyl)-1H-imidazol-5-yl]ethyl]-L-valinamide, dihydrochloride

The title compound was prepared from Compound B of Example 33 and 2-phenylethanol using the 2 step procedure reported for Example 34, mp: 83-93°C.
MS: $(M+H)^+$ 473.

| Analysis for $C_{25}H_{30}N_4OCl_2$-1.51 $H_2O$-2.0 HCl: | | | |
|---|---|---|---|
| Calculated: | C, 52.35; | H, 6.15; | N, 9.77. |
| Found: | C, 52.38; | H, 6.22; | N, 9.74. |

Example 37

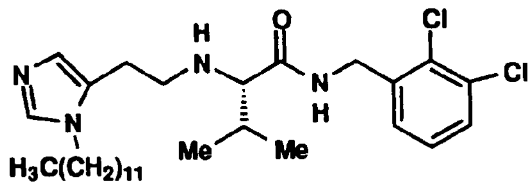

N-[(2,3-Dichlorophenyl)methyl]-$N^2$-[2-(1-dodecyl-1H-imidazol-5-yl)ethyl)-L-valinamide, dihydrochloride

The title compound was prepared from Compound B of Example 33 and 1-dodecanol using the 2 step procedure reported for Example 34,
mp: 123-133°C.
MS: $(M+H)^+$ 537.

| Analysis for $C_{29}H_{46}N_4OCl_2$-1.3 $H_2O$-2.0 HCl: | | | |
|---|---|---|---|
| Calculated: | C, 54.95; | H, 8.04; | N, 8.84. |
| Found: | C, 54.83; | H, 7.89; | N, 8.96. |

Example 38

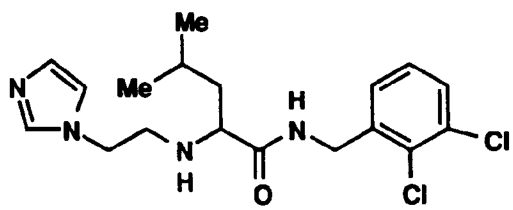

N-[(2,3-Dichlorophenyl)methyl]-2-[[2-(1H-imidazol-1-yl)ethyl]amino]-4-methylpentanamide, dihydrochloride

A. N-[2-(Imidazol-1-yl)ethyl]-phthalimide

To a solution of imidazole (680 mg, 10 mmol) and bromoethyl phthalimide (2.54 g, 10 mmol) in dry THF (15 mL) under argon was added sodium hydride (0.4 g, 10 mmol, 60% in oil). After stirring the mixture for 14 hours at room temperature, water (15 mL) and ethyl acetate (10 mL) were added and the two layers were separated. The organic layer was extracted with 1N HCl (2 x 15 mL). The acidic layer was carefully treated with solid $NaHCO_3$ (until pH 9) and extracted with chloroform (20 mL) and 10% i-propanol in chloroform (20 mL). The organic layers were combined, dried ($MgSO_4$) and concentrated *in vacuo* to afford Compound A as a white solid (230 mg, 11% yield).
MS $(M+H)^+ = 242^+$.

B. [2-(Imidazol-1-yl)ethyl]-amine, dihydrochloride

To a solution of Compound A (600 mg, 2.49 mmol) in methanol (15 mL) was added hydrazine hydrate. The mixture was stirred for 9 hours, the white precipitate was filtered through Celite® and the filtrate was concentrated. The residue was dissolved in chloroform (10 mL) and filtered, and the filtrate was extracted with 1N HCl (10 mL). The aqueous layer was concentrated to afford Compound B as a yellow solid (390 mg, 84% yield) which was used without further purification.

C. 2-[[2-(1H-imidazol-1-yl)ethyl]amino]-4-methylpentanoic acid

Compound C was prepared from Compound B and the sodium salt of 4-methyl-2-oxopentanoic acid as described for Compound A of Example 31. Trituration with dichloromethane afforded Compound C as a white solid.
MS $(M+H)^+ = 226^+$.

D. N-[(2,3-Dichlorophenyl)methyl]-2-[[2-(1H-imidazol-1-yl)ethyl]amino]-4-methyl pentanamide, dihydrochloride

To a suspension of Compound C (225 mg, 0.9 mmol) in DMF (3 mL) were added sequentially dimethylsulfoxide (DMSO) (0.5 mL), DIEA (350 μL, 2 mmol), 2,3-dichlorobenzylamine (176 mg, 1 mmol), HOAT (136 mg, 1 mmol) and EDC (191 mg, 1 mmol). The mixture was stirred under argon for 14 hours and concentrated. The residue was dissolved in 0.1% TFA in methanol and purified by preparative HPLC eluting with a linear gradient of 10% -90% aqueous methanol containing 0.1% TFA. Appropriate fractions were collected and concentrated. 10 mL of 1N HCl was added to the residue and the solution was lyophilized.
MS $(M+H)^+ = 383^+$.
$^1$H NMR : ($CD_3OD$, 400 MHz) d 9.10 (1H, s), 7.80 (1H, s), 7.65 (1H, s), 7.50 (1H, s), 7.42 (1H, s), 7.31 (1H, s), 4.80-4.50 (5H, m), 4.0 (1H, s), 3.80-3.50 (2H, m), 1.90-1.50 (3H, m), 0.98 (6H, d, J= 20 Hz).

Example 39

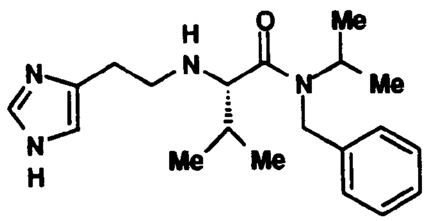

$N^2$-[2-(1H-Imidazol-5-yl)ethyl]-N-(1-methylethyl)-N-(phenylmethyl)-L-valinamide, dihydrochloride

To a solution of Compound D of Example 27 (100 mg, 0.18 mmol) in 360 μL of dry dichloromethane at 0°C were added N-isopropyl-N-benzylamine (60 μL, 0.36 mmol), DIEA (62 μL, 0.36 mmol), and PyBrop (127 mg, 0.27 mmol). The mixture was stirred at room temperature for 20 hours. Triethylsilane (50 μL) and TFA (1 mL) were added, the mixture was stirred at room temperature for 3 hours, and concentrated *in vacuo*. The residue was purified by preparative HPLC (YMC S-10 ODS 30 x 500 mm, 28 mL/minute, 20-90% aqueous methanol with 0.1% TFA over 60 minutes) and the concentrated fractions were freeze-dried. The hygroscopic lyophilate was chased with 1M HCl (1 x 2 mL, 1 x 0.5 mL) and the residue was dissolved in water and freeze-dried. The resulting yellow glass was triturated with ether, dissolved in water, and then freeze-dried to provide 26 mg (35%) of the title compound as a white solid.
MS: M+H = 343.
$^1$H NMR ($CD_3OD$) δ 1.05-1.35 (m, 12H), 2.20-2.40 (m, 1H), 3.1-3.4 (m, 2H), 3.55-3.70 (m, 2H), 4.03 (d, J=3.5, 1H) 4.20-4.30 (m, 1H), 4.61 (ABq, J=15.8, Δδ=0.22, 2H), 7.15-7.50 (m, 6H), 8.90 (s, ~0.3H, rotamer), 8.92 (s, ~0.7H, rotamer).

Example 40

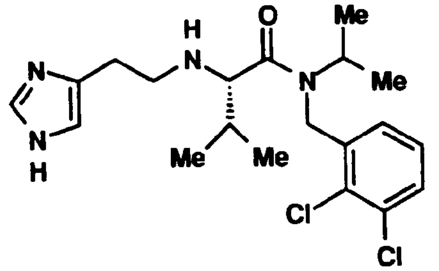

N-[(2,3-Dichlorophenyl)methyl]-$N^2$-[2-(1H-imidazol-5-yl)ethyl]-N-(1-methylethyl)-L-valinamide, dihydrochloride

A. N-[(2,3-dichlorophenyl)methyl]-N-(1-methylethyl)-amine

To a solution of isopropylamine (590 mg, 10 mmol) in 10 mL of 5% acetic acid-methanol were added 2,3-dichlorobenzaldehyde (350 mg, 2.0 mmol) and 0.5 g of 3Å molecular sieves. The mixture was stirred for 15 minutes, treated with sodium cyanoborohydride (140 mg, 2.0 mmol) and stirred for 12 hours. The mixture was filtered and the filtrate was concentrated *in vacuo*. The residue was dissolved in 20 mL of 1M HCl and extracted with diethyl ether (2 x 10 mL). The aqueous layer was made basic by adding 20 mL of 3M NaOH. This aqueous mixture was extracted with dichloromethane (2 x 20 mL). The dichloromethane extracts were dried over sodium sulfate, filtered and concentrated *in vacuo* to provide 346 mg (80%) of Compound A.

B. N-[(2,3-Dichlorophenyl)methyl]-$N^2$-[2-(1H-imidazol-5-yl)ethyl]-N-(1-methylethyl)-L-valinamide, dihydrochloride

The title compound was prepared as a white solid from Compound A as described for Example 39.
MS: M+H = 411.
$^1$H NMR ($CD_3OD$) δ 1.10-1.40 (m, 12H), 2.30-2.50 (m, 1H), 3.2-3.5 (m, ~2H) 3.99 (d, J=3.5, 1H), 4.20-4.40 (m, 1H), 4.65-4.75 (m, 2H), 4.7 (ABq, J=17, Δδ=0.2, 2H), 7.0-7.6 (m, 4H), 8.90 (s, 1H).

Example 41

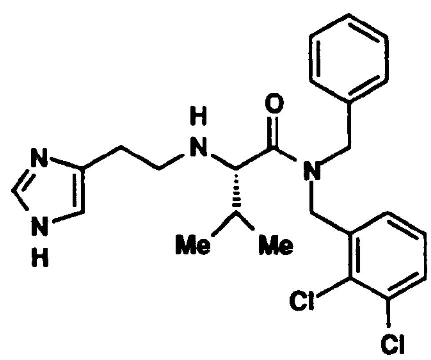

N-[(2,3-Dichlorophenyl)methyl]-$N^2$- [2-(1H-imidazol-5-yl)ethyl]-N-(phenylmethyl)-L-valinamide, dihydrochloride

A. N-[(2,3-dichlorophenyl)methyl]-N-(phenylmethyl)-amine

To a solution of benzylamine (240 mg, 2.2 mmol) in 10 mL of dry dichloromethane were added acetic acid (0.14 mL, 2.0 mmol), 2,3-dichlorobenzaldehyde (350 mg, 2.0 mmol) and sodium triacetoxyborohydride (640 mg, 3.0 mmol). The mixture was stirred for 4 hours, diluted with 50 mL of dichloromethane and washed with 50 mL of saturated aqueous $NaHCO_3$. The organic layer was dried over sodium sulfate, filtered and concentrated *in vacuo*. The residue (750 mg) was chromatographed (eluting with 50% ethyl acetatehexane) to provide 344 mg (65%) of Compound A.

B. N-[(2,3-Dichlorophenyl)methyl]-$N^2$-[2-(1H-imidazol-5-yl)ethyl]-N-(phenylmethyl)-L-valinamide, dihydrochloride

The title compound was prepared as a white solid from Compound A as described for Example 39.
MS: M+H = 459.
$^1$H NMR ($CD_3OD$) δ 1.10 (m, 3H), 1.17 (d, J=6.5, 3H), 2.25-2.45 (m, 1H), 3.1-3.4 (m, ~2H), 4.45-4.65 (m, 2H), 4.66 (ABq, J=15.9, Δδ=0.35, 2H), 5.0 (ABq, J=14.6, Δδ=0.46, 2H) 7.15-7.55 (m, 9H), 8.91 (s, 1H).

Example 42

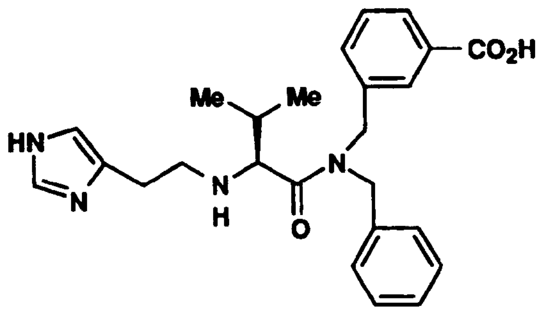

(S)-3-[[[2-[[2-(1H-Imidazol-4-yl)ethyl]amino]-3-methyl-1-oxobutyl](phenyl-methyl)amino]methyl]benzoic acid, dihydrochloride

The title compound was prepared from 3-carboxybenzaldehyde, benzylamine and Compound A of Example 30 using the 4 step procedure described for Example 17.
MS: $(M+H)^+$ 435.

Example 43

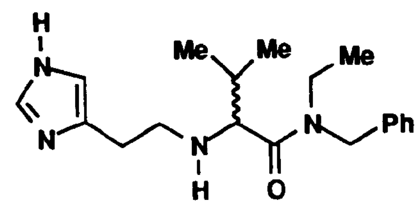

N-Ethyl-$N^2$-[2-(1H-imidazol-4-yl)ethyl]-N-(phenylmethyl)-D,L-valinamide, trifluoroacetate

The title compound was prepared as a white lyophilate from Compound A of Example 35 and N-ethyl-N-benzylamine as described for Compound B of Example 35.
MS: $(M+H)^+$ $329^+$.
$^1H$ NMR (270 MHz, DMSO-$d_6$, As a mixture of rotamers): δ 1.10 (6H, m, 1.23 (3H, m), 2.25 (1H, m), 3.15 (2H, m), 3.33 (1H, m), 3.60 (1H, m), 3.80 (1H, m), 4.50 (2H, m), 5.05 (1H, m), 7.3-7.6 6H, m), 9.04 (1H, s).

Example 44

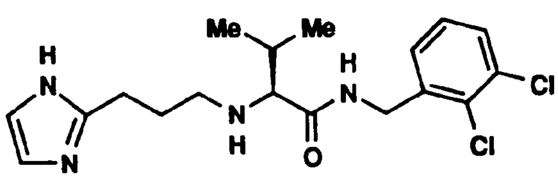

N-[(2,3-Dichlorophenyl)methyl]-$N^2$-[3-(1H-imidazol-2-yl)propyl]-L-valinamide, dihydrochloride

A. 3-[Imidazol-2-yl]-propenoic acid, ethyl ester

To a cooled (0°C) solution of sodium hydride (1.86 g, 45.8 mmol, 60% dispersion in mineral oil, prewashed with THF and dried over nitrogen) in 1,2-dimethoxyethane (DME, 20 mL) was added triethylphosphonoacetate (12 g, 54.1 mmol) dissolved in DME (10 mL) dropwise over 15 minutes. The solution was stirred for 1 hour at room temperature followed by the addition of 2-imidazole acetaldehyde (4 g, 41.6 mmol) in 20 mL of DME. The solution was stirred and heated to reflux (85°C) for 15 minutes followed by cooling to 60°C for 1 hour. On

cooling, the solution was concentrated to 1/2 volume and filtered. The solid was recrystallized from methanol/ethyl acetate/hexanes to give 5.1 g (74%) of Compound A as a white crystalline solid. MS $(M+H)^+$ $167^+$.

B. 3-[Imidazol-2-yl]-propanoic acid, ethyl ester

A solution of Compound A (4.01 g, 24.2 mmol) in absolute ethanol (100 mL, heated to dissolve) was hydrogenated using palladium on carbon (0.5 g) at room temperature for 16 hours. Following removal of hydrogen under vacuum, the catalyst was removed by filtration through a bed of Celite®. The filtrate was concentrated under vacuum to give 4.0 g (100%) of Compound B as a white crystalline solid. MS $(M+H)^+$ $169^+$.

C. 3-[N-Triphenylmethyl-imidazol-2-yl]propanoic acid, ethyl ester

Compound B was prepared from Compound A as described for Compound A of Example 6, using methylene chloride as solvent and triethylamine as base. After aqueous workup, recrystallization from ethyl acetate/hexanes afforded Compound B as a white microcrystalline solid. MS $(M+H)^+$ $411^+$.

D. 3-[N-Triphenylmethyl-imidazol-2-yl]-propan-1-ol

A solution of Compound C (0.80g, 1.95 mmol) in THF (15 mL) was cooled to 0°C under argon and a 1M solution of lithium aluminum hydride (2 mL, 2 mmol) was added dropwise with stirring. The reaction was stirred at ambient temperature for 16 hours. Water (2 mL) was added slowly and the solution was concentrated. Water (40 mL) and ethyl ether (60 mL) were added and the layers were separated. The ether layer was dried ($MgSO_4$) and concentrated. The residue was chromatographed (flash silica, 10:1 methylene chloride:methanol). Fractions containing product were pooled and concentrated to give 680 mg (95%) of Compound D as a white crystalline solid. MS $(M+H)^+$.

E. 3-[N-Triphenylmethyl-imidazol-2-yl]-propanal

A solution of oxalyl chloride (0.3 mL, 0.6 mmol) in 2 mL methylene chloride was cooled to -63°C under argon. DMSO (0.056 mL, 0.8 mmol) in methylene chloride (0.5 mL) was added over 10 minutes followed by addition of Compound D (147 mg, 0.4 mmol) in methylene chloride (6 mL) over 15 minutes keeping the reaction temperature below -50°C. The resulting clear solution was stirred for 50 minutes at -63°C. A solution of triethylamine (0.25 mL, 1.8 mmol) in methylene chloride (1 mL) was added over 15 minutes keeping the solution below -50°C. The mixture was stirred for 15 minutes followed by addition of 1M potassium hydrogen sulfate (4.5 mL), water (20 mL) and ethyl ether (60 mL). The layers were separated and the aqueous layer was made basic using half saturated aqueous sodium bicarbonate and washed with ethyl acetate (3 x 30 mL). The combined organic layers were dried ($MgSO_4$) and concentrated to yield 146 mg (>99%) of Compound E as a yellowish gum. $^1$H-NMR; ($CDCl_3$, 270 MHz) $\delta$ 9.53 (1H, s), 7.33-7.12 (15H , m), 6.94(1H, s), 6.76(1H, s), 2.37(2H, m), 2.22 (2H, m).

F. N-[(2,3-Dichlorophenyl)methyl]-$N^2$-[3-((1-triphenylmethyl)-imidazol-2-yl)propyl]-L-valinamide, dihydrochloride

Compound F was prepared from Compound E and L-valine, 2, 3-dichlorobenzylamide, hydrochloride (prepared from Boc-Val-OH and 2,3-dichlorobenzylamide as described for Compounds A and B of Example 4) as described for Compound C of Example 26 with the addition of 3Å molecular sieves during the reaction. Chromatography on silica gel with methylene chloride:methanol/9:1 afforded Compound F as a clear glass. MS $(M+H)^+$ $625^+$.

G. N-[(2,3-Dichlorophenyl)methyl]-$N^2$-[3-(1H-imidazol-2-yl)propyl]-L-valinamide, dihydrochloride

The title compound was prepared from Compound F as described for Compound D of Example 17.
MS $(M+H)^+$ $383^+$.
$[\alpha]_D$ -18.8° (c=0.13, methanol).

| Analysis for $C_{18}H_{24}N_4OCl_2$-2.21 $H_2O$-2 HCl: | | | |
|---|---|---|---|
| Calculated: | C, 43.58; | H, 6.18; | N, 11.29. |
| Found: | C, 43.59; | H, 5.88; | N, 11.52. |

Example 45

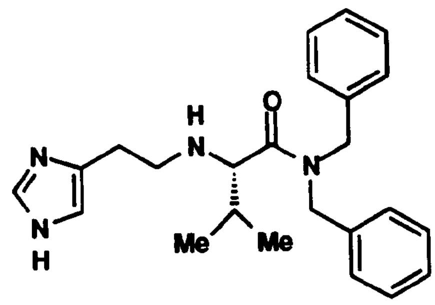

N2-[2-(1H-imidazol-5-yl)ethyl]-N,N-bis(phenylmethyl)-L-valinamide, dihydrochloride

A. N2-[(1,1-Dimethylethoxy)-carbonyl]-N2-[2-(1H-imidazol-5-yl)ethyl]-N,N-bis(phenylmethyl)-L-valinamide

A solution of 1.11g (2.0 mmol) of Compound A of Example 30 and 333 mg (2.45 mmol) of HOAT in 15 mL of DMF was treated with 411 mg (2.09 mmol) of dibenzylamine followed by 322 mg (2.56 mmol) of diisopropylcarbodiimide. After 30 hours, the mixture was diluted with 200 mL of ethyl acetate and washed with 2 x 100 mL of 5% sodium dihydrogen phosphate, 2 x 100 mL of 5% sodium bicarbonate and 2 x 100 mL of brine. Drying (sodium sulfate) and evaporation gave 2 g of a foam which was chromatographed on 300 mL of K-60 silica gel in heptane/ ethyl acetate, 1/1. Fractions pure by TLC were combined and evaporated to 1.1 g (75%) of Compound A.

B. N2-[2-(1H-imidazol-5-yl)ethyl]-N,N-bis(phenylmethyl)-L-valinamide, dihydrochloride

Compound A (1.1g, 1.5 mmol) was dissolved in 25 mL of cold methylene chloride and treated with 25 mL of TFA at 0°C. After warming to room temperature over 1 hour, the methylene chloride was removed *in vacuo* and the TFA solution allowed to stand at room temperature for 3 hours. The TFA was removed *in vacuo* and the residue treated with 10 mL of 1N HCl. The resulting slurry was evaporated to near dryness, 20 mL of water was added and the slurry filtered. The filtrate was extracted with ethyl acetate (2 x 50 mL) and the aqueous layer was evaporated to dryness *in vacuo*. Water (50 mL) was added and evaporated 4 times. Finally the residue was taken up in 50 mL of water and lyophilized to give a dense granular material. This was taken up in 150 mL of water and the lyophilization repeated to give 471 mg (80%) of the title compound as a less dense but still granular material.
MS $(M+H)^+$ $391^+$.

| Analysis for $C_{24}H_{30}N_4O$-0.4 $H_2O$-2 HCl: | | | | |
|---|---|---|---|---|
| Calculated: | C, 60.40; | H, 6.97; | N, 11.74; | Cl, 16.19. |
| Found: | C, 60.40; | H, 7.18; | N, 11.86; | Cl, 16.25. |

Example 46

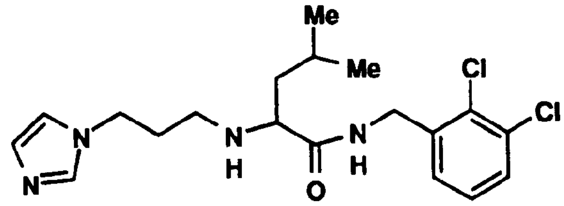

N-[(2,3-Dichlorophenyl)methyl]-2-[[3-(1H-imidazol-4-yl)propyl)amino]-4-methylpentanamide, dihydrochloride

The title compound was prepared as a foamy white solid in a 2 step sequence from 1-(3-aminopropyl)-imidazole, 4-methyl-2 oxopentanoic acid, sodium salt and 2,3-dichlorobenzylamine as described for Example 31, except that the reductive amination mixture was stirred 13 hours before hydrogenation and HOAT was used in place of HOBT in the coupling reaction.
MS: [M+H]+ = 397.
1H (CD3OD, 400 MHz) δ 9.04 (s, H), 7.71 (s, H) 7.62 (s, H), 7.52-7.50 (d, H, J = 18 MHz), 7.40-7.38 (d, H, J = 19 MHz), 7.32-7.28 (t, H), 4.63-4.50 (q, 2H), 4.41 (m, 2H), 3.90-3.89 (m, H), 3.12-2.95 (m, 2H), 2.34 (m, 2H), 1.80-1.64 (m, 3H), 0.99-0.93 (dd, 6H).

Example 47

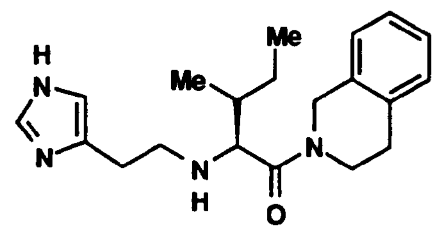

[S-(R*,R*)]-1,2,3,4-Tetrahydro-2-[2-[[2-(1H-imidazol-4-yl)ethyl]amino]-3-methyl-1-oxopentyl]isoquinoline, dihydrochloride

The title compound was prepared from the title compound of Example 5 as described for Compounds B and C of Example 16. After chromatography, the resulting viscous oil was evaporated from a solution of methanol containing hydrochloric acid to prepare the hydrochloride salt. The gummy residue was crystallized from ethyl acetate to yield a gummy solid which was triturated with ether. Filtration afforded the title compound as a pale yellow powder, mp 145° (d).
MS $(M+H)^+$ $341^+$.
$[\alpha]_D$ -8.2° (c 0.82, methanol).

| Analysis for $C_{20}H_{28}N_4O$-1.72 $H_2O$-2 HCl: | | | |
|---|---|---|---|
| Calculated: | C, 54.06; | H, 7.58; | N, 12.61. |
| Found: | C, 54.45; | H, 7.62; | N, 12.15. |

Example 48

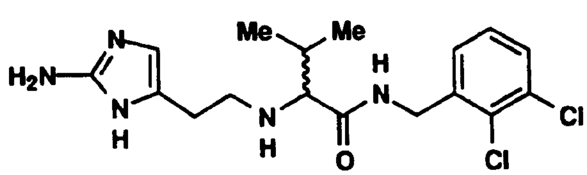

2-[[2-(2-Amino-1H-imidazol-4-yl)ethyl]amino]-N-[(2,3-dichlorophenyl)methyl]-3-methylbutanamide, dihydrochloride

A. [(2-Phenylazo-1H-imidazol-4-yl)ethyl]-D,L-valine, ethyl ester

A solution of the diazonium chloride of p-bromoaniline was prepared by the addition of a cold solution of 290 mg (4.18 mmol) of sodium nitrite ($NaNO_2$) in 1 mL of water to an ice cooled solution of 720 mg (4.18 mmol) of p-bromoaniline in 7 mL of 20% HCl. This solution was stirred for 0.5 hour with cooling and added dropwise to an ice cooled solution of 1.0 g (4.18 mmol) of Compound A of Example 23 in 168 mL of 0.25 M sodium carbonate. An immediate red-orange precipitate was obtained on addition. The reaction was stirred for an additional 2 hours with ice cooling. The precipitate was filtered and the filter cake washed well with water. The dark red filter cake was dissolved in methylene chloride, dried ($MgSO_4$), and the solution subjected to flash chromatography on a 50 cc column of silica gel. Elution with 50% ethyl acetate-hexanes afforded 1.0 g (57%) of Compound A as a bright yellow solid.

B. [(2-Amino-1H-imidazol-4-yl)ethyl]-D,L-valine, ethyl ester

A solution of 1.0 g (2.4 mmol) of Compound A in 50 mL of ethanol containing 100 mg of platinum oxide ($PtO_2$) catalyst was hydrogenated at 50 psi, at room temperature, in a Parr apparatus overnight. After removal of the catalyst by filtration through Celite®, the clear colorless filtrate was evaporated to dryness to yield a viscous oily residue. This material was dissolved in water and the solution washed twice with ether to remove the aniline liberated during the hydrogenation. The remaining aqueous solution was evaporated to dryness and the oily residue diluted with chloroform. A small amount of saturated $NaHCO_3$ was added and the mixture was stirred while additional solid $NaHCO_3$ was added in portions. Stirring was continued for 0.5 hour. Solid $MgSO_4$ was added to remove any remaining water. The solids were removed by filtration and the filter cake washed well with additional chloroform. The filtrate was evaporated to dryness and the brownish oily residue subjected to flash chromatography on a 45 cc column of silica gel. Elution with 25% methanol in chloroform afforded 590 mg (98%) of Compound B as a pale yellow foam.

C. [(2-Amino-1H-imidazol-4-yl)ethyl]-D,L-valine

Compound C was prepared as a tan foam from Compound B as described for Compound E of Example 16.

D. 2-[[2-(2-Amino-1H-imidazol-4-yl)ethyl]-amino]-N-[(2,3-dichlorophenyl)methyl]-3-methylbutanamide, dihydrochloride

Compound D was prepared from Compound C as described for Compound H of Example 21. Following evaporation of the reaction solvent, the residue was subjected to prep HPLC on a YMC S-10 ODS, 50 x 300 mm column. Gradient elution from 0 to 100% solvent B (A: 10% methanol:water + 0.1% TFA, B: 10% water:methanol + 0.1% TFA) afforded a tan solid foam. This material was resubjected to prep HPLC on a YMC S-5 ODS, 30 x 250 mm column, using the gradient elution described, to give a clear colorless glass which upon trituration with ether afforded the title compound as a white powder, mp 215° (d).
MS $(M+H)^+$ $384^+$.

| Analysis for $C_{17}H_{23}N_5OCl_2$-0.88 $H_2O$-2 HCl: | | | |
|---|---|---|---|
| Calculated: | C, 43.17; | H, 5.70; | N, 14.81. |
| Found: | C, 43.28; | H, 5.65; | N, 14.53. |

Example 49

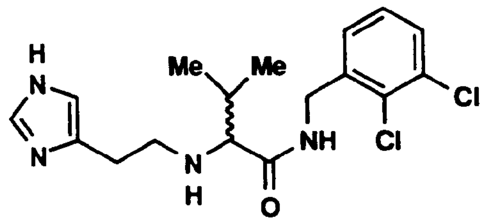

N-[(2,3-Dichlorophenyl)methyl]-N2-[2-(1H-imidazol-4-yl)ethyl]-D,L-valinamide, trifluoroacetate

To a homogeneous suspension of Compound A of Example 35 (85 mg, 0.4 mmol) in 60:40 DMF:DMSO (3.2 mL) were added sequentially a solution of 2,3-dichlorobenzylamine (70 mg, 0.4 mmol) in DMF (0.4 mL), NMM (44 µl, 0.4 mmol) and a solution of BOP (177 mg, 0.4 mmol) in methylene chloride (1.6 mL). The resulting mixture was allowed to stand at 25°C for 18 hours, after which the product was isolated by solid phase extraction as follows:

1) A solid phase extraction cartridge containing 1.0 g of SCX resin (Varian) was conditioned with 10 mL of methanol.

2) The reaction mixture was loaded onto the column.

3) The column was rinsed with 30 mL of methanol.

4) The column was rinsed with 7 mL of 0.1 N ammonia in methanol.

5) The column was eluted with 7.5 mL of 1.0 N ammonia in methanol, and the eluant was collected into a tared tube. The solution containing the product was concentrated *in vacuo* to give an oily residue (154 mg). The residue was purified by preparative HPLC on a YMC S10 ODS column (30 x 500 mm), eluting with a linear gradient over 30 minutes from 10% to 90% aqueous methanol containing 0.1% TFA at 50 mL/minute. Fractions containing the main UV absorbing peak (220 nm) were combined and concentrated, and the residue was lyophilized to give the title compound as a white solid (110 mg, 46%).

Analysis for $C_{17}H_{22}N_4OCl_2$-1.25 $H_2O$-2 $CF_3CO_2H$:
Calculated: C, 40.69; H, 4.31; N, 9.04; F, 18.39; Cl, 11.44.
Found: C, 41.09; H, 4.18; N, 8.62; F, 18.50; Cl, 11.43.

Example 50

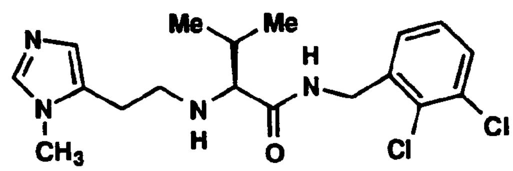

N-[(2,3-Dichlorophenyl)methyl]-N2-[2-(1-methyl-1H-imidazol-5-yl)ethyl]-L-valinamide, dihydrochloride

The title compound was prepared from Compound B of Example 33 and methyltriflate using the 2 step procedure reported for Example 34.

| Analysis for $C_{18}H_{24}N_4O_2Cl_2$-0.7 $H_2O$-2.11 HCl: | | | | |
|---|---|---|---|---|
| Calculated: | C, 45.73; | H, 5.86; | N, 11.85; | Cl, 30.82. |
| Found: | C, 45.93; | H, 5.50; | N, 11.65; | Cl, 30.81. |

Example 51

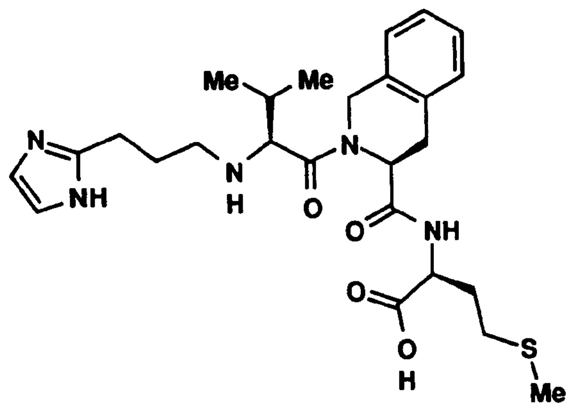

(S)-N-[[1,2,3,4-Tetrahydro-2-[N-[3-(1H-imidazol-2-yl)propyl]-L-valyl]-3-isoquinolinyl]carbonyl] -L-methionine, trifluoroacetate (1:2)

A. (S)-N-[[1,2,3,4-Tetrahydro-2-[N-[2-(1-triphenylmethyl-imidazol-4-yl)propyl]-L-valyl]-3-isoquinolinyl]-carbonyl]-L-glutamine, methyl ester

Compound A was prepared as a slightly yellow oil in 67% yield from Compound B of Example 14 and Compound D of Example 1 as described for Compound G of Example 6. Following chromatography on silica with ethyl acetate:hexanes (1:1) followed by methylene chloride:methanol (9:1), it was used without additional purification.
MS: (M+H) = 772.

B (S)-N-[[1,2,3,4-Tetrahydro-2-[N-[2-(1-triphenylmethyl-imidazol-4-yl)propyl]-L-valyl]-3-isoquinolinyl]-carbonyl]-L-glutamine

To a solution of Compound A (130 mg, 0.169 mmol) in THF (3 mL) and methanol (1 mL) was added lithium hydroxide (LiOH) (1 M, 0.2 mL, 0.2 mmol). The solution was stirred for 2 hours followed by the addition of 1N aqueous HCl (0.2 mL, 0.2 mmol). The solution was concentrated to dryness and washed with a 1:1 mixture of methylene chloride:methanol (3 x 20 mL), filtered and concentrated to afford 125 mg (98%) of Compound B as an opaque glass.
MS $(M+H)^+$ $758^+$.

C. (S)-N-[[1,2,3,4-Tetrahydro-2-[N-[3-(1H-imidazol-2-yl)propyl]-L-valyl]-3-isoquinolinyl]carbonyl]-L-methionine, trifluoroacetate (1:2)

The title compound was prepared in 54% yield as a white powder from Compound B as described for Compound F of Example 2.
MS $(M-H)^-$ $514^-$.
Analysis for $C_{26}H_{37}N_6O_4S$-0.35 $H_2O$-2.3 $CF_3CO_2H$:
Calculated: C, 46.87; H, 5.14; N, 8.93; S, 4.09; F, 16.72.
Found: C, 46.92; H, 5.05; N, 8.83; S, 4.11; F, 16.65.
See the table below for the following Examples 52 -148.

**Examples 52 - 72:**

A. N-[(1,1-Dimethylethoxy)-carbonyl]-N-[1-triphenylmethyl-imidazol-4-yl-ethyl]-L-valine, sodium salt

Sodium hydroxide (4.7 ml of 1.0 N aqueous solution, 4.7 mmol) was added to a solution of Compound A of Example 30 (2.61 g, 4.7 mmol) in methanol (10 ml). The resulting mixture was stirred for 5 minutes, then concentrated *in vacuo*. The residue was dried *in vacuo* to give Compound A as a white powder which was used without purification.

B. Examples 52 - 72

The coupling of Compound A with various amines was carried out using a standard diisopropylcarbodiimide (DIC) mediated coupling. The process was automated by using a Zymark Benchmate™ robotic workstation to carry out the acid-amine coupling and the purification of the resulting amide products. An IBM PC was used to run the Zymark Benchmate™ workstation operating program and to write the Benchmate™ procedures. The protocol for the preparation of the compounds of Examples 52 - 72 follows:
A 16 x 100 mm test tube was charged with 0.075 mmol of the amine component and capped with a plastic cap/column holder. For amines which were used as free bases, the Benchmate™ then carried out the following steps sequentially on each tube:

1. Add 0.3 mL of methylene chloride.
2. Add 0.3 mL of a 0.25 M solution of Compound A in DMF (0.075 mmol).
3. Add 0.3 mL of a 0.3 M solution of HOAt in DMF (0.09 mmol).
4. Vortex for 30 seconds at speed 3.
5. Add 0.3 mL of a 0.3 M solution of DIC in methylene chloride (0.09 mmol).
6. Vortex for 30 seconds at speed 3.
7. Wash syringe with 5 mL of methylene chloride.

For amines which were used as hydrochloride salts, the Benchmate™ then carried out the following steps sequentially on each tube:

1. Add 0.3 mL of dichloromethane.
2. Add 0.3 mL (7.6 mg, 0.075 mmol) of a 0.25 M solution of triethylamine in dichloromethane.
3. Mix tube contents by vortexing at speed 3 for 30 seconds.
4. Add 0.3 mL (45.8 mg, 0.075 mmol) of a 0.25 M solution of compound A in DMF.
5. Add 0.30 mL (12.2 mg, 0.090 mmol) of a 0.30 M solution of HOAT in DMF.
6. Mix tube contents by vortexing at speed 3 for 30 seconds.
7. Add 0.30 mL (11.3 mg, 0.090 mmol) of a 0.30 M solution of DIC in dichloromethane.
8. Mix tube contents by vortexing at speed 3 for 30 seconds.
9. Wash syringe with 5 mL of dichloromethane.

For both coupling protocols, the tubes were allowed to stand for 24 hours, after which 2.0 mL of TFA and 0.2 mL of triethylsilane were manually added to each tube. After 4 hours, the tubes were concentrated *in vacuo* in a Savant Speed-Vac™. The tubes were returned to the Benchmate™ and the contents were purified by ion exchange chromatography on a solid phase extraction cartridge mediated by the Benchmate workstation using the following protocol:

1. Add 5 mL of 1:1 methanol : methylene chloride to the test tube.
2. Vortex for 60 seconds at speed 3.
3. Condition a Varian solid phase extraction cartridge containing 1.0 g SCX resin with 10 mL of 1:1 methanol : methylene chloride at 0.15 mL/second.
4. Load reaction contents onto column at 0.02 mL/second.
5. Rinse column with 15 mL of 1:1 methanol : methylene chloride at 0.10 mL/second.
6. Rinse column with 7.5 mL of methanol at 0.10 mL/second.
7. Rinse column with 5 mL of 0.01 N ammonia in methanol at 0.10 mL/second.
8. Elute column with 7.5 mL of 1.0 N ammonia in methanol at 0.05 mL/second into a tared receiving tube.
9. Wash syringe with 5 mL of 1:1 methanol : methylene chloride.

The contents of the receiving tubes were then concentrated *in vacuo* in a Savant Speed-Vac™. Homogeneity of the products was determined by HPLC, and identity was confirmed by electrospray mass spectroscopy.

**Examples 73 - 83**

The compounds of Examples 73 - 83 were prepared from Compound A from Examples 52 - 72 as described for the compounds of Examples 52 - 72. All compounds were further purified by preparative HPLC under the following conditions: A 30 x 250 mm YMC S5 ODS column was eluted with 25 mL/minute of a gradient from 10% to 90% aqueous methanol containing 0.1% TFA. Fractions were monitored by ultraviolet absorbance at 220 nm. Fractions containing the desired product (usually the major peak) were combined and concentrated *in vacuo* to give the compounds of Examples 73 - 83 as glassy oils. Homogeneity was determined by analytical HPLC, and identity was confirmed by electrospray mass spectroscopy.

**Examples 84 - 100**

The protocol for preparation of the compounds of Examples 84 - 100 from Compound E of Example 20 follows:

A 16 x 100 mm tube was charged with 0.075 mmol of the amine component and capped loosely with a plastic cap/column holder. The Benchmate® then carried out the following steps on the tube:

1) Add 0.2 mL of dichloromethane.
2) Add 0.3 mL (0.075 mmol) of a 0.25 M solution of Compound E of Example 20 in DMF.
3) Mix tube contents by vortexing at speed 3 for 30 seconds.
4) Add 0.33 mL (0.082 mmol) of a 0.25 M solution of BOP in DMF.
5) Mix tube contents by vortexing at speed 3 for 30 seconds.
6) Wash syringe with 5 mL of dichloromethane.

After 20 hours, the reaction mixture contents were purified by ion exchange chromatography on a solid phase extraction cartridge mediated by the Benchmate® robotic workstation using the following protocol:

1) Condition a Varian solid phase extraction column (500 mg, SCX cation exchange) with 10 mL of methanol at 0.25 mL/second.
2) Load reaction contents onto column at 0.05 mL/second.
3) Wash column with 2 x 10 mL of methanol at 0.1 mL/second.
4) Wash column with 2 mL of 0.1 M ammonia in methanol at 0.1 mL/second.
5) Elute column with 5 mL of 1 M ammonia in methanol and collect into a tared receiving tube at 0.1 mL/second.

All solution/solvent deliveries were followed by 1.8 mL of air and a 10 second push delay was used after loading reaction contents onto the ion exchange column.

The product solution was concentrated on a Savant Speed Vac (approx. 2 mm Hg for 20 hours) to afford products.

**Examples 101 - 108**

The compounds of Examples 101 - 108 were prepared as described for the compounds of Examples 84 - 100 except that the following steps were introduced between steps 5 and 6 of the coupling protocol:

5b. Add 0.3 mL (7.5 mg, 0.075 mmol) of 0.25 M triethylamine; and
5c. Mix tube contents by vortexing at speed 3 for 30 seconds;

and the following steps were introduced before step 1 of the purification protocol:

1a. Add 0.2 mL of methanol to the reaction; and
1b. Mix tube contents by vortexing at speed 3 for 30 seconds.

**Examples 109 - 131**

A. <u>N-[(1,1-Dimethylethoxy)-carbonyl]-N-[imidazol-4-yl-ethyl]-L-valine, tetrabutylammonium salt</u>

To a solution of Compound E of Example 16 (778 mg, 2.5 mmol) in methanol (3.0 mL) was added 1.0 M tetrabutyl ammonium hydroxide (2.5 mL, 2.5 mmol, in methanol) and the mixture was concentrated in vacuo. The residue was dissolved in dichloromethane (10 mL) and filtered through a 0.5 μm frit. The solution was concentrated and dissolved in 10 mL of dichloromethane to give a 0.25 M solution of Compound A.

B. Example 109 - 131

A 16 x 100 mm tube was charged with 8.0 mg (0.075 mmol) of the amine component and capped loosely with a plastic cap/column holder. For amines which were used as free bases, the Benchmate™ then carried out the following steps sequentially on each tube:

1. Add 0.3 mL of dichloromethane.
2. Mix tube contents by vortexing at speed 3 for 30 seconds.
3. Add 0.3 mL (59.5 mg, 0.075 mmol) of a 0.25 M solution of Compound A in dichloromethane.
4. Mix tube contents by vortexing at speed 3 for 30 seconds.
5. Add 0.36 mL (39.8 mg, 0.090 mmol) of a 0.25 M solution of BOP in dichloromethane.
6. Mix tube contents by vortexing at speed 3 for 30 seconds.
7. Wash syringe with 5 ml of dichloromethane.

For amines which were used as hydrochloride salts, the Benchmate™ then carried out the following steps

sequentially on each tube:

1. Add 0.3 mL of DMF.
2. Mix tube contents by vortexing at speed 3 for 30 seconds.
3. Add 0.3 mL (7.6 mg, 0.075 mmol) of a 0.25 M solution of triethylamine.
4. Mix tube contents by vortexing at speed 3 for 30 seconds.
5. Add 0.3 mL (59.5 mg, 0.075 mmol) of a 0.25 M solution of the Compound A, in dichloromethane.
6. Mix tube contents by vortexing at speed 3 for 30 seconds.
7. Add 0.33 mL (36.4 mg, 0.082 mmol) of a 0.25 M solution of BOP in DMF.
8. Mix tube contents by vortexing at speed 3 for 30 seconds.
9. Wash syringe with 5 ml of DMF.

For both coupling protocols, after 20 hours, the reaction vessel was transferred to a fume hood and TFA (1 mL) was added. After 2 hours the reaction mixture was concentrated on a Savant Speed Vac (approx. 2 mm mercury for 20 hours). The residue was then purified by ion exchange chromatography on a solid phase extraction cartridge mediated by the Benchmate® robotic workstation using the following protocol:

1. Add 1.0 mL of methanol to the reaction.
2. Add 0.2 mL of dichloromethane to the reaction.
3. Mix tube contents by vortexing at speed 3 for 60 seconds.
4. Condition a Varian solid phase extraction column (1.0 g, SCX cation exchange) with 10 mL of methanol at 0.25 mL/second.
5. Load reaction contents onto column at 0.05 mL/second.
6. Wash column with 3 x 10 mL of methanol at 0.1 mL/second.
7. Elute column with 5 mL of 1 M ammonia in methanol and collect into a tared receiving tube at 0.1 mL/second.

All solution/solvent deliveries were followed by 1.8 mL of air and a 10 second push delay was used after loading reaction contents onto the ion exchange column.

The product solution was concentrated on a Savant Speed Vac (approx. 2 mm mercury for 20 hours). The compounds of Examples 109 - 112 and 127 - 131 were further purified by Preparative HPLC on a YMC ODS-A column (S-5mm, 250 x 30 mm, $\lambda$ = 220, 10-90% aqeous methanol, 0.1% TFA, 30 minutes gradient elution) and were produced as TFA salts.

**Examples 132 - 140**

The coupling of Compound A of Example 30 with various amines was carried out using a PyBrop mediated coupling. The process was automated by using a Shimdazu SIL10A autosampling workstation connected to a Shimdazu LC-8 preparative HPLC to carry out the acid-amine coupling and the purification of the resulting amide products. The workstation was programmed using a Shimdazu SCL-10A system controller to create a pretreatment file for reagent mixing. The protocol for preparation of amides from Compound A of Example 30 and the corresponding amines was as follows:

A 4 mL glass autosampler vial was charged with 0.2 mmol of the amine component and capped with a teflon coated rubber septum. The Shimadzu SIL10A then carried out the following steps sequentially on each vial:

1. Add 0.20 mL of a 0.5 M solution of Compound A of Example 30 (0.10 mmol) in methylene chloride.
2. Add 0.40 mL of a solution containing 0.5 M DMAP (0.2 mmol) and 1.0 M triethylamine (0.4 mmol) in methylene chloride.
3. Add 0.25 mL of a 1.0 M solution of PyBroP in methylene chloride (0.25 mmol).
4. Mix three times by needle aspiration.
5. Wash syringe with 2 mL of acetonitrile.

After all reagents were added, the rack containing the reaction vials was transferred to an orbital shaker and the vials were shaken at 200 rpm for 18 hours. TFA (1.0 mL) and 0.075 mL of triethylsilane were then manually added to each tube. After 4 hours, the vials were uncapped and their contents were concentrated *in vacuo* in a Savant Speed-Vac™. The residue in the vials was then dissolved in 1.0 mL of THF and 1.0 mL of methanol, and the solutions were injected sequentially by autosampler into the Shimadzu preparative HPLC. Chromatography was carried out on a YMC S5 ODS column (30 x 250 mm), eluting with 25 mL/minute of a linear gradient from 10% to 90% aqueous methanol containing 0.1% TFA over 30 minutes. Fractions were monitored by ultraviolet absorbance at 220 nm. Those containing the desired products (as determined by mass spectroscopy) were concentrated in the Speed-Vac to give the compounds of Examples 132 - 140.

**Examples 141 - 148**

A. <u>(3S)-1,2,3,4-Tetrahydro-2-[N²-[(1,1-dimethylethoxy)-carbonyl]-N²-[2-(1-triphenylmethyl-imidazol-4-yl)ethyl]-L-valyl]-3-isoquinolinecarboxylic acid, methyl ester</u>

HOAT (1.31 g, 9.63 mmol) was added to a slurry of Compound A of Example 30 (3.55 g, 6.42 mmol), methyl L-1,2,3,4-tetrahydroisoquinoline-3-carboxylate (4.38 g, 19.2 mmol), EDC (1.84 g, 9.63 mmol) and DIEA (5.59 mL, 32.1 mmol) in DMF (26 mL) under argon. After 16 hours, the reaction was diluted with $NaHCO_3$ (20 mL), 10% LiCl (10 mL) and ethyl acetate (75 mL). The aqueous layer was extracted with ethyl acetate (2 x 75 mL) and the combined organic extracts were washed with $NaHCO_3$ (30 mL) and LiCl (2 x 30 mL), dried over $MgSO_4$, filtered and concentrated. The residue was purified on a flash silica gel column eluting with a step gradient of 9/1, 8/2, 7/3, 6/4, hexane /ethyl acetate. The isolated product was dissolved in ethyl acetate, the solution was washed with 10% HCl, and the organic layers were concentrated to afford Compound A as a light yellow oil (2.75 g, 59%).

B. <u>(3S)-1,2,3,4-Tetrahydro-2-[N²-[ (1,1-dimethylethoxy)-carbonyl]-N²-[2-(1-triphenylmethyl-imidazol-4-yl)ethyl]-L-valyl]-3-isoquinolinecarboxylic acid</u>

1 N NaOH (9.1 mL, 9.1 mmol) was added to a solution of Compound A (2.75 g, 3.78 mmol) in methanol /THF (18 / 13 mL). After stirring for 15 hours, the reaction mixture was diluted with ethyl acetate (30 mL) and neutralized with 1N HCl (5 mL). The aqueous layer was extracted with ethyl acetate (2 x 100 mL). The combined organic layers were dried over $MgSO_4$, filtered and concentrated to afford Compound B a light yellow oil (2.56 g, 100 %).

A 16 x 100 mm tube was charged with 0.075 mmol of the amine and capped loosely with a plastic cap/column holder. The Benchmate® then carried out the following steps on the tube:

1. Add 0.3 mL of dichloromethane.
2. Add 0.3 mL (53.4 mg, 0.075 mmol) of a 0.25 M solution of Compound B in dichloromethane
3. Add 0.30 mL (12.2 mg, 0.090 mmol) of a 0.30 M solution of HOAT in DMF.
4. Mix tube contents by vortexing at speed 3 for 30 seconds.
5. Add 0.30 mL (11.3 mg, 0.090 mmol) of a 0.30 M solution of DIC in dichloromethane.
6. Mix tube contents by vortexing at speed 3 for 30 seconds.
7. wash syringe with 5 mL of dichloromethane.

After 20 hours, the reaction vessel was transferred to a fumehood and TFA (1.5 mL) was added. The mixture was allowed to stand for 20 minutes, and triethylsilane (150 mL) was added. After 24 hours, the mixture was concentrated on a Savant Speed Vac (approx. 2 mm mercury for 72 hours). The residue was purified by ion exchange chromatography on a solid phase extraction cartridge mediated by the Benchmate® robotic workstation using the following protocol:

1. Add 5.0 mL of methanol/dichloromethane (1:1).
2. Mix tube contents by vortexing at speed 3 for 60 seconds.
3. Condition a Varian solid phase extraction column (1.5 g, SCX cation exchange) with 10 mL of methanol/dichloromethane at 0.15 mL/second.
4. Load reaction contents onto column at 0.02 mL/second.
5. Wash column with 3 x 7.5 mL of methanol/dichloromethane (1:1) at 0.1 mL/second.
6. Wash column with 0.01 M ammonia in methanol.
7. Elute column with 7.5 ml of 1 M ammonia in methanol and collect into a tared receiving tube at 0.05 mL/second.

All solution/solvent deliveries were followed by 1.0 mL of air, and a 5 second push delay was used after loading reaction contents onto the ion exchange column. The product solution was concentrated on a Savant Speed Vac (approximately 2 mm mercury for 20 hours) to afford the compounds of Examples 141 - 148. For the compound of Example 148, triethylamine (0.3 mL of a 0.25 M solution, 0.075 mmol) was added to the reaction mixture since methionine benzyl sulfonamide, hydrochloride salt was used as the starting amine.

| | | | |
|---|---|---|---|
| 52 | 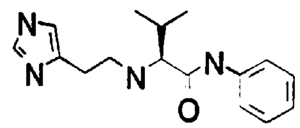 | N<2-[2-(1H-Imidazol-4-yl)ethyl]-N-phenyl-L-valinamide. | m/z 287 (M+H)+ |
| 53 | 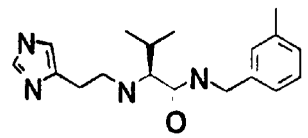 | N<2-[2-(1H-Imidazol-4-yl)ethyl]-N-[(3-methylphenyl)-methyl]-L-valinamide. | m/z 315 (M+H)+ |
| 54 | 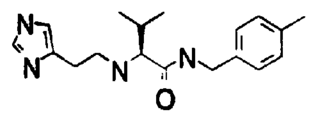 | N<2-[2-(1H-Imidazol-4-yl)ethyl]-N-[(4-methylphenyl)-methyl]-L-valinamide. | m/z 315 (M+H)+ |
| 55 | 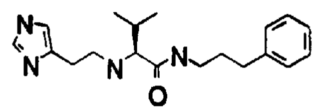 | N<2-[2-(1H-Imidazol-4-yl)ethyl]-N-(3-phenylpropyl)-L-valinamide. | m/z 329 (M+H)+ |
| 56 | 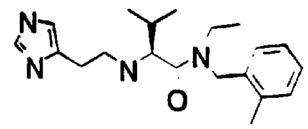 | N-Ethyl-N<2-[2-(1H-imidazol-4-yl)ethyl]-N-[(2-methyl-phenyl)methyl]-L-valinamide. | m/z 343 (M+H)+ |
| 57 | 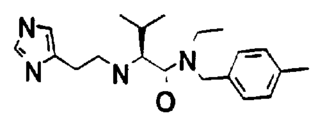 | N-Ethyl-N<2-[2-(1H-imidazol-4-yl)ethyl]-N-[(4-methyl-phenyl)methyl]-L-valinamide. | m/z 343 (M+H)+ |
| 58 | 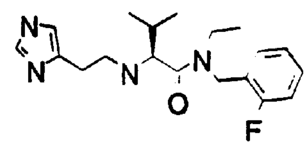 | N-Ethyl-N-[(2-fluorophenyl)methyl]-N<2-[2-(1H-imidazol-4-yl)ethyl]-L-valinamide. | m/z 347 (M+H)+ |
| 59 | 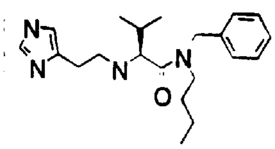 | N-Butyl-N<2-[2-(1H-imidazol-4-yl)ethyl]-N-(phenyl-methyl)-L-valinamide. | m/z 357 (M+H)+ |

| | | | |
|---|---|---|---|
| 60 | 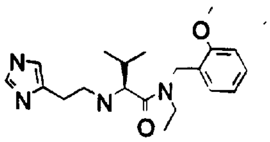 | N-Ethyl-N<2-[2-(1H-imidazol-4-yl)ethyl]-N-[(2-methoxy-phenyl)methyl]-L-valinamide. | m/z 359 (M+H)+ |
| 61 | 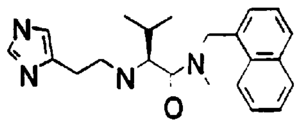 | N<2-[2-(1H-Imidazol-4-yl)ethyl]-N-methyl-N-(1-naphth-alenylmethyl)-L-valinamide. | m/z 365 (M+H)+ |
| 62 | 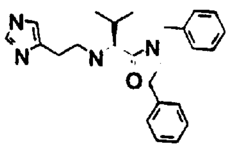 Chiral | N<2-[2-(1H-Imidazol-4-yl)ethyl]-N-(2-phenylethyl)-N-(phenylmethyl)-L-valinamide. | m/z 405 (M+H)+ |
| 63 | 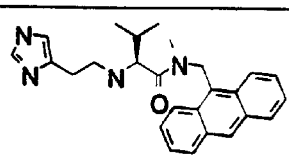 | N-(9-Anthracenylmethyl)-N<2-[2-(1H-imidazol-4-yl)-ethyl]-N-methyl-L-valinamide. | m/z 415 (M+H)+ |
| 64 | 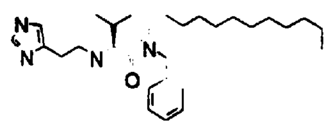 | N-Dodecyl-N<2-[2-(1H-imidazol-4-yl)ethyl]-N-(phenyl-methyl)-L-valinamide. | m/z 469 (M+H)+ |
| 65 | 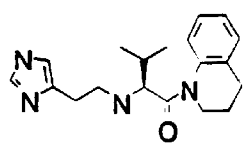 | (S)-1,2,3,4-Tetrahydro-1-[2-[[2-(1H-imidazol-4-yl)-ethyl]amino]-3-methyl-1-oxobutyl]quinoline. | m/z 327 (M+H)+ |
| 66 | 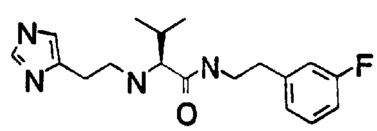 | N-[2-(3-Fluorophenyl)ethyl]-N<2-[2-(1H-imidazol-4-yl)ethyl]-L-valinamide. | m/z 333 (M+H)+ |
| 67 | 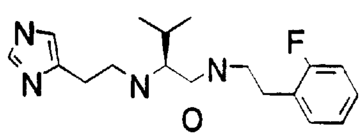 | N-[2-(2-Fluorophenyl)ethyl]-N<2-[2-(1H-imidazol-4-yl)ethyl]-L-valinamide. | m/z 333 (M+H)+ |

| | | | |
|---|---|---|---|
| 68 | 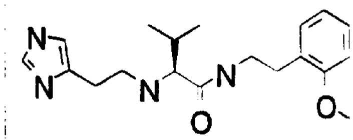 | N<2-[2-(1H-Imidazol-4-yl)ethyl]-N-[2-(2-methoxy-phenyl)ethyl]-L-valinamide. | m/z 345 (M+H)+ |
| 69 | 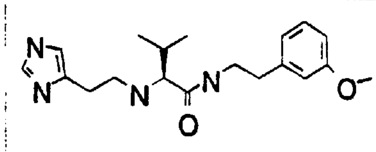 | N<2-[2-(1H-Imidazol-4-yl)ethyl]-N-[2-(3-methoxy-phenyl)ethyl]-L-valinamide. | m/z 345 (M+H)+ |
| 70 | 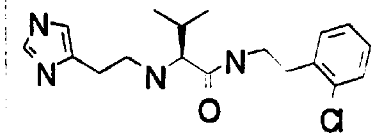 | N-[2-(2-Chlorophenyl)ethyl]-N<2-[2-(1H-imidazol-4-yl)ethyl]-L-valinamide. | m/z 349 (M+H)+ |
| 71 | 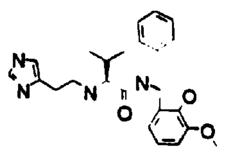 | N-[(2-Hydroxy-3-methoxyphenyl)methyl]-N<2-[2-(1H-imidazol-4-yl)ethyl]-N-(phenylmethyl)-L-valinamide. | m/z 437 (M+H)+ |
| 72 | 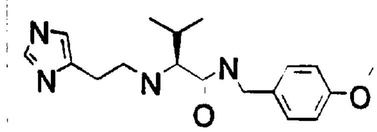 | N<2-[2-(1H-Imidazol-4-yl)ethyl]-N-[(4-methoxyphenyl)-methyl]-L-valinamide. | m/z 331 (m+H) |
| 73 | 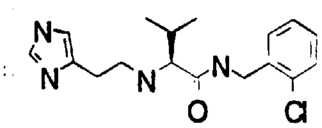 | N-[(2-Chlorophenyl)methyl]-N<2-[2-(1H-imidazol-4-yl)-ethyl]-L-valinamide, trifluoroacetate. | m/z 335 (M+H)+ |
| 74 | 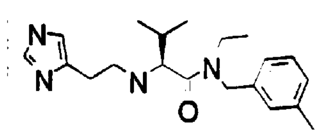 | N-Ethyl-N<2-[2-(1H-imidazol-4-yl)ethyl]-N-[(3-methyl-phenyl)methyl]-L-valinamide, trifluoroacetate. | m/z 343 (M+H)+ |
| 75 | 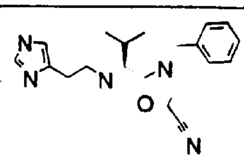 Chiral | N-(2-Cyanoethyl)-N<2-[2-(1H-imidazol-4-yl)ethyl]-N-(phenylmethyl)-L-valinamide, trifluoroacetate. | m/z 354 (M+H)+ |

| | | | |
|---|---|---|---|
| 76 | 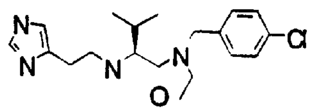 | N-[(4-Chlorophenyl)methyl]-N-ethyl-N<2-[2-(1H-imidazol-4-yl)ethyl]-L-valinamide, trifluoroacetate. | m/z 363 (M+H)+ |
| 77 | 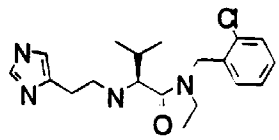 | N-[(2-Chlorophenyl)methyl]-N-ethyl-N<2-[2-(1H-imidazol-4-yl)ethyl]-L-valinamide, trifluoroacetate. | m/z 363 (M+H)+ |
| 78 | 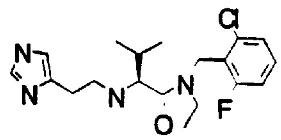 | N-[(2-Chloro-6-fluorophenyl)methyl]-N-ethyl-N<2-[2-(1H-imidazol-4-yl)ethyl]-L-valinamide, trifluoroacetate. | m/z 381 (M+H)+ |
| 79 | 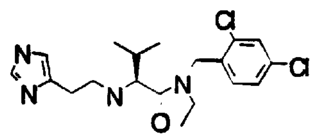 | N-[(2,4-Dichlorophenyl)methyl]-N-ethyl N<2-[2-(1H-imidazol-4-yl)ethyl]-L-valinamide, trifluoroacetate. | m/z 397 (M+H)+ |
| 80 | 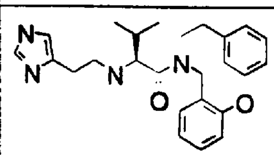 | N-[(2-Hydroxyphenyl)methyl]-N<2-[2-(1H-imidazol-4-yl)ethyl]-N-(2-phenylethyl)-L-valinamide,trifluoroacetate. | m/z 421 (M+H)+ |
| 81 | 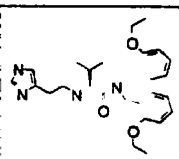 | N,N-Bis[(2-ethoxyphenyl)methyl]-N<2-[2-(1H-imidazol-4-yl)ethyl]-L-valinamide, trifluoroacetate. | m/z 479 (M+H)+ |
| 82 | 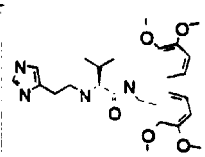 | N,N-Bis[(2,3-dimethoxyphenyl)methyl]-N<2-[2-(1H-imidazol-4-yl)ethyl]-L-valinamide, trifluoroacetate. | m/z 511 (M+H)+ |
| 83 | 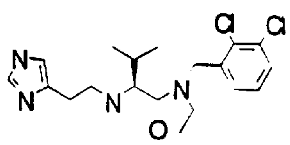 | N-[(2,3-Dichlorophenyl)methyl]-N-ethyl N<2-[2-(1H-imidazol-4-yl)ethyl]-L-valinamide, trifluoroacetate. | m/z 398 (M+H)+ |

| | | | |
|---|---|---|---|
| 84 | 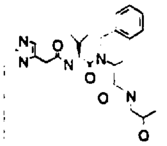 | (3S)-N-(2-Hydroxypropyl)-N<2-[2-(1H-imidazol-4-yl)-ethyl]-L-valinamide. | m/z 442 (M+H)+ |
| 85 | 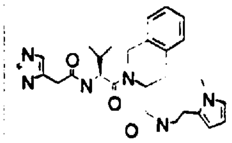 | (S)-1,2,3,4-Tetrahydro-2-[N-(1H-imidazol-4-ylacetyl)-L-valyl]-N-[2-(1-methyl-1H-pyrrol-2-yl)ethyl]-3-isoquinolinecarboxamide. | m/z 491 (M+H)+ |
| 86 | 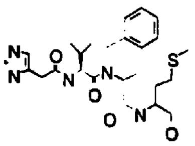 | (3S)-1,2,3,4-Tetrahydro-N-[1-(hydroxymethyl)-3-(methylthio)propyl]-2-[N-(1H-imidazol-4-ylacetyl)-L-valyl]-3-isoquinolinecarboxamide. | m/z 502 (M+H)+ |
| 87 | 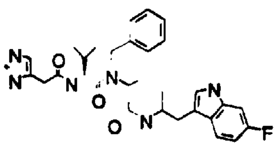 | (3S)-N-[2-(6-Fluoro-1H-indol-3-yl)-1-methylethyl]-1,2,3,4-tetrahydro-2-[N-(1H-imidazol-4-ylacetyl)-L-valyl]-3-isoquinolinecarboxamide. | m/z 559 (M+H)+ |
| 88 | 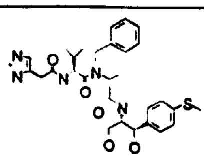 | [1S-[1R*(R*),2R*]]-1,2,3,4-Tetrahydro-N-[2-hydroxy-1-(hydroxymethyl)-2-[4-(methylthio)phenyl]ethyl]-2-[N-(1H-imidazol-4-ylacetyl)-L-valyl]-3-isoquinoline-carboxamide. | m/z 580 (M+H)+ |
| 89 | 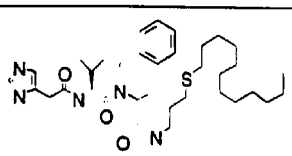 | (S)-N-[3-(Dodecylthio)propyl]-1,2,3,4-tetrahydro-2-[N-(1H-imidazol-4-ylacetyl)-L-valyl]-3-isoquinoline-carboxamide. | m/z 626 (M+H)+ |
| 90 | 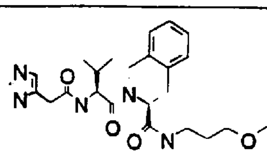 | (S)-1,2,3,4-Tetrahydro-2-[N-(1H-imidazol-4-ylacetyl)-L-valyl]-N-(3-methoxypropyl)-3-isoquinolinecarboxamide. | m/z 456 (M+H)+ |
| 91 | 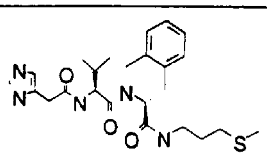 | (S)-1,2,3,4-Tetrahydro-2-[N-(1H-imidazol-4-ylacetyl)-L-valyl]-N-[3-(methylthio)propyl]-3-isoquinoline-carboxamide. | m/z 472 (M+H)+ |

| | | | |
|---|---|---|---|
| 92 | 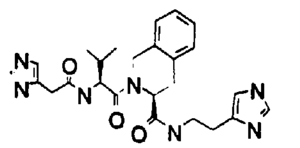 | (S)-1,2,3,4-Tetrahydro-2-[N-(1H-imidazol-4-ylacetyl)-L-valyl]-N-[2-(1H-imidazol-4-yl)ethyl]-3-isoquinolinecarboxamide. | m/z 478 (M+H)+ |
| 93 | 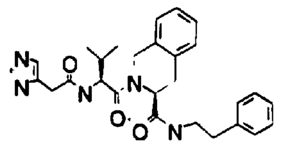 | (S)-1,2,3,4-Tetrahydro-2-[N-(1H-imidazol-4-ylacetyl)-L-valyl]-N-(2-phenylethyl)-3-isoquinolinecarboxamide. | m/z 488 (M+H)+ |
| 94 | 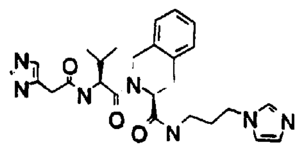 | (S)-1,2,3,4-Tetrahydro-2-[N-(1H-imidazol-4-ylacetyl)-L-valyl]-N-[3-(1H-imidazol-1-yl)propyl]-3-isoquinolinecarboxamide. | m/z 492 (M+H)+ |
| 95 | 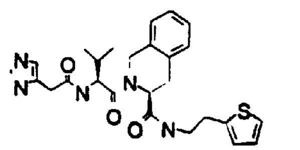 | (S)-1,2,3,4-Tetrahydro-2-[N-(1H-imidazol-4-ylacetyl)-L-valyl]-N-[2-(2-thienyl)ethyl]-3-isoquinolinecarboxamide. | m/z 494 (M+H)+ |
| 96 | 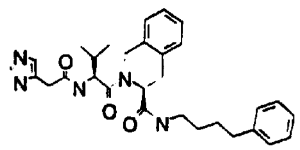 | (S)-1,2,3,4-Tetrahydro-2-[N-(1H-imidazol-4-ylacetyl)-L-valyl]-N-(4-phenylbutyl)-3-isoquinolinecarboxamide. | m/z 516 (M+H)+ |
| 97 | 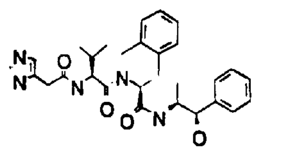 | [1S-[1R*(R*),2S*]]-1,2,3,4-Tetrahydro-N-(2-hydroxy-1-methyl-2-phenylethyl)-2-[N-(1H-imidazol-4-ylacetyl)-L-valyl]-3-isoquinolinecarboxamide. | m/z 518 (M+H)+ |
| 98 | 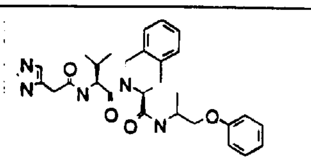 | (3S)-1,2,3,4-Tetrahydro-2-[N-(1H-imidazol-4-ylacetyl)-L-valyl]-N-(1-methyl-2-phenoxyethyl)-3-isoquinolinecarboxamide. | m/z 518 (M+H)+ |
| 99 | 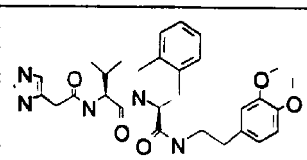 | (S)-N-[2-(3,4-Dimethoxyphenyl)ethyl]-1,2,3,4-tetra-hydro-2-[N-(1H-imidazol-4-ylacetyl)-L-valyl]-3-isoquinolinecarboxamide. | m/z 548 (M+H)+ |

| | | | |
|---|---|---|---|
| 100 | 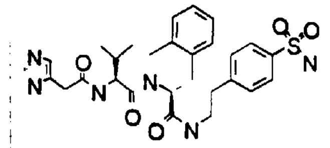 | (S)-N-[2-[4-(Aminosulfonyl)phenyl]ethyl]-1,2,3,4-tetrahydro-2-[N-(1H-imidazol-4-ylacetyl)-L-valyl]-3-isoquinolinecarboxamide. | m/z 567 (M+H)+ |
| 101 | 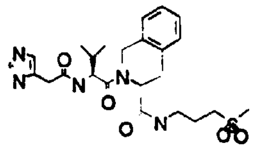 | (S)-1,2,3,4-Tetrahydro-2-[N-(1H-imidazol-4-ylacetyl)-L-valyl]-N-[3-(methylsulfonyl)propyl]-3-isoquinoline-carboxamide. | m/z 504 (M+H)+ |
| 102 | 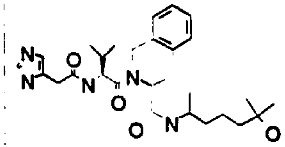 | (S)-1,2,3,4-Tetrahydro-N-(5-hydroxy-1,5-dimethylhexyl)-2-[N-(1H-imidazol-4-ylacetyl)-L-valyl]-3-isoquinoline-carboxamide. | m/z 512 (M+H)+ |
| 103 | 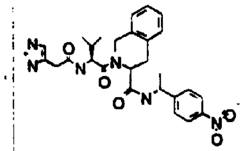 | [S-(R*,S*)]-1,2,3,4-Tetrahydro-2-[N-(1H-imidazol-4-ylacetyl)-L-valyl]-N-[2-(4-nitrophenyl)ethyl]-3-isoquinolinecarboxamide. | m/z 533 (M+H)+ |
| 104 | 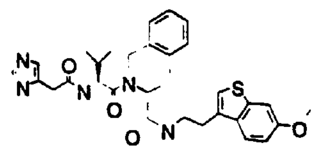 | (S)-1,2,3,4-tetrahydro-2-[N-(1H-imidazol-4-ylacetyl)-L-valyl]-N-[2-(6-methoxybenzo[b]thiophen-3-yl)ethyl]-3-isoquinolinecarboxamide. | m/z 574 (M+H)+ |
| 105 | 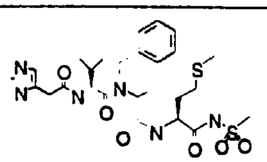 | (S)-N-(Methylsulfonyl)-N<2-[[1,2,3,4-tetrahydro-2-[N-(1H-imidazol-4-ylacetyl)-L-valyl]-3-isoquinolinyl]-carbonyl]-L-methioninamide. | m/z 593 (M+H)+ |
| 106 | 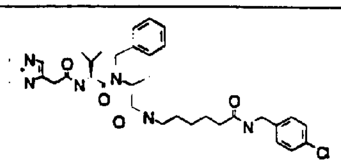 | (S)-N-[6-[[(4-Chlorophenyl)methyl]amino]-6-oxohexyl]-1,2,3,4-tetrahydro-2-[N-(1H-imidazol-4-ylacetyl)-L-valyl]-3-isoquinolinecarboxamide. | m/z 622 (M+H)+ |
| 107 | 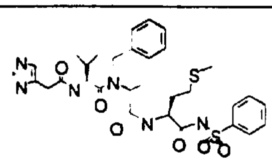 | (S)-N-(Phenylsulfonyl)-N<2-[[1,2,3,4-tetrahydro-2-[N-(1H-imidazol-4-ylacetyl)-L-valyl]-3-isoquinolinyl]-carbonyl]-L-methioninamide. | m/z 655 (M+H)+ |

| | | | |
|---|---|---|---|
| 108 | 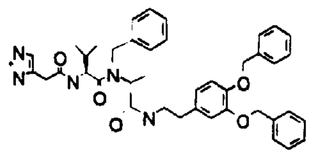 | (S)-N-[2-[3,4-Bis(phenylmethoxy)phenyl]ethyl]-1,2,3,4-tetrahydro-2-[N-(1H-imidazol-4-ylacetyl)-L-valyl]-3-isoquinolinecarboxamide. | m/z 700 (M+H)+ |
| 109 | 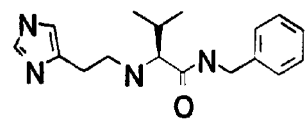 | N<2-[2-(1H-Imidazol-4-yl)ethyl]-N-(phenylmethyl)-L-valinamide, trifluoroacetate (1:2). | m/z 301 (M+H)+ |
| 110 | 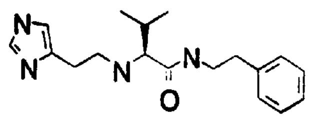 | N<2-[2-(1H-Imidazol-4-yl)ethyl]-N-(2-phenylethyl)-L-valinamide, trifluoroacetate (1:2). | m/z 315 (M+H)+ |
| 111 | 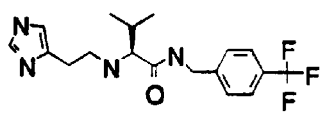 | N<2-[2-(1H-Imidazol-4-yl)ethyl]-N-[[4-(trifluorometh-yl)phenyl]methyl]-L-valinamide. | m/z 369 (M+H)+ |
| 112 | 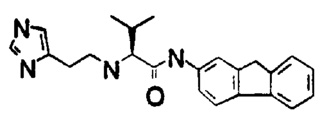 | N-(9H-Fluoren-2-yl)-N<2-[2-(1H-imidazol-4-yl)ethyl]-L-valinamide, trifluoroacetate (1:2). | m/z 375 (M+H)+ |
| 113 | 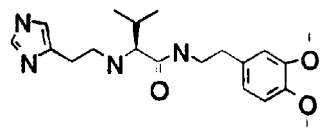 | N-[2-(3,4-Dimethoxyphenyl)ethyl]-N<2-[2-(1H-imidazol-4-yl)ethyl]-L-valinamide. | m/z 375 (M+H)+ |
| 114 | 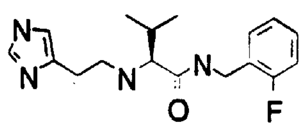 | N-[(2-Fluorophenyl)methyl]-N<2-[2-(1H-imidazol-4-yl)ethyl]-L-valinamide. | m/z 309 (M+H)+ |
| 115 | 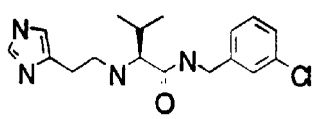 | N-[(3-Chlorophenyl)methyl]-N<2-[2-(1H-imidazol-4-yl)ethyl]-L-valinamide. | m/z 335 (M+H)+ |

| | | | |
|---|---|---|---|
| 116 | 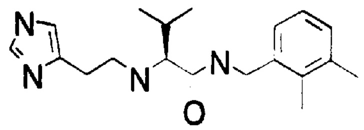 | N-[(2,3-Dimethylphenyl)methyl]-N<2-[2-(1H-imidazol-4-yl)ethyl]-L-valinamide. | m/z 329 (M+H)+ |
| 117 | 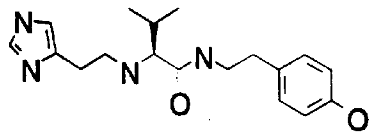 | N-[2-(4-Hydroxyphenyl)ethyl]-N<2-[2-(1H-imidazol-4-yl)ethyl]-L-valinamide. | m/z 331 (M+H)+ |
| 118 | 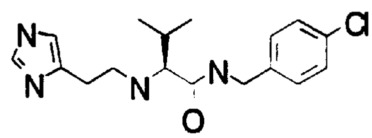 | N-[(4-Chlorophenyl)methyl]-N<2-[2-(1H-imidazol-4-yl)ethyl]-L-valinamide. | m/z 335 (M+H)+ |
| 119 | 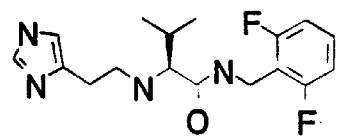 | N-[(2,6-Difluorophenyl)methyl]-N<2-[2-(1H-imidazol-4-yl)ethyl]-L-valinamide. | m/z 337 (M+H)+ |
| 120 | 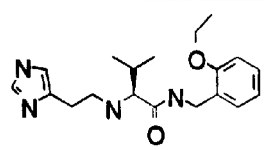 | N-[(2-Ethoxyphenyl)methyl]-N<2-[2-(1H-imidazol-4-yl)ethyl]-L-valinamide. | m/z 345 (M+H)+ |
| 121 | 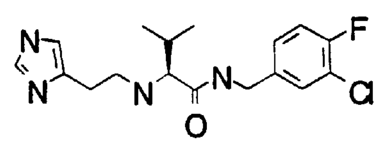 | N-[(3-Chloro-4-fluorophenyl)methyl]-N<2-[2-(1H-imidazol-4-yl)ethyl]-L-valinamide. | m/z 353 (M+H)+ |
| 122 | 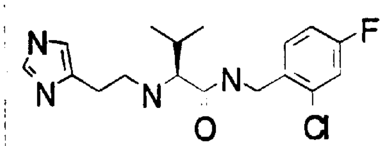 | N-[(2-Chloro-4-fluorophenyl)methyl]-N<2-[2-(1H-imidazol-4-yl)ethyl]-L-valinamide. | m/z 353 (M+H)+ |
| 123 | 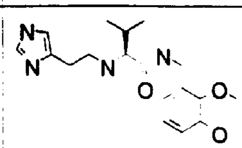 | N-[(2,3-Dimethoxyphenyl)methyl]-N<2-[2-(1H-imidazol-4-yl)ethyl]-L-valinamide. | m/z 361 (M+H)+ |

| | | | |
|---|---|---|---|
| 124 | 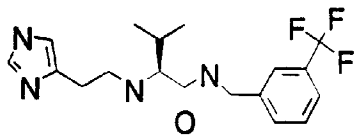 | N<2-[2-(1H-Imidazol-4-yl)ethyl]-N-[[3-(trifluorometh-yl)phenyl]methyl]-L-valinamide. | m/z 369 (M+H)+ |
| 125 | 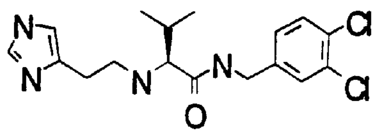 | N-[(3,4-Dichlorophenyl)methyl]-N<2-[2-(1H-imidazol-4-yl)ethyl]-L-valinamide. | m/z 369 (M+H)+ |
| 126 | 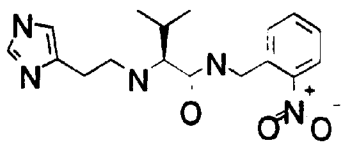 | N<2-[2-(1H-Imidazol-4-yl)ethyl]-N-[(2-nitrophenyl)-methyl]-L-valinamide. | m/z 346 (M+H)+ |
| 127 | 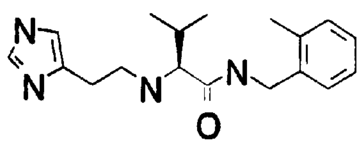 | N<2-[2-(1H-Imidazol-4-yl)ethyl]-N-[(2-methylphenyl)-methyl]-L-valinamide, trifluoroacetate. | m/z 315 (M+H)+ |
| 128 | 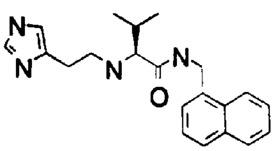 | N<2-[2-(1H-Imidazol-4-yl)ethyl]-N-(1-naphthalenylmeth-yl)-L-valinamide, trifluoroacetate. | m/z 351 (M+H)+ |
| 129 | 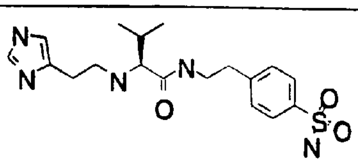 | N-[2-[4-(Aminosulfonyl)phenyl]ethyl]-N<2-[2-(1H-imidazol-4-yl)ethyl]-L-valinamide, trifluoroacetate. | m/z 394 (M+H)+ |
| 130 | 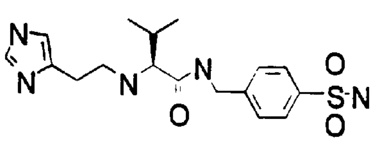 | N-[[4-(Aminosulfonyl)phenyl]methyl]-N<2-[2-(1H-imidazol-4-yl)ethyl]-L-valinamide, trifluoroacetate. | m/z 380 (M+H)+ |
| 131 | 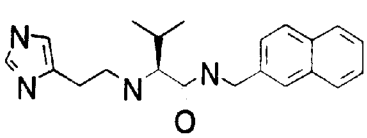 | N<2-[2-(1H-Imidazol-4-yl)ethyl]-N-(2-naphthalenylmeth-yl)-L-valinamide, trifluoroacetate. | m/z 351 (M+H)+ |

| | | | |
|---|---|---|---|
| 132 | 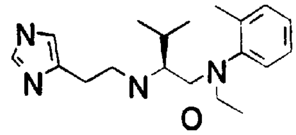 | N-Ethyl-N<2-[2-(1H-imidazol-4-yl)ethyl]-N-(2-methyl-phenyl)-L-valinamide, trifluoroacetate. | m/z 329 (M+H)+ |
| 133 | 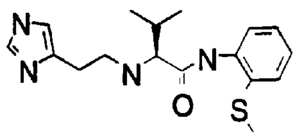 | N<2-[2-(1H-Imidazol-4-yl)ethyl]-N-[2-(methylthio)-phenyl]-L-valinamide, trifluoroacetate. | m/z 333 (M+H)+ |
| 134 | 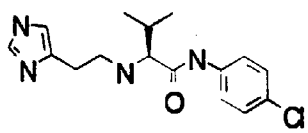 | N-(4-Chlorophenyl)-N<2-[2-(1H-imidazol-4-yl)ethyl]-N-methyl-L-valinamide, trifluoroacetate. | m/z 335 (M+H)+ |
| 135 | 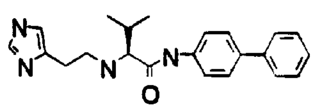 | N-([1,1'-Biphenyl]-4-yl)-N<2-[2-(1H-imidazol-4-yl)ethyl]-L-valinamide, trifluoroacetate. | m/z 363 (M+H)+ |
| 136 | 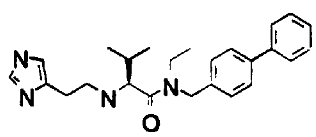 | N-([1,1'-Biphenyl]-4-ylmethyl)-N-ethyl-N<2-[2-(1H-imidazol-4-yl)ethyl]-L-valinamide, trifluoroacetate. | m/z 405 (M+H)+ |
| 137 | 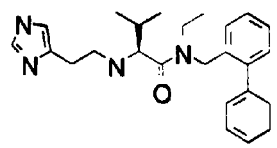 | N-([1,1'-Biphenyl]-2-ylmethyl)-N-ethyl-N<2-[2-(1H-imidazol-4-yl)ethyl]-L-valinamide, trifluoroacetate. | m/z 405 (M+H)+ |
| 138 | 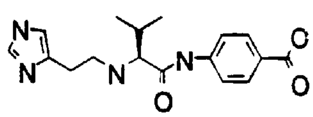 | 4-[[N-[2-(1H-Imidazol-4-yl)ethyl]-L-valyl]amino]benzoicacid, trifluoroacetate. | m/z 331 (M+H)+ |
| 139 | 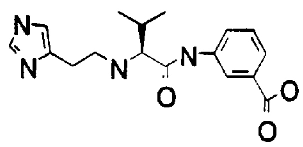 | 3-[[N-[2-(1H-Imidazol-4-yl)ethyl]-L-valyl]amino]benzoicacid, trifluoroacetate. | m/z 331 (M+H)+ |

| | | | |
|---|---|---|---|
| 140 | 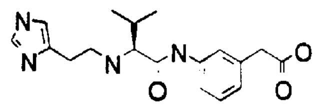 | 3-[[N-[2-(1H-Imidazol-4-yl)ethyl]-L-valyl]amino]-benzeneacetic acid, trifluoroacetate. | m/z 345 (M+H)+ |
| 141 | 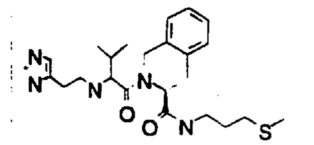 | (S)-1,2,3,4-Tetrahydro-2-[N-[2-(1H-imidazol-4-yl)-ethyl]-DL-valyl]-N-[3-(methylthio)propyl]-3-isoquinolinecarboxamide. | m/z 458 (M+H)+ |
| 142 | 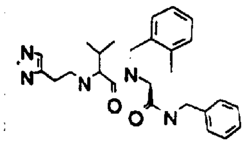 | (S)-1,2,3,4-Tetrahydro-2-[N-[2-(1H-imidazol-4-yl)-ethyl]-DL-valyl]-N-(phenylmethyl)-3-isoquinoline-carboxamide. | m/z 460 (M+H)+ |
| 143 | 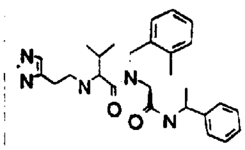 | (3S)-1,2,3,4-Tetrahydro-2-[N-[2-(1H-imidazol-4-yl)-ethyl]-DL-valyl]-N-(1-phenylethyl)-3-isoquinoline-carboxamide. | m/z 474 (M+H)+ |
| 144 | 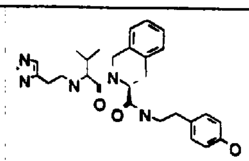 | (S)-1,2,3,4-Tetrahydro-N-[2-(4-hydroxyphenyl)ethyl]-2-[N-[2-(1H-imidazol-4-yl)ethyl]-DL-valyl]-3-isoquinolinecarboxamide. | m/z 490 (M+H)+ |
| 145 | 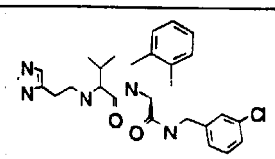 | (S)-N-[(3-Chlorophenyl)methyl]-1,2,3,4-tetrahydro-2-[N-[2-(1H-imidazol-4-yl)ethyl]-DL-valyl]-3-isoquinolinecarboxamide. | m/z 495 (M+H)+ |
| 146 | 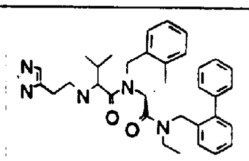 | (S)-N-([1,1'-Biphenyl]-2-yl)-N-ethyl-1,2,3,4-tetrahydro-2-[N-[2-(1H-imidazol-4-yl)ethyl]-DL-valyl]-3-isoquinolinecarboxamide. | m/z 564 (M+H)+ |
| 147 | 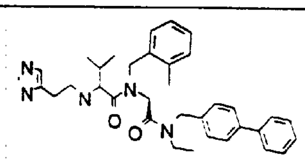 | (S)-N-([1,1'-Biphenyl]-4-yl)-N-ethyl-1,2,3,4-tetrahydro-2-[N-[2-(1H-imidazol-4-yl)ethyl]-DL-valyl]-3-isoquinolinecarboxamide. | m/z 564 (M+H)+ |

| | | | |
|---|---|---|---|
| 148 | 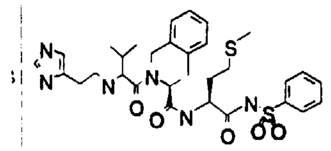 | (S)-N-(Phenylsulfonyl)-N<2-[[1,2,3,4-tetrahydro-2-[N-[2-(1H-imidazol-4-yl)ethyl]-L-valyl]-3-isoquinolinyl]-carbonyl]-DL-methioninamide. | m/z 641 (M+H)+ |

## Claims

1. A compound of the formula

I

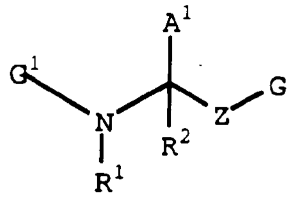

or such a compound in enantiomer, diastereomer, pharmaceutically acceptable salt, prodrug or solvate form, wherein:

G is

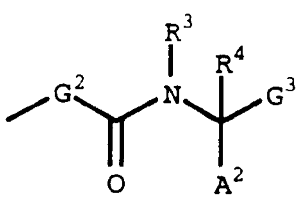 , 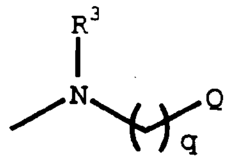 ,

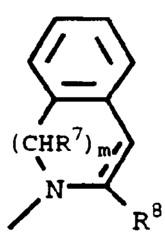 or 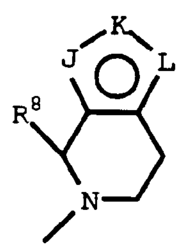 ;

$G^1$ is

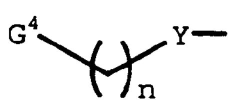

or

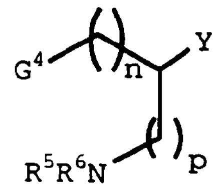

;

$G^2$ is

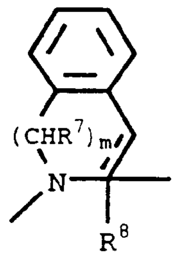

,

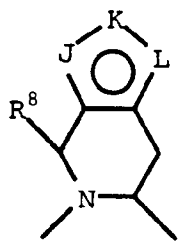

or $-NR^{10}-CH(Q^1)-$;

J, K and L are each, independently, N, $NR^9$, O, S or $CR^{10}$ with the provisos that only one of the groups J, K and L can be O or S, and at least one of the groups J or L must be N, $NR^9$, O or S to form a fused five-membered heteroring; the bond between J and K or K and L may also form one side of a phenyl ring fused to the fused five-membered heteroring;

Q is aryl;

$Q^1$, $A^1$ and $A^2$ are each, independently, H, alkyl, substituted alkyl, phenyl or substituted phenyl;

$G^3$ is $R^{11}$, $-C(O)OR^{11}$, $-C(O)NR^{11}R^{12}$, 5-tetrazolyl, $-C(O)N(R^{13})OR^{11}$, $C(O)NHSO_2R^{14}$ or $-CH_2OR^{11}$;

$G^4$ is

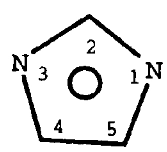

attached at the 1,2, 4 or 5 position and optionally substituted, at any of the available position or positions on the ring, with halo, alkyl or substituted alkyl having 1 to 20 carbon atoms, alkoxy, aryl, aralkyl, hydroxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, N-hydroxycarbamyl, N-alkylcarbamyl, N-dialkylcarbamyl, alkoxycarbonyl, phenyl, substituted phenyl, or a combinaton of these groups;

Y and Z are each, independently, $-CH_2-$ or $-C(O)-$;

$R^1$ - $R^{14}$ are each, independently, H or alkyl having 1 to 20 carbon atoms;

$R^7$, $R^8$ and $R^{14}$ may also be aryl or aralkyl, and $R^3$, $R^9$, $R^{11}$, $R^{12}$ and $R^{13}$ may also be aralkyl;

m, n and p are each, independently, 0 or an integer from 1 to 2;

q is 0 or an integer from 1 to 4; and

the dotted line represents an optional double bond.

**2.** A compound of Claim 1, wherein

G is

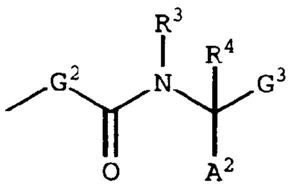 or 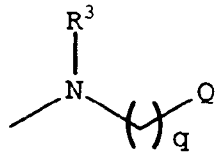 ;

$G^2$ is

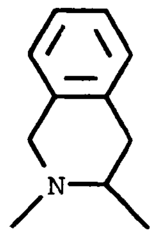 ;

$G^4$ is

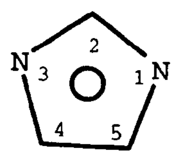

attached at the 1, 2, 4 or 5 position, and optionally substituted at any available position or positions on the ring, with alkyl having 1 to 20 carbon atoms or aralkyl; and $A^1$ and $A^2$ are each, independently, H or D-, L-or D,L- $-CH_3$, $-CH(CH_3)_2$, $-CH_2CH(CH_3)_2$, $-CH(CH_3)CH_2CH_3$, $-C(CH_3)_3$, $-CH_2OH$, $-CH_2CH_2OH$, $-CH_2CH_2CH_2OH$, $-CH(OH)CH_3$,

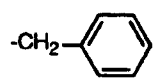 ,

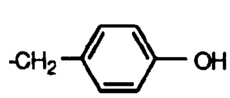 , 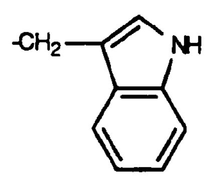 ,

$-CH_2CH_2CH_2CH_2NH_2$,

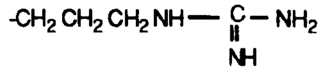 ,

$-CH_2C(O)OH$, $-CH_2CH_2C(O)OH$, $-CH_2C(O)NH_2$, $-CH_2CH_2C(O)NH_2$, $-CH_2CH_2OCH_3$, $-CH_2CH_2CONR^{15}R^{16}$,

$-CH_2SH$, $-CH_2CH_2SH$, -CH2$CH_2SOCH_3$, $-CH_2CH_2SO_2CH_3$ or $-CH_2CH_2SCH_3$, where $R^{15}$ and $R^{16}$ are each, independently, hydrogen or alkyl, or $R^{15}$ and $R^{16}$, taken together, form a 5- to 7-membered, optionally substituted, saturated ring with the N-atom to which they are attached.

**3.** A compound of Claim 1, wherein $G^2$ is $-NR^{10}CH(Q^1)-$ and $Q^1$ is

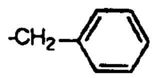 , 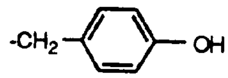 ,

or

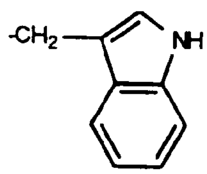 .

**4.** A compound of Claim 1, 2 or 3 wherein $A^1$ and $A^2$ are each, independently, H or D-, L- or D,L- $-CH_3$, $-CH(CH_3)_2$, $-CH_2CH(CH_3)_2$, $-CH(CH_3)CH_2CH_3$, $-C(CH_3)_3$, $-CH_2OH$, $-CH_2CH_2OH$, $-CH_2CH_2CH_2OH$, $-CH(OH)CH_3$,

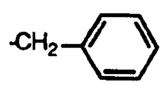 , 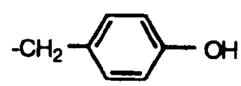 ,

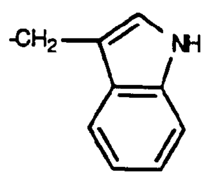 ,

$-CH_2\ CH_2\ CH_2\ CH_2\ NH_2$,

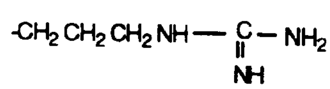 ,

$-CH_2C(O)OH$, $-CH_2CH_2C(O)OH$, $-CH_2C(O)NH_2$, $-CH_2CH_2C(O)NH_2$, $-CH_2CH_2OCH_3$, $-CH_2CH_2CONR^{15}R^{16}$, $-CH_2SH$, $-CH_2CH_2SH$, $-CH_2CH_2SOCH_3$, $-CH_2CH_2SO_2CH_3$ or $-CH_2CH_2SCH_3$, where $R^{15}$ and $R^{16}$ are each, independently, hydrogen or alkyl, or $R^{15}$ and $R^{16}$, taken together, form a 5- to 7-membered, optionally substituted, saturated ring with the N-atom to which they are attached.

**5.** A compound of Claim 1, 2 or 3 wherein $A^1$ is L- $-CH_3$, $-CH(CH_3)_2$, $-CH_2CH(CH_3)_2$, $-C(CH_3)_3$,

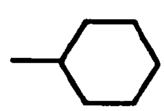

$-CH(CH_3)CH_2CH_3$, $-CH_2OH$ or $-CH(OH)CH_3$.

6. A compound of Claim 1, 2 or 3 wherein $A^2$ is L- $-CH_2CH_2SCH_3$, $-CH_2CH_2OH$, $-CH_2CH_2CH_2OH$, $-CH_2CH_2C(O)NH_2$, $-CH_2CH_2OCH_3$, $-CH_2CH_2CONR^{15}R^{16}$, $-CH_2CH_2SH$, $-CH_2CH_2SOCH_3$ or $-CH_2CH_2SO_2CH_3$.

7. A compound of Claim 1, wherein $G^2$ is

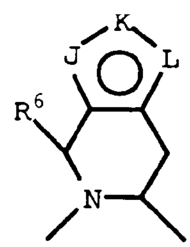

and the fused five-membered optionally substituted heteroring is

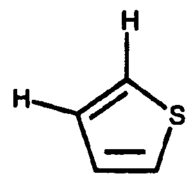

,

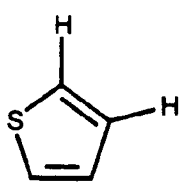

,

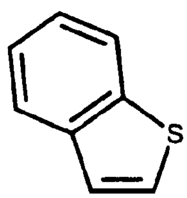

,

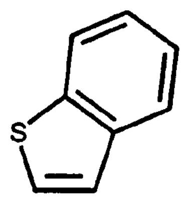

,

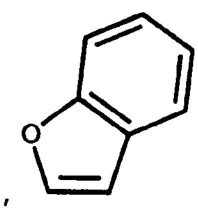

,

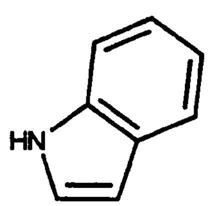

or

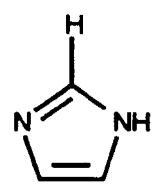

.

8. A compound of Claim 1, wherein $G^4$ is

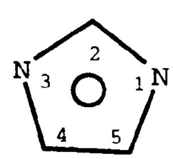

attached at the 1, 2, 4 or 5 position, and optionally substituted at any available position or positions on the ring, with alkyl having 1 to 20 carbon atoms or aralkyl.

9. A compound of Claim 1, wherein $G^2$ is

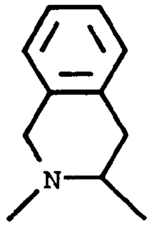

10. A compound of Claim 1, wherein $G^2$ is

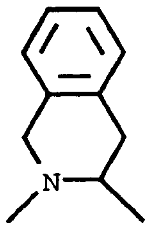

;

$G^3$ is $-C(O)OH$, $-C(O)OR^{11}$ or $-C(O)NHSO_2R^{14}$; $G^4$ is

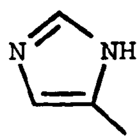

;

$A^1$ is L- $-CH_3$, $-CH(CH_3)_2$, $-CH_2CH(CH_3)_2$, $-C(CH_3)_3$,

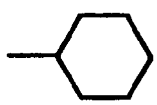

$-CH(CH_3)CH_2CH_3$, $-CH_2OH$ or $-CH(OH)CH_3$; $A^2$ is L- $-CH_2CH_2SCH_3$, $-CH_2CH_2OH$, $-CH_2CH_2CH_2OH$ $-CH_2CH_2C(O)NH_2$, $-CH_2CH_2OCH_3$, $-CH_2CH_2CONR^{15}R^{16}$, $-CH_2CH_2SH$, $-CH_2CH_2SOCH_3$ or $-CH_2CH_2SO_2CH_3$; and $R^1$, $R^2$, $R^3$ and $R^4$ are H.

11. A compound of Claim 1, which is
(R*)-N-[[1,2,3,4-Tetrahydro-2-[N-[2-(1H-imidazol-4-ylacetyl)]-L-valyl]-3-isoquinolinyl]carbonyl]-L-methionine;
(R*)-N-[[1,2,3,4-Tetrahydro-2-[N-(1H-imidazol-4-ylcarbonyl)-L-valyl]-3-isoquinolinyl]carbonyl]-L-methionine;
(R*)-N-[[1,2,3,4-Tetrahydro-2-[N-(2-(1H-imidazol-4-yl)ethyl]-L-valyl]-3-isoquinolinyl]carbonyl]-L-methionine;
N-[(2,3-Dichlorophenyl)methyl]-$N^2$-(1H-imidazol-4-ylacetyl)-L-isoleucinamide;
(S)-N-[2-Methyl-1-[(1,2,3,4-tetrahydro-2-isoquinolinyl)carbonyl]butyl]-1H-imidazole-4 acetamide;
(R*)-N-2-[[1,2,3,4-Tetrahydro-2-[N-(1H-imidazol-2-ylmethyl)-3-methyl-L-valyl]-3-isoquinolinyl]carbonyl]-L-glutamine;
(R*)-$N^2$-[[1,2,3,4-Tetrahydro-2-[N-[(1-methyl-1H-imidazol-4-yl)acetyl]-3-methyl-L-valyl]-3-isoquinolinyl]carbonyl]-L-glutamine;
(R*)-$N^2$-[[1,2,3,4-Tetrahydro-2-[N-[3-(1H-imidazol-4-yl)-1-oxopropyl]-N-methyl-L-valyl]-3-isoquinolinyl]-carbonyl]-L-glutamine;
(R*)-N-[[1,2,3,4-Tetrahydro-2-[N-(1H-imidazol-4-ylmethyl)-L-valyl]-3-isoquinolinyl]carbonyl]-L-methionine;
(S)-N-[[1,2,3,4-Tetrahydro-2-[N-(1H-imidazol-4-ylacetyl)-3-methyl-L-valyl]-3-isoquinolinyl]carbonyl]-L-glutamine;
(S)-N-[[1,2,3,4-Tetrahydro-2-[N-(1H-imidazol-4-ylacetyl)-3-methyl-L-valyl]-3-isoquinolinyl]carbonyl]-L-glutamic acid;
$N^2$-[[(S)-2-[N-[2-amino-2-(1H-imidazol-4-yl)ethyl]-3-methyl-L-valyl]-1,2,3,4-tetrahydro-3-isoquinolinyl]carbonyl]-L-glutamine;

(S,S)-$N^2$-[[2-[[2-Amino-3-(1H-imidazol-4-yl)-1-oxopropyl]-3-methyl-L-valyl]-1,2,3,4-tetrahydro-3-isoquinolinyl]-carbonyl]-L-glutamine;
(S)-N-[[1,2,3,4-tetrahydro-2-[N-[2-(1H-imidazol-4-yl)propyl]-3-methyl-L-valyl]-3-isoquinolinyl]carbonyl]-L-glutamine;
(S)-N-[[1,2,3,4-tetrahydro-2-[N-[2-(lH-imidazol-4-yl)ethyl]-3-methyl-L-valyl]-3-isoquinolinyl]carbonyl]-L-glutamine;
(S)-1,2,3,4-Tetrahydro-2-[2-[[2-(1H-imidazol-4-yl)ethyl]amino]-3-methyl-1-oxobutyl]isoquinoline;
(S)-3-[[[(2,3-Dichlorophenyl)methyl][2-[[2-(1H-imidazol-4-yl)ethyl]amino]-3-methyl-1-oxobutyl]amino]methyl]benzoic acid;
N-[(2,3-Dichlorophenyl)methyl]-$N^2$-[2-(1H-imidazol-4-yl)ethyl-L-valinamide;
N-[(2,3-Dichlorophenyl)methyl]-$N^2$-[2-(1H-imidazol-2-yl)ethyl]-L-valinamide;
[S-(R*,R*)]-1,2,3,4-Tetrahydro-2-[N-(1H-imidazol-4-ylacetyl)-L-valyl]-N-[3-(methylsulfonyl)-1-[(phenylmethoxy)methyl]propyl]-3-isoquinolinecarboxamide;
N-[(2,3-Dichlorophenyl)methyl]-$N^2$-[2-(2-methyl-1H-imidazol-4-yl)ethyl]-L-valinamide;
N-[(2,3-Dichlorophenyl)methyl]-$N^2$-[2-(5-methyl-1H- imidazol-4-yl)ethyl]-L-valinamide;
(3S)-1,2,3,4-Tetrahydro-2-[N-[2-(1H-imidazol-4-yl)ethyl]-D,L-valyl]-N-[3-(methylsulfonyl)propyl]-3-isoquinolinecarboxamide;
(3S)-N-[[1,2,3,4-Tetrahydro-2-[N-[2-(1H-imidazol-4-yl)ethyl]-D,L-valyl]-3-isoquinolinyl]carbonyl]-3-[(methylsulfonyl)methyl]-L-alanine, methyl ester;
(3S)-N-[[1,2,3,4-Tetrahydro-2-[N-[2-(1H-imidazol-4-yl)ethyl]-D,L-valyl]-3-isoquinolinyl]carbonyl]-3-[(methylsulfonyl)methyl]-L-alanine;
N-[(S)-2-[12-[[2-(1H-imidazol-4-yl)ethyl]amino]-3-methylbutyl]amino]-1-oxo-3-phenylpropyl]-3-[(methylsulfonyl)methyl]-L-alanine;
N-[(2,3-Dichlorophenyl)methyl]-2-[[2-(1H-imidazol-4-yl)ethyl]amino]acetamide;
N-[(2,3-Dichlorophenyl)methyl]-$N^2$-[2-(1-methyl-1H-imidazol-5-yl)ethyl]-D,L-valinamide;
N-[(2,3-Dichlorophenyl)methyl]-$N^2$-[2-(1-methyl-1H-imidazol-4-yl)ethyl]-D,L-valinamide;
N-Ethyl-$N^2$-[2-(1H-imidazol-4-yl)ethyl]-N-[(4-methoxyphenyl)methyl]-L-valinamide;
N-[(2,3-Dichlorophenyl)methyl]-2-[[2-(1H-imidazol-4-yl)ethyl]amino]-3-methylpentanamide;
N-[(2,3-Dichlorophenyl)methyl]-2-[[2-(1H-imidazol-4-yl)ethyl]amino]-4-phenylbutanamide;
N-[(2,3-Dichlorophenyl)methyl]-$N^2$-[2-[1-(phenylmethyl)-(1H-imidazol-5-yl)]ethyl]-L-valinamide;
N-[(2,3-Dichlorophenyl)methyl]-$N^2$-[2-[1-(3-methylbutyl)-1H-imidazol-5-yl]ethyl]-L-valinamide;$N^2$-[2-(1H-Imidazol-4-yl)ethyl]-N,N-bis(phenylmethyl)-D,L-valinamide;
N-[(2,3-Dichlorophenyl)methyl]-$N^2$-[2-[1-(2-phenylethyl)-1H-imidazol-5-yl]ethyl]-L-valinamide;
N-[(2,3-Dichlorophenyl)methyl]-$N^2$-[2-(1-dodecyl-1H-imidazol-5-yl)ethyl]-L-valinamide;
N-[(2,3-Dichlorophenyl)methyl]-$N^2$-[2-(imidazol-1-yl)ethyl]-D,L-valinamide;
$N^2$-[2-(1H-Imidazol-5-yl)ethyl]-N-(1-methylethyl)-N-(phenylmethyl)-L-valinamide;
N-[(2,3-Dichlorophenyl)methyl]-$N^2$-[2-(1H-imidazol-5-yl)ethyl]-N-(1-methylethyl)-L-valinamide;
N-[(2,3-Dichlorophenyl)methyl]-$N^2$-[2-(1H-imidazol-5-yl)ethyl]-N-(phenylmethyl)-L-valinamide;
(S)-3-[[[2-[[2-(1H-Imidazol-4-yl)ethyl]amino]-3-methyl-1-oxobutyl](phenyl-methyl)amino]methyl]benzoic acid;
N-Ethyl-$N^2$-[2-(1H-imidazol-4-yl)ethyl]-N-(phenylmethyl)-D,L-valinamide;
N-[(2,3-Dichlorophenyl)methyl]-$N^2$-[3-(1H-imidazol-2-yl)propyl]-L-valinamide;
$N^2$-[2-(1H-imidazol-5-yl)ethyl]-N,N-bis(phenylmethyl)-L-valinamide;
N-[(2,3-Dichlorophenyl)methyl]-2-[[3-(1H-imidazol-4-yl)propyl]amino]-4-methylpentanamide;
[S-(R*,R*)]-1,2,3,4-Tetrahydro-2-[2-[[2-(1H-imidazol-4-yl)ethyl]amino]-3-methyl-1-oxopentyl]isoquinoline;
2-[[2-(2-Amino-1H-imidazol-4-yl)]ethyl]-N-[(2,3-dichlorophenyl)methyl]-3-methylbutanamide;
N-[(2,3-Dichlorophenyl)methyl]-$N^2$-[2-(1H-imidazol-4-yl)ethyl]-D,L-valinamide;
N-[2,3-Dichlorophenyl)methyl]-$N^2$-[2-(1-methyl-1H-imidazol-5-yl)ethyl]-L-valinamide;
(S)-N-[[1,2,3,4-Tetrahydro-2-[N-[3-(1H-imidazol-2-yl)propyl]-L-valyl]-3-isoquinolinyl]carbonyl]-L-methionine;

| | |
|---|---|
| N<2-[2-(1H-Imidazol-4-yl)ethyl]-N-phenyl-L-valinamide. | N-Ethyl-N-[(2-fluorophenyl)methyl]-N<2-[2-(1H-imidazol-4-yl)ethyl]-L-valinamide. |
| N<2-[2-(1H-Imidazol-4-yl)ethyl]-N-[(3-methylphenyl)-methyl]-L-valinamide. | N-Butyl-N<2-[2-(1H-imidazol-4-yl)ethyl]-N-(phenyl-methyl)-L-valinamide. |
| N<2-[2-(1H-Imidazol-4-yl)ethyl]-N-[(4-methylphenyl)-methyl]-L-valinamide. | N-Ethyl-N<2-[2-(1H-imidazol-4-yl)ethyl]-N-[(2-methoxy-phenyl)methyl]-L-valinamide. |
| N<2-[2-(1H-Imidazol-4-yl)ethyl]-N-(3-phenylpropyl)-L-valinamide. | N<2-[2-(1H-Imidazol-4-yl)ethyl]-N-methyl-N-(1-naphth-alenylmethyl)-L-valinamide. |
| N-Ethyl-N<2-[2-(1H-imidazol-4-yl)ethyl]-N-[(2-methyl-phenyl)methyl]-L-valinamide. | N<2-[2-(1H-Imidazol-4-yl)ethyl]-N-(2-phenylethyl)-N-(phenylmethyl)-L-valinamide. |
| N-Ethyl-N<2-[2-(1H-imidazol-4-yl)ethyl]-N-[(4-methyl-phenyl)methyl]-L-valinamide. | N-(9-Anthracenylmethyl)-N<2-[2-(1H-imidazol-4-yl)-ethyl]-N-methyl-L-valinamide. |

| | |
|---|---|
| N-Dodecyl-N<2-[2-(1H-imidazol-4-yl)ethyl]-N-(phenyl-methyl)-L-valinamide. | N-[(2-Chlorophenyl)methyl]-N<2-[2-(1H-imidazol-4-yl)-ethyl]-L-valinamide, trifluoroacetate. |
| (S)-1,2,3,4-Tetrahydro-1-[2-[[2-(1H-imidazol-4-yl)-ethyl]amino]-3-methyl-1-oxobutyl]quinoline. | N-Ethyl-N<2-[2-(1H-imidazol-4-yl)ethyl]-N-[(3-methyl-phenyl)methyl]-L-valinamide, trifluoroacetate. |
| N-[2-(3-Fluorophenyl)ethyl]-N<2-[2-(1H-imidazol-4-yl)ethyl]-L-valinamide. | N-(2-Cyanoethyl)-N<2-[2-(1H-imidazol-4-yl)ethyl]-N-(phenylmethyl)-L-valinamide, trifluoroacetate. |
| N-[2-(2-Fluorophenyl)ethyl]-N<2-[2-(1H-imidazol-4-yl)ethyl]-L-valinamide. | N-[(4-Chlorophenyl)methyl]-N-ethyl-N<2-[2-(1H-imidazol-4-yl)ethyl]-L-valinamide, trifluoroacetate. |
| N<2-[2-(1H-Imidazol-4-yl)ethyl]-N-[2-(2-methoxy-phenyl)ethyl]-L-valinamide. | N-[(2-Chlorophenyl)methyl]-N-ethyl-N<2-[2-(1H-imidazol-4-yl)ethyl]-L-valinamide, trifluoroacetate. |
| N<2-[2-(1H-Imidazol-4-yl)ethyl]-N-[2-(3-methoxy-phenyl)ethyl]-L-valinamide. | N-[(2-Chloro-6-fluorophenyl)methyl]-N ethyl-N<2-[2-(1H-imidazol-4-yl)ethyl]-L valinamide, trifluoroacetate. |
| N-[2-(2-Chlorophenyl)ethyl]-N<2-[2-(1H-imidazol-4-yl)ethyl]-L-valinamide. | N-[(2,4-Dichlorophenyl)methyl]-N-ethy N<2-[2-(1H-imidazol-4-yl)ethyl]-L-valinamide, trifluoroacetate. |
| N-[(2-Hydroxy-3-methoxyphenyl)methyl]-N<2-[2-(1H-imidazol-4-yl)ethyl]-N-(phenylmethyl)-L-valinamide. | N-[(2-Hydroxyphenyl)methyl]-N<2-[2-(1H-imidazol-4-yl)ethyl]-N-(2-phenylethyl)-L-valinamide,trifluoroacetate. |
| N<2-[2-(1H-Imidazol-4-yl)ethyl]-N-[(4-methoxyphenyl)-methyl]-L-valinamide. | N,N-Bis[(2-ethoxyphenyl)methyl]-N<2-[2-(1H-imidazol-4-yl)ethyl]-L-valinamide, trifluoroacetate. |

| | |
|---|---|
| (3S)-N-(2-Hydroxypropyl)-N<2-[2-(1H-imidazol-4-yl)-ethyl]-L-valinamide. | N-[(2,3-Dichlorophenyl)methyl]-N-ethyl-N<2-[2-(1H-imidazol-4-yl)ethyl]-L-valinamide, trifluoroacetate. |
| (S)-1,2,3,4-Tetrahydro-2-[N-(1H-imidazol-4-ylacetyl)-L-valyl]-N-[2-(1-methyl-1H-pyrrol-2-yl)ethyl]-3-isoquinolinecarboxamide. | (S)-1,2,3,4-Tetrahydro-2-[N-(1H-imidazol-4-ylacetyl)-L-valyl]-N-[2-(1H-imidazol-4-yl)ethyl]-3-isoquinoline-carboxamide. |
| (3S)-1,2,3,4-Tetrahydro-N-[1-(hydroxymethyl)-3-(methylthio)propyl]-2-[N-(1H-imidazol-4-ylacetyl)-L-valyl]-3-isoquinolinecarboxamide. | (S)-1,2,3,4-Tetrahydro-2-[N-(1H-imidazol-4-ylacetyl)-L-valyl]-N-(2-phenylethyl)-3-isoquinolinecarboxamide. |
| (3S)-N-[2-(6-Fluoro-1H-indol-3-yl)-1-methylethyl]-1,2,3,4-tetrahydro-2-[N-(1H-imidazol-4-ylacetyl)-L-valyl]-3-isoquinolinecarboxamide. | (S)-1,2,3,4-Tetrahydro-2-[N-(1H-imidazol-4-ylacetyl)-L-valyl]-N-[3-(1H-imidazol-1-yl)propyl]-3-isoquinoline-carboxamide. |
| [1S-[1R*(R*),2R*]]-1,2,3,4-Tetrahydro-N-[2-hydroxy-1-(hydroxymethyl)-2-[4-(methylthio)phenyl]ethyl]-2-[N-(1H-imidazol-4-ylacetyl)-L-valyl]-3-isoquinoline-carboxamide. | (S)-1,2,3,4-Tetrahydro-2-[N-(1H-imidazol-4-ylacetyl)-L-valyl]-N-[2-(2-thienyl)ethyl]-3-isoquinoline-carboxamide. |
| (S)-N-[3-(Dodecylthio)propyl]-1,2,3,4-tetrahydro-2-[N-(1H-imidazol-4-ylacetyl)-L-valyl]-3-isoquinoline-carboxamide. | (S)-1,2,3,4-Tetrahydro-2-[N-(1H-imidazol-4-ylacetyl)-L-valyl]-N-(4-phenylbutyl)-3-isoquinolinecarboxamide. |
| (S)-1,2,3,4-Tetrahydro-2-[N-(1H-imidazol-4-ylacetyl)-L-valyl]-N-(3-methoxypropyl)-3-isoquinolinecarboxamide. | [1S-[1R*(R*),2S*]]-1,2,3,4-Tetrahydro-N-(2-hydroxy-1-methyl-2-phenylethyl)-2-[N-(1H-imidazol-4-ylacetyl)-L-valyl]-3-isoquinolinecarboxamide. |
| (S)-1,2,3,4-Tetrahydro-2-[N-(1H-imidazol-4-ylacetyl)-L-valyl]-N-[3-(methylthio)propyl]-3-isoquinoline-carboxamide. | (3S)-1,2,3,4-Tetrahydro-2-[N-(1H-imidazol-4-ylacetyl)-L-valyl]-N-(1-methyl-2-phenoxyethyl)-3-isoquinoline-carboxamide. |
| N,N-Bis[(2,3-dimethoxyphenyl)methyl]-N<2-[2-(1H-imidazol-4-yl)ethyl]-L-valinamide, trifluoroacetate. | (S)-N-[2-(3,4-Dimethoxyphenyl)ethyl]-1,2,3,4-tetra-hydro-2-[N-(1H-imidazol-4-ylacetyl)-L-valyl]-3-isoquinolinecarboxamide. |

| | |
|---|---|
| (S)-N-[2-[4-(Aminosulfonyl)phenyl]ethyl]-1,2,3,4-tetrahydro-2-[N-(1H-imidazol-4-ylacetyl)-L-valyl]-3-isoquinolinecarboxamide. | (S)-N-[2-[3,4-Bis(phenylmethoxy)phenyl]ethyl]-1,2,3,4-tetrahydro-2-[N-(1H-imidazol-4-ylacetyl)-L-valyl]-3-isoquinolinecarboxamide. |
| (S)-1,2,3,4-Tetrahydro-2-[N-(1H-imidazol-4-ylacetyl)-L-valyl]-N-[3-(methylsulfonyl)propyl]-3-isoquinolinecarboxamide. | N<2-[2-(1H-Imidazol-4-yl)ethyl]-N-(phenylmethyl)-L-valinamide, trifluoroacetate (1:2). |
| (S)-1,2,3,4-Tetrahydro-N-(5-hydroxy-1,5-dimethylhexyl)-2-[N-(1H-imidazol-4-ylacetyl)-L-valyl]-3-isoquinolinecarboxamide. | N<2-[2-(1H-Imidazol-4-yl)ethyl]-N-(2-phenylethyl)-L-valinamide, trifluoroacetate (1:2). |
| [S-(R*,S*)]-1,2,3,4-Tetrahydro-2-[N-(1H-imidazol-4-ylacetyl)-L-valyl]-N-[2-(4-nitrophenyl)ethyl]-3-isoquinolinecarboxamide. | N<2-[2-(1H-Imidazol-4-yl)ethyl]-N-[[4-(trifluorometh-yl)phenyl]methyl]-L-valinamide. |
| (S)-1,2,3,4-tetrahydro-2-[N-(1H-imidazol-4-ylacetyl)-L-valyl]-N-[2-(6-methoxybenzo[b]thiophen-3-yl)ethyl]-3-isoquinolinecarboxamide. | N-(9H-Fluoren-2-yl)-N<2-[2-(1H-imidazol-4-yl)ethyl]-L-valinamide, trifluoroacetate (1:2). |
| (S)-N-(Methylsulfonyl)-N<2-[[1,2,3,4-tetrahydro-2-[N-(1H-imidazol-4-ylacetyl)-L-valyl]-3-isoquinolinyl]-carbonyl]-L-methioninamide. | N-[2-(3,4-Dimethoxyphenyl)ethyl]-N<2-[2-(1H-imidazol-4-yl)ethyl]-L-valinamide. |
| (S)-N-[6-[[(4-Chlorophenyl)methyl]amino]-6-oxohexyl]-1,2,3,4-tetrahydro-2-[N-(1H-imidazol-4-ylacetyl)-L-valyl]-3-isoquinolinecarboxamide. | N-[(2-Fluorophenyl)methyl]-N<2-[2-(1H-imidazol-4-yl)ethyl]-L-valinamide. |
| (S)-N-(Phenylsulfonyl)-N<2-[[1,2,3,4-tetrahydro-2-[N-(1H-imidazol-4-ylacetyl)-L-valyl]-3-isoquinolinyl]-carbonyl]-L-methioninamide. | N-[(3-Chlorophenyl)methyl]-N<2-[2-(1H-imidazol-4-yl)ethyl]-L-valinamide. |

| | |
|---|---|
| N-[(2,3-Dimethylphenyl)methyl]-N<2-[2-(1H-imidazol-4-yl)ethyl]-L-valinamide. | N<2-[2-(1H-Imidazol-4-yl)ethyl]-N-[[3-(trifluorometh-yl)phenyl]methyl]-L-valinamide. |
| N-[2-(4-Hydroxyphenyl)ethyl]-N<2-[2-(1H-imidazol-4-yl)ethyl]-L-valinamide. | N-[(3,4-Dichlorophenyl)methyl]-N<2-[2(1H-imidazol-4-yl)ethyl]-L-valinamide. |
| N-[(4-Chlorophenyl)methyl]-N<2-[2-(1H-imidazol-4-yl)ethyl]-L-valinamide. | N<2-[2-(1H-Imidazol-4-yl)ethyl]-N-[(2-nitrophenyl)-methyl]-L-valinamide. |
| N-[(2,6-Difluorophenyl)methyl]-N<2-[2-(1H-imidazol-4-yl)ethyl]-L-valinamide. | N<2-[2-(1H-Imidazol-4-yl)ethyl]-N-[(2-methylphenyl)-methyl]-L-valinamide, trifluoroacetate. |
| N-[(2-Ethoxyphenyl)methyl]-N<2-[2-(1H-imidazol-4-yl)ethyl]-L-valinamide. | N<2-[2-(1H-Imidazol-4-yl)ethyl]-N-(1-naphthalenylmeth-yl)-L-valinamide, trifluoroacetate. |
| N-[(3-Chloro-4-fluorophenyl)methyl]-N<2-[2-(1H-imidazol-4-yl)ethyl]-L-valinamide. | N-[2-[4-(Aminosulfonyl)phenyl]ethyl]-N<2-[2-(1H-imidazol-4-yl)ethyl]-L-valinamide, trifluoroacetate. |
| N-[(2-Chloro-4-fluorophenyl)methyl]-N<2-[2-(1H-imidazol-4-yl)ethyl]-L-valinamide. | N-[[4-(Aminosulfonyl)phenyl]methyl]-N<2-[2-(1H-imidazol-4-yl)ethyl]-L-valinamide, trifluoroacetate. |
| N-[(2,3-Dimethoxyphenyl)methyl]-N<2-[2-(1H-imidazol-4-yl)ethyl]-L-valinamide. | N<2-[2-(1H-Imidazol-4-yl)ethyl]-N-(2-naphthalenylmeth-yl)-L-valinamide, trifluoroacetate. |

| | |
|---|---|
| 3-[[N-[2-(1H-Imidazol-4-yl)ethyl]-L-valyl]amino]-benzeneacetic acid, trifluoroacetate. | N-Ethyl-N<2-[2-(1H-imidazol-4-yl)ethyl]-N-(2-methyl-phenyl)-L-valinamide, trifluoroacetate. |
| (S)-1,2,3,4-Tetrahydro-2-[N-[2-(1H-imidazol-4-yl)-ethyl]-DL-valyl]-N-[3-(methylthio)propyl]-3-isoquinolinecarboxamide. | N<2-[2-(1H-Imidazol-4-yl)ethyl]-N-[2-(methylthio)-phenyl]-L-valinamide, trifluoroacetate. |
| (S)-1,2,3,4-Tetrahydro-2-[N-[2-(1H-imidazol-4-yl)-ethyl]-DL-valyl]-N-(phenylmethyl)-3-isoquinoline-carboxamide. | N-(4-Chlorophenyl)-N<2-[2-(1H-imidazol-4-yl)ethyl]-N-methyl-L-valinamide, trifluoroacetate. |
| (3S)-1,2,3,4-Tetrahydro-2-[N-[2-(1H-imidazol-4-yl)-ethyl]-DL-valyl]-N-(1-phenylethyl)-3-isoquinoline-carboxamide. | N-([1,1'-Biphenyl]-4-yl)-N<2-[2-(1H-imidazol-4-yl)ethyl]-L-valinamide, trifluoroacetate. |
| (S)-1,2,3,4-Tetrahydro-N-[2-(4-hydroxyphenyl)ethyl]-2-[N-[2-(1H-imidazol-4-yl)ethyl]-DL-valyl]-3-isoquinolinecarboxamide. | N-([1,1'-Biphenyl]-4-ylmethyl)-N-ethyl-N<2-[2-(1H-imidazol-4-yl)ethyl]-L-valinamide, trifluoroacetate. |
| (S)-N-[(3-Chlorophenyl)methyl]-1,2,3,4-tetrahydro-2-[N-[2-(1H-imidazol-4-yl)ethyl]-DL-valyl]-3-isoquinolinecarboxamide. | N-([1,1'-Biphenyl]-2-ylmethyl)-N-ethyl-N<2-[2-(1H-imidazol-4-yl)ethyl]-L-valinamide, trifluoroacetate. |
| (S)-N-([1,1'-Biphenyl]-2-yl)-N-ethyl-1,2,3,4-tetrahydro-2-[N-[2-(1H-imidazol-4-yl)ethyl]-DL-valyl]-3-isoquinolinecarboxamide. | 4-[[N-[2-(1H-Imidazol-4-yl)ethyl]-L-valyl]amino]benzoicacid, trifluoroacetate. |
| (S)-N-([1,1'-Biphenyl]-4-yl)-N-ethyl-1,2,3,4-tetrahydro-2-[N-[2-(1H-imidazol-4-yl)ethyl]-DL-valyl]-3-isoquinolinecarboxamide. | 3-[[N-[2-(1H-Imidazol-4-yl)ethyl]-L-valyl]amino]benzoicacid, trifluoroacetate. |
| or | (S)-N-(Phenylsulfonyl)-N<2-[[1,2,3,4-tetrahydro-2-[N-[2-(1H-imidazol-4-yl)ethyl]-L-valyl]-3-isoquinolinyl]-carbonyl]-DL-methioninamide. |

**12.** Use of a compound of any one of Claims 1-11 for the manufacture of a medicament for treatment of conditions requiring inhibition of farnesyl protein transferase.

**13.** Use of a compound of any one of Claims 1-11 for the manufacture of a medicament for treatment of conditions requiring inhibition of prenyl transferases.

**14.** Use of a compound of any one of Claims 1-11 for the manufacture of a medicament for treatment of conditions requiring inhibition of tumors.

**15.** Use of a compound of any one of Claims 1-11 for the manufacture of a medicament for treatment of diseases associated with signal transduction pathways operating through Ras.

**16.** Use of a compound of any one of Claims 1-11 for the manufacture of a medicament for treatment of diseases associated with proteins that are post-translationally modified by the enzyme farnesyl protein transferase.

**17.** Use of a compound of any one of Claims 1-11 for the manufacture of a medicament for treatment of diseases associated with proteins that are post-translationally modified by the enzyme geranylgeranyl protein transferase.

**18.** A compound of any one of Claims 1-11 for use as an active pharmaceutical substance.

**19.** A compound of the formula

I

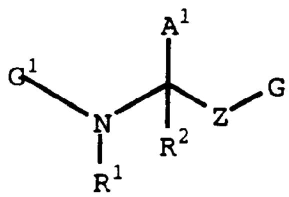

or an enantiomer, diastereomer, pharmaceutically acceptable salt, prodrug or solvate thereof, wherein:

G is

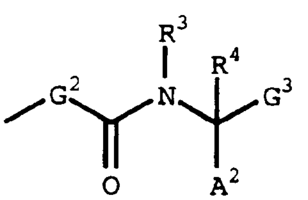 , 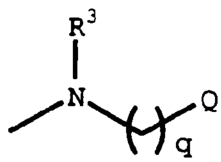 ,

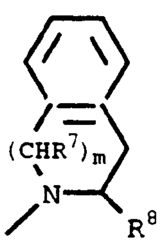 or 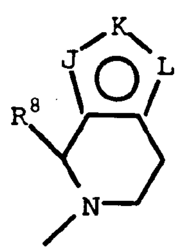 ;

$G^1$ is

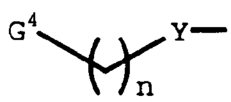

or

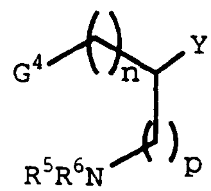

;

$G^2$ is

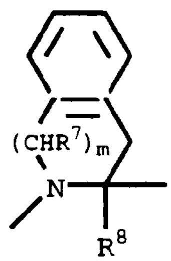

,

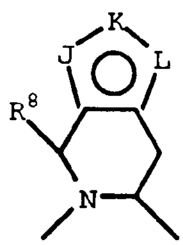

or $-NR^{10}-CH(Q^1)-$;

J, K and L are each, independently, N, $NR^9$, O, S or $CR^{10}$ with the provisos that only one of the groups J, K and L can be O or S, and at least one of the groups J or L must be N, $NR^9$, O or S to form a fused five-membered heteroring; the bond between J and K or K and L may also form one side of a phenyl ring fused to the fused five-membered heteroring;

Q is aryl;

$Q^1$, $A^1$ and $A^2$ are each, independently, H, alkyl, substituted alkyl, phenyl or substituted phenyl;

$G^3$ is $^{R11}$ $-C(O)OR^{11}$, $-C(O)NR^{11}R^{12}$, 5-tetrazolyl, $-C(O)N(R^{13})OR^{11}$, $-C(O)NHSO_2R^{14}$ or $CH_2OR^{11}$;

$G^4$ is

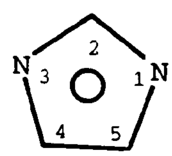

attached at the 1, 2, 4 or 5 position and optionally substituted, at any of the available position or positions on the ring, with halo, alkyl or substituted alkyl having 1 to 20 carbon atoms, alkoxy, aryl, aralkyl, hydroxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, N-hydroxycarbamyl, N-alkylcarbamyl, N-dialkylcarbamyl, alkoxycarbonyl, phenyl, substituted phenyl, or a combinaton of these groups;

Y and Z are each, independently, $-CH_2-$ or -C(O)-;

$R^1$ - $R^{14}$ are each, independently, H or alkyl having 1 to 20 carbon atoms;

$R^7$, $R^8$ and $R^{14}$ may also be aryl or aralkyl, and $R^9$, $R^{11}$, $R^{12}$ and $R^{13}$ may also be aralkyl;

m, n and p are each, independently, 0 or an integer from 1 to 2; and

q is 0 or 1.

**20.** A compound of the formula

I

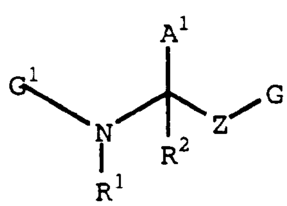

or an enantiomer, diastereomer, pharmaceutically acceptable salt, prodrug or solvate thereof, wherein:

G is

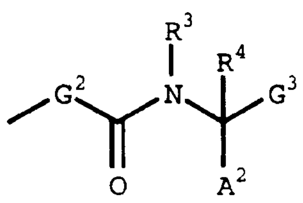 , 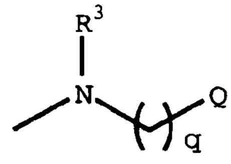 ,

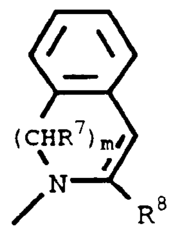 or 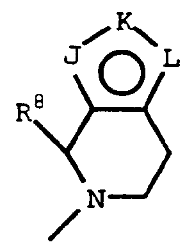 ;

$G^1$ is

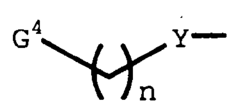 or 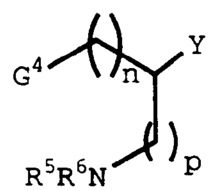 ;

$G^2$ is

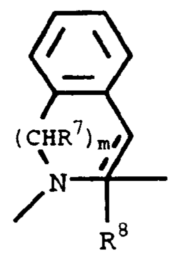 , 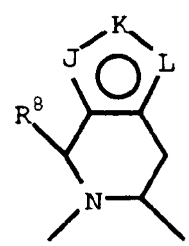

or $-NR^{10}-CH(Q^1)-$;

J, K and L are each, independently, N, $NR^9$, O, S or $CR^{10}$ with the provisos that only one of the groups J, K and L can be O or S, and at least one of the groups J or L must be N, $NR^9$, O or S to form a fused five-membered heteroring; the bond between J and K or K and L may also form one side of a phenyl ring fused to the fused five-membered heteroring;

Q is aryl;

$Q^1$, $A^1$ and $A^2$ are each, independently, H, alkyl, substituted alkyl, phenyl or substituted phenyl;

$G^3$ is $R^{11}$, $-C(O)OR^{11}$, $-C(O)NR^{11}R^{12}$, 5-tetrazolyl, $-C(O)N(R^{13})OR^{11}$, $-C(O)NHSO_2R^{14}$ or $CH_2OR^{11}$;

$G^4$ is

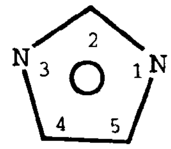

attached at the 1, 2, 4 or 5 position and optionally substituted, at any of the available position or positions on the ring, with halo, alkyl or substituted alkyl having 1 to 20 carbon atoms, alkoxy, aryl, aralkyl, hydroxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, N-hydroxycarbamyl, N-alkylcarbamyl, N-dialkylcarbamyl, alkoxycarbonyl, phenyl, substituted phenyl, or a combinaton of these groups;

Y and Z are each, independently, $-CH_2-$ or -C(O)-;

$R^1$ - $R^{14}$ are each, independently, H or alkyl having 1 to 20 carbon atoms;

$R^7$, $R^8$ and $R^{14}$ may also be aryl or aralkyl, and $R^9$, $R^{11}$, $R^{12}$ and $R^{13}$ may also be aralkyl;

m, n and p are each, independently, 0 or an integer from 1 to 2;

q is 0 or 1; and

the dotted line represents an optional double bond.

European Patent Office

# PARTIAL EUROPEAN SEARCH REPORT

which under Rule 45 of the European Patent Convention shall be considered, for the purposes of subsequent proceedings, as the European search report

Application Number EP 95 30 2188

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | JOURNAL OF BIOLOGICAL CHEMISTRY, vol.266, no.24, 25 August 1991, BALTIMORE, MD US pages 15575 - 15578 J.L. GOLDSTEIN ET AL. 'Nonfarnesylated Tetrapeptide Inhibitors of Protein Farnesyltransferase' * the whole document * --- | 1-20 | C07D233/54 A61K31/415 A61K38/05 A61K38/06 A61K38/07 C07D521/00 C07D401/12 C07K5/023 C07K5/027 C07K5/062 C07K5/065 C07K5/083 C07K5/117 |
| A | WO-A-94 00419 (MERCK & CO., INC.) 6 January 1994 * page 28 - page 29; claim 1 * * page 3, line 32 - page 4, line 3 * --- | 1-20 | |
| P,X | BIOORGANIC & MEDICINAL CHEMISTRY LETTERS, vol.4, no.7, 1994, OXFORD, GB pages 887 - 892 K. LEFTHERIS ET AL. 'Peptide Based P21RAS Farnesyl Transferase Inhibitors: Systematic Modification of the Tetrapeptide CA1A2X Motif' * page 889; table 1, compound no. 5 * --- -/-- | 1,3-6,8, 19,20 | TECHNICAL FIELDS SEARCHED (Int.Cl.6) C07D C07K |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 19 July 1995 | Fink, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 95 30 2188

| DOCUMENTS CONSIDERED TO BE RELEVANT | | | CLASSIFICATION OF THE APPLICATION (Int.Cl.6) |
|---|---|---|---|
| Category | Citation of document with indication, where appropriate, of relevant passages | Relevant to claim | |
| E | WO-A-95 12612 (WARNER-LAMBERT COMPANY) 11 May 1995<br>* page 56 - page 57; claim 1 *<br>--- | 1,3,4,8, 19,20 | |
| E | WO-A-95 11917 (PARKE, DAVIS & COMPANY) 4 May 1995<br>* page 57 - page 58; claim 1 *<br>----- | 1,3,4,8, 19,20 | |
| | | | TECHNICAL FIELDS SEARCHED (Int.Cl.6) |

EPO FORM 1503 03.82 (P04C10)

EP 95 30 2188

-C-

As the drafting of claim 1 encompasses such an enormous amount of compounds, a complete search is not possible on economic grounds (cf. Guidelines for the Examination in the EPO; Part B, Chapter III, item 2).

Therefore, the search has been limited to the compounds of claim 1 wherein:

$G^1 = G^4-(CH_2)_n-Y-$; and

$A^1 = -CH(CH_3)_2, -C(CH_3)_3, -CH(CH_3)CH_2CH_3$